(19) Europäisches Patentamt
European Patent Office
Office européen des brevets

(11) EP 3 290 500 B1

(12) EUROPÄISCHE PATENTSCHRIFT

(45) Veröffentlichungstag und Bekanntmachung des Hinweises auf die Patenterteilung:
**15.05.2019 Patentblatt 2019/20**

(21) Anmeldenummer: **17020388.9**

(22) Anmeldetag: **25.08.2017**

(51) Int Cl.:
***C11D 1/37*** *(2006.01)*
***C11D 1/83*** *(2006.01)*
***C11D 1/86*** *(2006.01)*
***C11D 10/04*** *(2006.01)*
***A61K 8/36*** *(2006.01)*
***A61K 8/362*** *(2006.01)*
***A61Q 5/00*** *(2006.01)*
***A61K 8/60*** *(2006.01)*
***A61Q 9/02*** *(2006.01)*
***A61Q 11/00*** *(2006.01)*
***A61Q 19/00*** *(2006.01)*

(54) **WASCH-, PFLEGE- UND REINIGUNGSMITTEL MIT POLYOXYALKYLEN CARBOXYLAT**

DETERGENT COMPOSITION AND CARE COMPOSITION CONTAINING POLYOXYALKYLENE CARBOXYLATE

COMPOSITION DE LAVAGE, DE NETTOYAGE ET DE SOIN CONTENANT DE CARBOXYLATE POLYOXYALKYLÉNÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.08.2016 CH 11132016**

(43) Veröffentlichungstag der Anmeldung:
**07.03.2018 Patentblatt 2018/10**

(73) Patentinhaber: **Richli, Remo**
**6052 Hergiswil (CH)**

(72) Erfinder:
- **Der Erfinder hat auf sein Recht verzichtet, als solcher bekannt gemacht zu werden.**

(56) Entgegenhaltungen:
**WO-A1-2016/146497 WO-A2-2013/098066**
**DE-A1- 4 409 189**

EP 3 290 500 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand dieser Anmeldung sind Mittel, die mindestens ein Glycolipid-Biotensid und mindestens ein Polyoxyalkylen Carboxylat enthalten. Das oder die Polyoxyalkylen Carboxylate basieren auf Fettsäuren aus Pflanzenölen und weisen einen aussergewöhnlich hohen Anteil an langkettigen (≥ C17), mehrheitlich ungesättigten Kohlenwasserstoffketten auf, wobei das Polyoxyalkylen Carboxylate in einem Verhältnis von ≥ 1: 6 zum Biotensid vorliegt. Weiterer Gegenstand ist die Verwendung als Wasch-, Pflege- und Reinigungsmittel.

## Problem - Stand der Technik

Die vorliegende Erfindung richtet sich auf ein Mittel mit verbesserter Reinigungsleistung, insbesondere von kohlenhydrathaltiger Verschmutzung und von Anfärbungen, sowie ein Verfahren zur Verbesserung der Reinigungsleistung eines Wasch- und Reinigungsmittels sowie die Verwendung des Mittels.

Die Entfernung von kohlenhydrathaltigen Verunreinigungen ist in der Körperpflege, Textilpflege und Reinigung von Oberflächen, z.B.im Lebensmittelbereich ausserordentlich wichtig.

In der Körperpflege spielen insbesondere bei erkrankter Haut Pilze oder Bakterien eine zentrale Rolle wie z.B. Propionibacterium acnes (Akne), Streptococcen (Zahnbelag), Malassezia furfur (Haarschnuppen). Bekannt ist, dass oberflächenaktiven Mittel die Permeabilität der biologischen Membranen modifizieren und damit antibakteriell, antiviral oder fungizid wirken. So offenbart z.B. EP 209 783 (Wella) Sophorolipide und deren Verwendung als Antischuppenmittel und bakteriostatisches Mittel.

Abgesehen von der antimikrobiellen Wirkung, ist es jedoch von grösster Bedeutung, der Besiedelung durch Mikroorganismen von vornherein präventiv entgegenzutreten. Dies ist möglich, indem man den Mikroorganismen ihre Nahrungsgrundlage - welche in erster Linie aus Kohlenhydraten bestehen- entzieht.

Insbesondere können Kohlenhydrate die Zahngesundheit beeinträchtigen, da sie von bestimmten Bakteriensorten in der Mundhöhle beispielsweise zu Milchsäure abgebaut werden und so die Kariesbildung unterstützen können.

Ein weiterer Aspekt hygienischer Körperreinigung ist die Entfernung der häufig pathogenen Stoffwechselprodukte. So produziert beispielsweise Malassezia furfur fluoreszierende Pigmente, welche mit der pathogenen Wirkung in Zusammenhang gebracht werden.

Die Kohlenhydratentfernung ist auch in der Gesichtspflege und Rasur wichtig. Insbesondere das Gesicht ist Umwelteinflüssen und -verschmutzungen ausgesetzt. So können sich z.B. Pollen, deren Hauptbestandteil Kohlenhydrate sind sowie Russ- und Staubteilchen an Haut und Haaren adsorbieren.

Weiterhin ist in der Körperpflege, wo Grenzen durch Temperatur, Mechanik und Inhaltstoffe gesetzt sind, die Entfernung von Anfärbungen wie z.B. Makeup durch milde Tensidsystem eine Herausforderung an Produktentwickler. Damit ist für die Körperreinigung, sei es auf der Haut oder den Haaren, wichtig, neben der guten Entfernung von Kohlenhydraten, auch die Entfernung von Farb-bzw. Pigmentschmutz zu erzielen.

In der Textilpflege werden farbige Stärke- und Zuckerflecken, wie beispielsweise Tomaten-, Karotten- oder Senfflecken, aber auch Grasflecken auf Cellulosebasis, üblicherweise durch Enzyme und/oder Bleichmittel entfernt. Ein Verzicht auf diese Waschmittelrohstoffe hat Vorteile für die Stabilität der Formulierung sowie für die Umwelt. Wünschenswert ist daher, eine hohe Reinigungsleistung für Kohlenhydratflecken allein schon durch das Tensidsystem zu erzielen.

Weiterhin ist die gründliche Reinigung auf Oberflächen aller Art erforderlich. Im Lebensmittelbereich findet man beispielsweise Kohlenhydratablagerungen von Speisen. Diese sind oft hartnäckig und die eingesetzten Reinigungsmittel dementsprechend aggressiv. Ferner sind im Lebensmittelbereich Reinigungsmittel mit geringem toxikologischen Profil gefordert, während gleichzeitig spezielle, technisch anspruchsvolle Eigenschaften gefordert werden, wie beispielsweise eine gute Kohlenhydratentfernung bei saurem pH. So erfordert beispielsweise die Reinigung von Brauereien eine gleichzeitige Entfernung von Kohlenhydratablagerungen aus Zucker und Stärke sowie Biersteinablagerungen. In den porösen Biersteinablagerungen finden bisweilen Mikroorganismen einen Unterschlupf, der sie vor allem einer alkalischen Behandlung gegenüber oft unempfindlicher macht.

Neben der Anforderung an Tensidsysteme, flexibel in einem breiten pH-Bereich einsetzbar zu sein, wächst der Bedarf an umweltfreundlichen und toxikologisch sicheren Inhaltsstoffen. Als Standard werden in der Reinigungsmittelindustrie als primäre Tenside Natrium lauryl sulfat bzw. Natrium laurylether sulfat und Alkylpolyglycoside (APGs) eingesetzt, nicht zuletzt aufgrund der vorteilhaften Kosten-Nutzen Betrachtung. Diese Tenside erweisen sich als robust über einen hohen pH-Bereich, kompatibel mit fast allen üblichen Inhaltsstoffen und damit flexibel einsetzbar bei einer guten Reinigungsleistung gegenüber allen üblichen Schmutzarten.

Jedoch wird die Nachhaltigkeit dieser Tenside zunehmend in Frage gestellt, da sie auf Erdöl oder pflanzlichen Ölen aus tropischen Monokulturen basieren. Diese Pflanzenöle, wie z.B. Kokos- oder Palmkernöl, werden aufgrund ihrer technischen Eigenschaften wie vorteilhafte Schaum-, Wasch- und Reinigungsleistung eingesetzt, die sie dank ihrem hohen Laurinsäuregehalt (C12) besitzen.

Es ist jedoch eine Tatsache, dass durch die unkontrollierte Rodung wertvoller tropischer Regenwälder zur

Gewinnung von Anbaufläche für Palmöl zahlreiche Tier- und Pflanzenarten vom Aussterben bedroht sind. Der Anbau von Palmöl wird zwischenzeitlich in den Medien als der grösste Klimakiller unseres Jahrhunderts bezeichnet. Eine Alternative im Wasch- und Reinigungsbereich ist bis heute nicht verfügbar.

In einem geringeren Mass werden seit jeher tierische Öle und Fette, insbesondere Rindertalg für Reinigungsmittel eingesetzt. Aufgrund der Fettsäurezusammensetzung können diese nur begrenzt in bestimmten Anwendungen eingesetzt werden. Aus Konsumentensicht sind tierische Rohstoffe aus hygienischen (z.B. TSE-Problematik) und ethischen Gründen (z.B. vegan-Trend) oft nicht erwünscht. Weiterhin wird Pflanzenseife seit Jahrtausenden für Wasch- und Reinigungszwecke eingesetzt, deren Anwendung ist aufgrund der Bildung von Kalkseifen und Bindung an einen alkalischen pH-Wert ebenfalls begrenzt.

Die Herausforderung besteht daher darin, auf Erdöl, tierische Fette und Öle, sowie auf Palmöle (d.h. Palmöl, Palmkernöl, Kokosöl, Babassuöl) als Fettsäurequelle zu verzichten und stattdessen in einem grösstmöglichen Mass Tenside aus weniger problematischen Quellen, wie zum Beispiel pflanzlichen Ölen aus europäischem Anbau oder Fermentation einzusetzen.

Technisch ist dies ein Problem, da aus verfügbaren Ölen, zum Beispiel aus Mitteleuropa, die gewünschte Laurinsäure nicht in ausreichendem Mass gewonnen werden kann.

Glycolipid-Biotenside, welche über Fermentation aus unterschiedlichsten Substraten hergestellt werden können, erfüllen die Anforderungen an Nachhaltigkeit und zeichnen sich zudem durch geringes Hautirritationspotential und Toxizität aus. Im Gegensatz zu synthetischen Glycolipiden, wie z.B. Alkylpolyglycosiden, Sorbitanestern, Methylglycosidestern oder Methylglucamiden, werden Biotensid-Glycolipide über Mikroorganismen hergestellt und es sind keine chemischen Umsetzungsschritte notwendig. Um eine optimale Reinigungsleistung zu erhalten, ist es für viele Anwendungen jedoch erforderlich, Glycolipid-Biotenside mit anderen Tensiden zu kombinieren.

Hierbei wird nach Stand der Technik wiederum mit Tensiden auf Laurinsäurebasis aus Palmen oder petrochemischer Herkunft kombiniert; zumeist mit anionischen schwefelhaltigen Tensiden wie z.B. Natrium lauryl sulfat oder Natrium laurylether sulfat.

Nach Stand der Technik sind Kombinationen von Glycolipid Biotensiden mit anionischen und nichtionischen nicht-glycolipidischen Tensiden mit Alkylkettenlängen von C8-C18 bekannt, DE19600743 (Henkel) offenbart Mischungen von Glucoselipiden und Sophoroselipiden mit unterschiedlichen Tensiden für Geschirrspülmittel. In den Beispielen offenbart werden Kombinationen mit Tensiden auf Basis Laurinsäure für eine synergistische Verstärkung hinsichtlich der Spülleistung, des Dispergiervermögens und der Schaumkraft.

WO2011051161A1 (Henkel) offenbart rückstandsarme Reiniger für harte Oberflächen mit einem Glycolipid-Biotensid und einem Lösungsmittel. In den Beispielen offenbart werden Tensidkombinationen mit C12-C14-Tensiden.

EP 0 499434 A1 (Unilever): Kombination von mindestens einem Glycolipid-Biotensid und mindestens einem nicht- glycolipidischen anionischen oder nichtionischen Tensid, wobei je eines der Tenside in der mizellaren und eines in der lamellaren Phase vorliegt. Die offenbarten Textilwaschmittel zeichnen sich durch eine erhöhte Öllösekraft von den Textilien aus.

EP 1 445 302 A1/US 2014113818 (Ecover): Kombination von mindestens einem Glycolipid Biotensid und mindestens einem nicht- glycolipidischen Tensid jeweils in der mizellaren Phase. Alle vorgestellten Lösungen enthalten kurzkettige, gesättigte Kohlenwasserstoffketten als hydrophoben Teil der Tenside. Durch das Vorliegen des Biotensids und des weiteren Tensids in der mizellaren Phase wird eine erhöhte durchschnittliche Reinigungsleistung auf Standardschmutz oder Mineralöl bei geringer Schaumbildung erreicht.

JP 2009275145 (Seraya) offenbart Mischungen für die Hautreinigung mit Sophorolipiden in Kombination mit Seifen. Durch die Kombination von Seifen mit Glycolipiden wird eine bessere Abwaschbarkeit der Seife erreicht.

WO 2012617815 (Ecover) offenbart Mischungen basierend auf konzentrierten Sophorolipidmischungen von 70-99%, welche hydrolysiert mit anderen Tensiden auf C12-Fettsäurebasis und mit Natrium Cocoamphoacetat als Cotensid gemischt werden um den Schaum zu verstärken.

WO 2016050439 (Evonik) offenbart biotensidhaltige Formulierungen mit verbessertem Schaumbildungs- und Fettlösevermögen enthaltend mindestens ein Tensid ausgewählt aus der Gruppe der Betaine, alkoxylierte Fettalkoholsulfate und Alkylaminoxide.

Insbesondere bei sauren Reinigern und Waschmitteln werden in Kombination mit Biotensiden hauptsächlich C12-C18-Tenside auf Basis von Schwefelverbindungen als anionische Tenside eingesetzt. Beispiele offenbarter Kombinationen (WO2014166796, WO2014/118095) enthalten etwa Sulfate, Sulfonate, Isethionate, Sulphosuccinate u.a., insbesondere Laurylethersulfate und Laurylsulfonate.

WO 2016/066464 (Henkel) offenbart Waschmittel mit Mannosylerythritol für fett- und ölhaltige Flecken in Kombination mit C12-C14 bzw. C12-C18 Ethersulfaten und ethoxylierten Alkoholen.

Weiterhin bekannt aus EP 0499434, US 5520839 ist die Kombination von Biotensiden mit anionischen Tensiden u.a. Rapsseife für eine verbesserte Öl- und Fettlöslichkeit.

US 2004/0265264 (Beiersdorf) offenbart die Verwendung von Sodium PEG-7 Olivenölcarboxylat in "katalytischen" Mengen zur Reduktion von Hautirritation durch Natrium Laurethsulfat. In WO 2013098066 (Evonik) wird Sodium

PEG-7 Olivenölcarboxylat in vergleichbar geringen Mengen zusammen mit weiteren Tensiden auf Basis Laurylbasis und Sophorolipid verwendet für ein Babyreinigungsmittel. Das Ausführungsbeispiel offenbart die sensorisch positive Auswirkung auf die Haut durch die Kombination von Biotensiden mit Ölsäure, die Reinigungsleistung wird nicht erwähnt. DE 10147049 (Beiersdorf) offenbart Tensidgemische aus Natrium Cocoylglutamat, Natrium Myristylethersulfat und Natrium PEG-7 Olivenölcarboxylat, welche selektiv Oberflächenlipide statt Sebumlipide auswaschen und somit Hautrauigkeit vermindern.

Damit ist aber der Problem nicht gelöst, umweltfreundliche Reiniger mit Biotensid-Glycolipiden und Tensiden auf Basis von Pflanzenölen mit einem hohen Gehalt an langkettigen (≥ C18) und vorwiegend ungesättigten Fettsäuren herzustellen, welche gleichzeitig eine hohe Reinigungsleistung, insbesondere auf Kohlenhydrate und Farbverschmutzungen aufweisen, sowie über einen breiten pH-Bereich anwendbar und vorzugsweise biologisch abbaubar sind.

Die komplexe technische Aufgabe der Erfindung hat darin bestanden, ein oder mehrere Tenside auf Basis Pflanzenöl zu identifizieren, die sich mit Biotensid-Glycolipiden kombinieren lassen. Im Gegensatz zu bisher bestehenden Kombinationen, sollen die Fettsäuren der Tenside aufgrund von Nachhaltigkeitsbetrachtungen möglichst nicht aus Kokos-, Palm- , Babassu- oder Palmkern- Pflanzenölen gewonnen werden. Dies ist technisch insofern anspruchsvoll, da die gewünschten Tenside statt Laurinsäure einen hohen Anteil an ungesättigten, langen Fettsäureresten ≥ C18 aufweisen, welche in üblich eingesetzten Tensidkonzentrationen komplett neue Eigenschaften wie Schaum, Stabilität, Reinigungsleistung, Kompatibilität, u.a. mit sich bringen. Gleichzeitig sollen die erfindungsgemässen Tensidkombinationen eine gute Reinigungsleistung aufweisen, auch über Fett- und Ölschmutz hinaus; d.h. eine hohe Reinigungsleistung auf spezifische Flecken wie Kohlenhydratflecken oder Anfärbungen. Weiterhin war es ein Ziel, ein und dieselbe Tensidmischung über einen breiten pH-Bereich einzusetzen, sowie mit unterschiedlichen Inhaltsstoffen zu kombinieren um zugunsten einer guten Wirtschaftlichkeit und Kosteneffizienz eine Basis für unterschiedliche Verwendungen zur Verfügung zu haben. Dazu müssen die Mischungen, anders als beispielsweise Seife, auch im sauren pH stabil sein.

Die erfindungsgemässen Zusammensetzungen sollten vorzugsweise zu einem grösstmöglichen Umfang auf natürlichen Rohstoffen basieren und biologisch gut abbaubar sein.

## Beschreibung der Erfindung

Überraschenderweise wurde gefunden, dass binäre Kombinationen von Glycolipid-Biotensiden mit Polyoxyalkylen Carboxylate auf Basis von C-18-Pflanzenölen, wie in den Ansprüchen beschrieben, eine oder mehrere der genannten Aufgaben lösen, wobei das Polyoxyalkylen Carboxylate in einem Verhältnis von ≥ 1: 6 zum Biotensid vorliegt. Die Mittel sind in den Ansprüchen 1-5, 10-12 beschrieben. Unerwarteterweise zeigte sich, dass die erfindungsgemässen Mittel eine für den Fachmann in keiner Weise vorhersehbare Reinigungswirkung auf spezifische Verschmutzungen zeigt. Dies ermöglicht die Herstellung von umweltfreundlichen Mitteln, selbst für hartnäckige Verschmutzungen wie Anfärbungen oder Kohlenhydratverunreinigungen.

Es wurde festgestellt, dass die erfindungsgemässen Mittel wider Erwarten synergistisch auf das Lösen von Kohlenhydratflecken wirken. Hierbei wird unter synergistisch verstanden, dass die Flecklösekraft der Mischung höher ist als die Flecklösekraft der einzelnen Komponenten. In der enzymfreien Ausführungsform wird eine vergleichbare Reinigungsleistung zu enzymhaltigen Marktprodukten erreicht.

Überraschenderweise wurde auch eine synergistische Wirkung bei Farbstoffflecken festgestellt. Eine deutliche Verbesserung von bleichbaren Flecken wird auch in der Ausführungsform ohne Bleichmittel beobachtet.

Weiterhin ist Gegenstand dieser Anmeldung die Verwendung der erfindungsgemässen Zusammensetzungen als oder zur Herstellung von Wasch-, Pflege- und Reinigungsmitteln für synthetische, biologische oder natürliche Oberflächen, harte oder flexible Oberflächen, sowie für Textilien, Teppiche oder Naturfasern.

In einem weiteren Erfindungsgegenstand richtet sich die Erfindung auf ein Wasch- und Reinigungsverfahren umfassend

a) die Bereitstellung einer Wasch- und Reinigungslösung umfassend ein Mittel gemäss des ersten Erfindungsgegenstandes

b) in Kontakt bringen einer Oberfläche oder Textilien, Teppiche oder Naturfasern mit der Waschlösung gemäss (a).

Eine besondere Produktform stellen feste Substrate, wie Tücher dar. Diese werden mit einer Zubereitung getränkt und haben den Vorteil, dass bereits die richtige Dosierung vorgegeben ist. Dies kommt insbesondere dem Konsumentenwunsch der Convenience entgegen, sie sind einfach handhabbar, direkt zu verwenden ohne zusätzliche Arbeitsschritte und können auch unterwegs, z.B. auf Reisen gut angewendet werden, auch wenn kein Wasser zur Verfügung steht.

Tücher werden aus Textilien hergestellt, welche gewebt, gestrickt, oder gewirkt sein können oder als Verbundstoff in Vlies, Papier, Watte oder Filz vorliegen, wobei Vliese meist aus Polypropylen, Polyester oder Viskose hergestellt

werden.

Mit Mitteln imprägnierte Substrate und Tücher können auf unterschiedliche Weisen hergestellt werden - dem Tauch-, dem Abstreif- und dem Sprühverfahren. Letzteres wird insbesondere für nicht oder schwach schäumende Zubereitungen angewendet.

Vorteilhaft ist, dass das erfindungsgemässe Mittel über den gesamten pH-Bereich eingesetzt werden kann, und somit eine breite Gamme an Produkten erlaubt.

Ein weiterer Vorteil der erfindungsgemässen Mittel besteht darin, dass sie schäumen, wobei die Schaumstabilität in dem Mittel erstaunlich hoch ist.

Überraschenderweise zeigen die Mittel zudem eine hohe Kalklösekraft in saurer Lösung.

Ein weiterer Vorteil ist, dass sich das erfindungsgemässe Mittel durch eine hohe Stabilität auszeichnet. Das erfindungsgemässe Mittel wird auch in einer konservierungs-mittelfreien Ausführungsform offenbart.

Weiterhin zeigen die Mittel eine so hohe Wasch- und Reinigungskraft, dass auf die gängigen Tenside wie Laurylsulfat auf Basis Palmkernöl vollständig verzichtet werden kann, welche in höheren Dosen haut- und schleimhautreizend wirken können. Eine Ausführungsvariante ist daher frei von Schwefeltensiden, insbesondere frei von Laurylsulfat und Laurylethersulfat und trägt dem Trend "sulfatfrei" Sorge.

Ferner entwickeln die erfindungsgemässen Mittel einen stabilen Schaum und machen damit Alkylamidobetaine, Monoethanolamide und Diethanolamide entbehrlich. Diese Substanzen weisen ein erhebliches Irritationspotential auf. Eine bevorzugte Ausführungsvariante ist daher frei von Alkylamidobetaine, wie bspw. Cocoamidopropylbetain. Eine weitere bevorzugte Ausführungsvariante ist frei von Mono- und Diethanolamiden, wie Cocamid DEA, Cocamid MEA u.a.

**Definitionen:**

Technisch unterscheiden sich die Pflanzenöle aus Ölpalmen, Babassu, Palmkernen, oder Kokosnüssen deutlich in der Fettsäurezusammensetzung von den erfindungsgemässen C-18-Pflanzenölen:
In dieser Erfindung werden folgende Pflanzenöle, - fette, -wachse oder -harze als C-18-Pflanzenöl bezeichnet:
Bevorzugt handelt es sich bei den C-18-Pflanzenölen um natürliche Triglyceride. C-18-Pflanzenöle weisen ein Gemisch an gesättigten und ungesättigten Fettsäuren auf, wobei die Fettsäureverteilung von Fettsäuren mit 18 und mehr Kohlenstoffatomen über 60 Gew.-%, besonders bevorzugt über 72 Gew.-% und ganz besonders bevorzugt von über 77 Gew.-% liegt und wobei der Anteil an ungesättigten Fettsäuren über 55 Gew.-%, vorzugsweise über 65 Gew.-% und besonders bevorzugt über 72 Gew.-% liegt.

Bevorzugt liegt der Anteil an Fettsäuren mit 16 und weniger Kohlenstoffatomen unter 30 Gew.-%, bevorzugt unter 27 Gew.-% und besonders bevorzugt unter 17 Gew.-%. Bevorzugt enthalten die C-18-Pflanzenöle einen Anteil von < 0.5%, besonders bevorzugt > 0.05% Fettsäuren mit 6 Kohlenstoffatomen.

Bevorzugt enthalten die C-18-Pflanzenöle einen Anteil von < 75 Gew-% Hydroxyfettsäuren, bevorzugt < 25 Gew.-%, besonders bevorzugt < 5 Gew.-%.

Bevorzugt enthalten C-18-Pflanzenöle gesättigte oder ungesättigte Fettsäuren mit 20 und mehr Kohlenstoffatomen, wobei deren Gehalt bis zu 96 Gew.-% betragen kann. Bevorzugt enthalten C-18-Pflanzenöle einen Anteil von gesättigten oder ungesättigten Fettsäuren mit 20 und mehr Kohlenstoffatomen von > 0.01 Gew.-% und besonders bevorzugt > 0.05 Gew.-% und ganz besonders bevorzugt > 0.1 Gew-% und äusserst bevorzugt >= 0.2 Gew.-%

Bevorzugt enthalten die C-18-Pflanzenöle einen Anteil von weniger als 95 Gew.-% Ölsäure, besonders bevorzugt unter 85 Gew.-% Ölsäure

Gew.-% hier jeweils bezogen auf den Gesamtgehalt an Fettsäuren im Pflanzenöl.

Aus folgenden Pflanzen bzw. Pflanzenteilen, wie Samen, Kerne, Früchte, Blätter, Wurzeln und andere, im folgenden C-18-Pflanzen genannt, können C-18 Pflanzenöle gewonnen werden, welche die technischen Merkmale betreffend Fettsäurezusammensetzungen für die erfindungsgemässen Mittel erfüllen und definiert sind wie folgt: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kreuzblütengewächse, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen.

Vorzugsweise ist das Öl ausgewählt aus der Gruppe: Aprikose, Avocado, Baumwolle, Brokkoli, Buche, Distel, Dinkel, Erdmandel, Gerste, Hanf, Haselnuss, Jojoba, Kirsche, Königskerze, Krambe, Kreuzblättrige Wolfsmilch, Kürbis, Iberischer Drachenkopf, Leindotter, Leinsamen, Lupine, Luzerne, Macademia, Mandel, Mais, Mohn, Nachtkerze, Olive, Ölrettich, Ölrauke, Pfirsich, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube und Weizen, sowie deren Kombinationen.

Ganz besonders bevorzugt ist das Öl ausgewählt aus der Gruppe Aprikose, Distel, Erdmandel, Hanf, Krambe, Iberischer Drachenkopf, Leindotter, Leinsamen, Lupine, Luzerne, Mais, Mandel, Olive, Ölrettich, Pfirsich, Raps, Rübsen, Sesam, Sesamblatt, Sonnenblume, Soja, Weintraube und Weizen, sowie deren Kombinationen.

Der Begriff Öle wird in dieser Erfindung stellvertretend für Fette, Wachse und Harze verwendet.

Im Rahmen der vorliegenden Erfindung steht - soweit nicht anders angegeben- auf Basis von oder abgeleitet von Pflanzenölen, -fetten oder -wachsen stellvertretend für Derivate aus Fettsäuren - gereinigt oder als Gemisch - und/oder deren Reaktionsprodukte, wie beispielsweise Additionsprodukte an die Doppelbindung, Reaktionen an der Fettsäurefunktion, wie z.B. Fettalkohole und deren Ether und/ oder Carboxyether, Amine oder Fettsäureamide, Fettsäureester, sowie Imine. Bevorzugt liegen diese Fettsäurederivate als Mischung gemäss der Fettsäureverteilung im nativen Öl vor oder wie sie bei der Umsetzung von natürlich vorkommenden Pflanzenöle oder Fetten anfallen.

Im Rahmen der vorliegenden Erfindung stehen Fettsäuren bzw. Fettalkohole bzw. deren Derivate soweit nicht anders angegeben - stellvertretend für verzweigte oder unverzweigte, lineare oder substituierte, insbesondere hydroxysubstituierte, gesättigte, einfach oder mehrfach ungesättigte Carbonsäuren bzw. Alkohole bzw. deren Derivaten mit vorzugsweise 6 bis 24 Kohlenstoffatomen.

Unter Tensid werden im Zusammenhang dieser Erfindung amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Mizellen, lamellare Strukturen u.a. Im Zusammenhang mit dieser Erfindung werden als Tenside Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

PEGylierte Pflanzenöle sind ethoxylierte Pflanzenöle wie definiert in "Safety Assessment of PEGylated Oils as Used in Cosmetics", International Journal of Toxicology November/December 2014, 33. Im Rahmen dieser Erfindung wird die Terminologie verwendet, welche bei kosmetischen Inhaltsstoffen Anwendung findet, welche die Veretherungs- und Veresterungsprodukte von Glyceriden und Fettsäuren mit Ethylenoxid beschreibt. Im Rahmen der Erfindung sind hier insbesondere Vertreter abgeleitet von C-18-Pflanzen bevorzugt;

PEGylierte Fettsäureglyceride sind Mono-, Di- und/oder Triglyceride, welche mit einer spezifischen Anzahl an Alkylenglycol-Einheiten, meist Ethylenglycoleinheiten modifiziert wurden und Nebenprodukte der Reaktion enthalten können. Im Rahmen dieser Erfindung werden PEGylierte Fettsäureglyceride definiert wie in "Safety Assessment of PEGylated Alkyl Glycerides as Used in Cosmetics", Cosmetic Ingredient Review (CIR) 2014. Zu bemerken ist, dass CIR unter "Alkyl" auch ungesättigte Fettsäuren berücksichtigt. Im Rahmen der Erfindung sind hier insbesondere Vertreter abgeleitet von C-18-Pflanzen bevorzugt;

Im Rahmen dieser Erfindung steht soweit nicht anders vermerkt Biotensid für die erfindungsgemäss definierten Biotensid-Glycolipide.

Im Rahmen dieser Anmeldung wird unter "Schwefeltenside" anionische oder amphotere Tenside mit einem schwefelhaltigen hydrophilen Rest verstanden wie z.B. Alkylsulfate, Alkylethersulfate, (alkoxylierte) Sulfosuccinate, (alkoxylierte) Sulfonate, (alkoxylierte) Isethionate, (alkoxylierte) Taurate, Sulfobetaine und Sultaine. Beispiele für sulfathaltige Tenside stellen Sodium Laureth Sulfate, Sodium Lauryl Sulfate, Ammonium Laureth Sulfate, Ammonium Lauryl Sulfate, Sodium Myreth Sulfate, Sodium Coco Sulfate, Sodium Trideceth Sulfate oder MIPA-Laureth Sulfate dar.

Frei von Schwefeltensiden, Phosphaten, Phosphonaten bedeutet, dass die Formulierung keine nennenswerten Mengen an Schwefeltensiden, Phosphaten, Phosphonaten aufweisen. Insbesondere ist hierunter zu verstehen, dass Schwefeltenside, Phosphate, Phosphonate jeweils in Mengen von kleiner 0.1 Gew.-%, bevorzugt von kleiner 0.01 Gew.-% bezogen auf die Gesamtformulierung, insbesondere keine nachweisbaren Mengen, enthalten sind.

"Mindestens ein" wie hierin verwendet, bezieht sich auf 1 oder mehr, beispielsweise 1, 2, 3, 4, 5, 6, 7, 8, 9 oder mehr.

Unter "Wasch-, Pflege- und Reinigungsmittel" wird im Rahmen der Erfindung ein Mittel zur Entfernung unerwünschter Verschmutzungen oder Belägen verstanden wie beispielweise Flecken, Rückstände, Verunreinigungen, Stoffwechselprodukte von biologischen Vorgängen von natürlichen oder biologischen Oberflächen, harten Oberflächen, sowie Textilien, Teppiche oder Naturfasern. Das Mittel kann reinigen, die Attraktivität einer Person erhöhen, die Gesundheit von Zähnen, Haut und Haar erhalten, inklusive der Rasur, verschönern und pflegen. In den Begriff eingeschlossen ist die Tierpflege und -reinigung.

Die Mittel können durch einreiben, zudosieren, sprayen, schäumen und andere Methoden (z.B. salben, auflegen, etc.) direkt oder über ein Hilfsmittel wie beispielsweise ein Tuch, verdünnt oder unverdünnt, auf das zu reinigende Material, Haut, Haar etc. aufgebracht werden.

Unter "Reinigungsleistung" oder "Waschkraft" wird im Rahmen dieser Erfindung die Entfernung von einer oder mehreren Anschmutzungen verstanden.

Die Entfernung kann über eine Aufhellung oder Verringerung der Anschmutzung messtechnisch erfasst oder

visuell beurteilt werden.

Unter Anfärbungen oder Farbflecken werden sowohl Farbstoff-, als auch Pigmentflecken verstanden.

Der HLB (hydrophile-lipophile balance) Wert ist ein Mass für die Hydrophilie, bzw. Lipophilie eines Stoffes, in der Regel eines nichtionischen Tensids. Der Wert kann theoretisch wie in einschlägiger Literatur beschrieben (z.B. nach der Griffin-Methode) oder experimentell durch den Vergleich des Löslichkeitsverhaltens von Standardzusammensetzungen mit bekanntem HLB gemessen werden.

Stoffe, die auch als Inhaltsstoffe von kosmetischen Mitteln dienen, werden nachfolgend gegebenenfalls gemäss der International Nomenclature Cosmetic Ingredient- (INCI-) Nomenklatur bezeichnet. Die INCI-Bezeichnungen sind dem "International Cosmetic Ingredient Dictionary and Handbook, 13th Edition (2010)" zu entnehmen. Herausgeber: The Personal Care Products Council.

Soweit nicht explizit anders angegeben, beziehen sich die angegeben Mengen in Gewichtsprozent (Gew.-%) auf das gesamte Mittel. Dabei beziehen sich die prozentualen Mengenangaben auf Aktivgehalte.

Ein erster Gegenstand der Erfindung richtet sich auf ein Mittel enthaltend mindestens ein alkoxyliertes Tensid (A) aus der Gruppe der Polyoxyalkylen Carboxylate (I oder II) und mindestens ein Glycolipid-Biotensid (B) umfassend Rhamnolipide, Sophorolipide, Trehaloselipide, Mannosylerythritollipide, und Cellobioselipide, wobei das Polyoxyalkylen Carboxylat in einem Verhältnis von ≥ 1: 6 zum Biotensid vorliegt; bezogen auf Gewichtsprozent Aktivstoff im gesamten Mittel;.

Verhältnis (A): (B)

Das oder die Tenside (A) und das oder die Glycolipid-Biotenside (B) liegen in einem Verhältnis (A): (B) ≥ 1: 6 vor, zum Beispiel 1:6, 1:5, 1:4, 1:3, 1:2, 1:1, 2:1, 3:1, u.s.w. Bevorzugt liegen die Tenside (A) zu den Glycolipid-Biotensiden (B) in einem Verhältnis zwischen (A) :(B) ≤ 10:1 und ≥1:6 vor;
besonders bevorzugt liegt (A) :(B) ≤ 10:1 und ≥1:3;
äusserst bevorzugt liegt (A) :(B) ≤ 10:1 und ≥1:2; jeweils bezogen auf Gewichtsprozent Aktivgehalt im gesamten Mittel.

**Tensid (A) : Polyoxyalkylen Carboxylat bzw. Carbonsäure**

Polyoxyethylen Carboxylate liegen vorzugsweise als Gemische vor und folgen bevorzugt der Formel (I). Weiterhin kann das Produktgemisch Nebenkomponenten enthalten, wie beispielsweise freie Polyoxyalkylenketten, weitere Glyceride, Edukte u.a.

$$R'\text{-}O(C_bH_{2b}O)_o\text{-}\{CH_2\text{-}CH[O(C_bH_{2b}O)_pR'']\text{-}CH_2O\}_r\text{-}(C_bH_{2b}O)_q\text{-}R''' \qquad (I)$$

mit

**b** einer ganzen Zahl zwischen 2 und 4, bevorzugt 2 oder 3, besonders bevorzugt 2,

**o, p, q** sind unabhängig voneinander Zahlen von 0 bis 75, wobei o+p+q mindestens 2 ist. Die Zahlen o, p, q repräsentieren den Ethoxylierungsgrad. Obwohl diese Zahlen auf molekularer Ebene nur ganze Zahlen einschliesslich der Null annehmen können, kann der Gesamtethoxylierungsgrad x als Dezimalzahl angegeben werden, da dieser die stöchiometrische Äquivalente von Butylen-, Propylen- oder Ethylenoxid darstellt,

**r** ist 0 oder 1,

**R', R" und R"'** sind jeweils unabhängig voneinander H, $CH_2COOM$, oder COR, wobei einer oder zwei, bevorzugt einer der Reste R', R" und R"' $CH_2COOM$ darstellen: Wenn R' = $CH_2COOM$ dann gilt o≠0, wenn R" = $CH_2COOM$ dann gilt p≠0, wenn R"'= $CH_2COOM$ dann gilt q≠0, mit **M** = H, Alkali- oder Ammoniumkation, und wobei einer oder zwei der Reste R', R" und R'" RCO darstellen;

mit **R** einer gesättigten, ein- oder mehrfach ungesättigten Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt;

und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (I);

und wobei das Tensid (I) aus einem Gemisch unterschiedlicher Kettenlängen und Sättigungsgrade des Fettsäurerests RCO wie oben definiert besteht und abgeleitet ist von einem C-18-Pflanzenöl aus der Gruppe umfassend: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Moringa, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen.

Bevorzugt liegt der Fettsäurerest RCO mit einem Anteil von 20 oder mehr Kohlenstoffatomen bevorzugt > 0.01 Gew.-%, besonders bevorzugt > 0.05 Gew.-% und ganz besonders bevorzugt ≥ 0.1 Gew.-% beträgt und äusserst bevorzugt >= 0.2 Gew.-%.

Bevorzugt liegt der der Anteil an Fettsäurerest RCO mit Fettsäuren von 16 und weniger Kohlenstoffatomen unter 30 Gew.-%, bevorzugt unter 27 Gew.% und besonders bevorzugt unter 17 Gew.%;

bevorzugt liegt der der Anteil an Fettsäurerest RCO mit Fettsäuren von 6 und weniger Kohlenstoffatomen < 0.5%, besonders bevorzugt < 0.05%;

bevorzugt liegt der der Anteil an Hydroxyfettsäurerest < 75 Gew-%, bevorzugt < 25 Gew.-%, besonders bevorzugt < 5 Gew.-%;

bevorzugt kann der Anteil an Fettsäurerest RCO mit 20 und mehr Kohlenstoffatomen bis zu 96 Gew.-% betragen kann;

bevorzugt liegt der der Anteil des Ölsäureacylrestes RCO bei weniger als 95 Gew.-%, besonders bevorzugt unter 85 Gew.-%, jeweils bezogen auf den Gesamtanteil an Fettsäureresten RCO des eingesetzten Tensids (I);

Erfindungsgemässe Tenside dieser Klasse werden bevorzugt erhalten durch die dem Fachmann bekannte Reaktion von Monochloressigsäure an einer terminalen Hydroxylgruppe eines alkoxylierten Fettsäureesters, eines alkoxylierten Alkyl Glycerids, bevorzugt eines Mono- oder Diglycerids, eines alkoxylierten Polyglycerids, oder eines alkoxylierten C-18-Pflanzenöls, oder deren Gemische und anschliessend mit einer Lauge neutralisiert.

Bevorzugt werden die erhaltenen Produktgemische für b=2 allgemein bezeichnet als (II) oder (III):

Metall PEG-x Pflanzenöl Carboxylat (II)
Metall PEG-x Pflanzenglycerid Carboxylat oder Metall Pflanzenöl
Glycereth-x carboxylat (III)

Mit Metall = Alkalimetallkation, Erdalkalimetallkation, Ammoniumkation (i.e. Carboxylat) oder H (i.e. Carbonsäure)

mit Ethoxylierungsgrad x = 2-75, bevorzugt 2-20, besonders bevorzugt 2-8

Beispielhafte Vertreter der erfindungsgemäss geeigneten Produkte sind

Natrium PEG-6 Mandelöl Carboxylat, Natrium PEG-8 Mandelöl Carboxylat, Natrium PEG-8 Aprikosenkernöl Carboxylat, Natrium PEG-8 Buxus Chinensis Oil Carboxylat, Natrium PEG-6 Aprikosenkernöl Carboxylat, Natrium PEG-40 Aprikosenkernöl Carboxylat, Natrium PEG-8 Arganöl Carboxylat, Natrium PEG-8 Avocadoöl Carboxylat, Natrium PEG-11 Avocadoöl Carboxylat, Natrium PEG-8 Borretschsamenöl Carboxylat, Natrium PEG-8 Macademia Tenuifolia Öl Carboxylat, Natrium PEG-6 Maisöl Carboxylat, Natrium PEG-8 Maisöl Carboxylat, Natrium PEG-8 Traubenkernöl Carboxylat, Natrium PEG-8 Haselnussöl Carboxylat, Natrium PEG-8 Leinsamenöl Carboxylat, Natrium PEG-6 Olivenöl Carboxylat, Natrium PEG-7 Olivenöl Carboxylat, Kalium PEG-7 Olivenöl Carboxylat, Natrium PEG-8 Olea Europaea Öl Carboxylat, Ammonium PEG-7 Olivenöl Carboxylat, PEG-7 Olivenöl Carbonsäure, Natrium PEG-8 Olivenöl Carboxylat, Natrium PEG-10 Olivenöl Carboxylat, Natrium PEG-8 Oryza Sativa Öl Carboxylat, Natrium PEG-8 Prunus Dulcis Carboxylat, Natrium PEG-8 Persea Gratissma Öl Carboxylat, Natrium PEG-8 Passiflora edulis seed oil Carboxylat, Natrium PEG-6 Erdnussöl Carboxylat, Natrium PEG-45 Crambe Absyssinica Seed oil Carboxylat, Natrium PEG-75 Wiesenschaumkrautöl Carboxylat, Natrium PEG-8 Kürbiskernöl Carboxylat, Natrium PEG-3 Rapssamenöl Carboxylat, Natrium PEG-20 Rapssamenöl Carboxylat, Natrium PEG-8 Diestelöl Carboxylat, Natrium PEG-8 Schinziophyton Rautaneii Kernöl Carboxylat, Natrium PEG-8 Sesamsamenöl Carboxylat, Natrium PEG-8 Senum Indicum Öl Carboxylat Natrium PEG-8 Sojabohnenöl Carboxylat, Natrium PEG-20 Sojabohnenöl Carboxylat, Natrium PEG-36 Sojabohnenöl Carboxylat, Natrium PEG-8 Sonnenblumenöl Carboxylat, Natrium PEG-32 Sonnenblumenöl Carboxylat, Natrium PEG-8 Süssmandelöl Carboxylat, Natrium PEG-8 Wassermelonenkernöl Carboxylat, Natrium PEG-8 Weizenkeimöl Carboxylat, Natrium PEG-8 Zea Mais Öl Carboxylat,

Natrium PEG-6 Mandelglycerid Carboxylat, Natrium Mandelöl Glycereth-8 Carboxylat, Carboxylat Natrium PEG-20 Mandelglycerid Carboxylat, Kalium PEG-35 Mandelglycerid Carboxylat, Ammonium PEG-60 Mandelglycerid Carboxylat, Natrium Avocadoöl Glycereth-8 Carboxylat, Natrium PEG-11 Avocadoglycerid Carboxylat, Natrium Arganöl Glycereth-8 Carboxylat, Natrium Mandelöl Glycereth-8 Carboxylat, Natrium PEG-14 Mandelglycerid Carboxylat, Natrium Maisöl Glycereth-8 Carboxylat, Natrium PEG-20 Maisglycerid Carboxylat, Natrium PEG-60 Maisglycerid Carboxylat, Natrium PEG-20 Nachtkerzenglycerid Carboxylat, Natrium PEG-60 Nachtkerzenglycerid Carboxylat, Natrium Traubenkernöl Glycereth-8 Carboxylat, Natrium Cannabis Sativa Kernöl Glycereth-8 Carboxylat, Natrium Jojobaöl Glycereth-8 Carboxylat, Natrium PEG-16 Macademiaglycerid Carboxylat, Natrium PEG-25 Moringaglycerid Carboxylat, Natrium PEG-2 Olivenglycerid Carboxylat, Natrium PEG-6 Olivenglycerid Carboxylat, Natrium PEG-7 Olivenglycerid Carboxylat, Natrium Olivenöl Glycereth-8 Carboxylat, Natrium PEG-10 Olivenglycerid Carboxylat, Natrium PEG-40 Olivenglycerid Carboxylat, Natrium Pfirsichkernöl Glycereth-8 Carboxylat, Natrium PEG-60 Passiflora edulis seed glycerid Carboxylat, Natrium PEG-60 Passiflora Incarnata seed glycerid Carboxylat, Natrium PEG-40 Diestelglycerid Carboxylat, Natrium Sojaöl Glycereth-8 Carboxylat, Natrium PEG-35 Soja glycerid Carboxylat, Natrium PEG-75 Soja glycerid Carboxylat, Natrium PEG-2 Sonnenblumenglycerid Carboxylat, Natrium PEG-7 Sonnenblumenglycerid Carboxylat, Natrium Sonnenblumenöl Glycereth-8 Carboxylat, Natrium PEG-10 Sonnenblumenglycerid Carboxylat, Natrium PEG-13 Sonnenblumenglycerid Carboxylat, Natrium PEG-7 Rapsglycerid Carboxylat, Natrium PEG-4 Rapsglycerid Carboxylat, Natrium PEG-10 Canolaglycerid Carboxylat, Natrium PEG-5 Tsubakiateglycerid Carboxylat, Natrium PEG-10 Tsubakiateglycerid Carboxylat, Natrium PEG-20 Tsubakiateglycerid Carboxylat, Natrium PEG-60 Tsubakiateglycerid Carboxylat, Natrium Baumwollöl Glycereth-8 Carboxylat, Natrium Reisöl Glycereth-8 Carboxylat, Natrium Sesamöl Glycereth-8 Carboxylat, Natrium Weizenkeimöl Glycereth-8 Carboxylat.

In dieser Erfindung besonders bevorzugt sind die Vertreter der Tenside mit der allgemeinen Bezeichnung Metall Pflanzenöl PEG-n Carboxylat,
Mit Metall = Na, K, H, Ammonium;
n=ganze Zahlen zwischen 2 und 8,
und den bevorzugten Pflanzenölen ausgewählt aus der Gruppe Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen.

Besonders bevorzugt sind Vertreter mit einem HLB > 10.5 und <12.0.

Ganz besonders bevorzugt ist der Vertreter, INCI Name: Natrium Olivenöl PEG-7 Carboxylat bzw. Sodium PEG-7 olive oil carboxylate, IUPC-Name Poly(Oxy-1,2-Ethandiyl), .Alpha.-Carboxy-.Omega.-(Olivenölfettsäuren)Oxy-, Natriumsalz mit 7 mol EO durchschnittlichem EO-Gehalt), HLB = 11, erhältlich von der Fa. Hallstar unter dem Handelsnamen Olivem 400.

Dieses ist erfindungsgemäss besonders bevorzugt, da es zusätzlich über eine gute biologische Abbaubarkeit verfügt (>95% nach OECD) und hervorragende dermatologische Eigenschaften hat. Bei vergleichbaren Konzentrationen weist es gemäss Herstellerangaben einen deutlich geringeren Hautirritationsfaktor als das üblicherweise verwendete Natrium Laureth sulfat (1.0 = minimum irritant vs. 1.64 = mild irritant).

Verwendung von Fettsäuregemischen

Im Sinne dieser Anmeldung liegt dem alkoxylierten Tensid (A) eine Mischung von Fettsäurederivaten auf Basis von C18-Pflanzenölen mit unterschiedlicher Kettenlänge und Sättigungsgrad zugrunde. Die Mischung folgt bevorzugt der Fettsäureverteilung im nativen Öl oder wie sie bei der Umsetzung von natürlich vorkommenden Pflanzenöle oder Fette anfallen. Indem für die Synthese der Tensidklasse Gemische von Fettsäurederivaten verwendet werden - wie sie bei der Umsetzung von natürlich vorkommenden Pflanzenöle oder Fette anfallen - können die Tenside kostengünstig, ressourceneffizient und umweltschonend produziert werden. Zusätzliche Reinigungsverfahren, wie z.B. die Trennung der Fettsäuren, bzw. Fettsäureester durch fraktionierte Destillation oder zusätzliche Syntheseschritte, wie z.B. zum Fettalkohol, werden hier nicht benötigt. Neben den ökologischen und ökomischen Vorteilen der Verwendung von natürlichen Fettsäuregemischen, zeigen die verwendeten Tensidmischungen eine erhöhte Reinigungsleistung.

Die Mittel enthalten bevorzugt 0.1 bis 50 Gew.-% eines oder mehrere alkoxylierter Tenside (A), bevorzugter 0.25 bis 25 Gew-%, besonders bevorzugt 0.6 bis 10 Gew.-% und ganz besonders bevorzugt 0.6 Gew.-% bis 8 Gew.-%.

**Biotenside allgemein**

Als Biotenside werden allgemein Tenside biologischen Ursprungs bezeichnet. Sie erfahren keine chemische Umsetzung wie die synthetischen Glycolipide z.B. Alkylpolyglycoside (APG). Biotenside können in lebenden Organismen vorkommen, oder entstehen bei der Kultivierung diverser Mikroorganismen wie z.B. Pilze, Hefen, Algen, Viren oder Bakterien oder Enzymen.

Glycolipid- Biotenside können unmittelbar durch die Verwendung von natürlichen Rohstoffen durch mikrobiologische Prozesse gewonnen werden, während die Herstellung von synthetischen Glycolipide meist weitere chemische Schritte, wie bspw. die Reduktion der Fettsäure in den Fettalkohol bedingt. Durch die biologische Modifizierung des Zuckerteils weisen Glycolipid-Biotenside spezielle Eigenschaften auf, wie zum Beispiel dem Fachmann bekannt, ein gutes Fettlösevermögen. Die Struktur der Glycolipid- Biotenside sowie die Kettenlängen des hydrophoben Teils variiert je nach verwendeten Mikroorganismus bzw. Substrat.

Im Rahmen dieser Erfindung umfassen Glycolipid-Biotenside (B) Rhamnolipide, insbesondere Mono-, Di- oder Polyrhamnolipide, Sophorolipide in ihrer Säure- oder Laktonform oder als deren Gemische, diacetyliert, acetyliert oder nicht-acetyliert, Trehaloselipiden, Mannosylerythritollipide und Cellulobioselipide.

Als Substrate für Glycolipid- Biotenside eignen sich die unterschiedlichsten, literaturbekannten Kohlenstoffquellen, wie z.B. Pflanzenöle und den daraus erhaltenen Glyceriden oder Fettsäuremethylestern, Fettsäuren, Fettalkohole, Fettsäuremethylester oder -ethylester, Kohlenhydrate, z.B. Cellulose, Glucose, Stärke, C4-Quellen wie Succinate, Butan, Buttersäure, C1-Quellen wie $CO_2$, CO oder Methan, öl- und/oder kohlenhydrathaltige Abwässer aus Raffinerien oder der Lebensmittelindustrie und auch Mischungen derselben.

## Biotensid Mikroorganismen

Bevorzugte Mikroorganismen zur Herstellung der Biotenside sind Bacillus, Candida, Pseudomonas, Trichosporan und/oder Pseudozyma Stämme und weitere wie in einschlägiger Literatur beschrieben, insbesondere Arthrobacter spec., Bacillus licheniformis, Bacillus subtilis, Burkholderia, Candida apicola, Candida antarctica, Candida batistae, Candida bogoriensis, Candida bombicola, Candida sp., Cryptococcus humicola, Gordonia, Mycobacterium tuberculosis, Nocardia cornynebacterium, Pichia anomala, Pseudomonas aeruginosa, Pseudozyma aphidis (MEL), Pseudomonas sp, Pseudozyma antarctica (MEL), Pseudozyma parantarctica, Pseudozyma fusiformata, Rhodococcus, Rhodococcus erythropolis, Rhodotorula bogoriensis, Sphingomonas sp. NM05, Starmerella bombicola, Trichosporon cutaneum, Trichosporan loubieri; Torulopsis magnoliae, Torulopsis bombicola, Tsukamurella spec., Yarrowia alipolytica, Yarrowia lipolytica, Ustilago maydis (MEL), Wickerhamiella domercqiae Y2A.

Die Glycolipid-Biotenside können z. B. wie in EP 0 499 434, US 7,985 722, WO 03/006146, JP 60-183032, DE 19648439, DE 19600743, JP 01-304034, CN 1337439, JP 2006- 274233, KR 2004033376, JP 2006-083238, JP 2006-070231 , WO 03/002700, FR 2740779, DE 2939519, US 7,556,654, FR 2855752, EP 1445302, JP 2008-062179 und JP 2007-181789 , Mannosylerythritol: WO 2004/020647, JP 20042544595, oder den dort zitierten Schriften hergestellt werden.

## Biotenside Strukturen

### Rhamnolipide

Unter dem Begriff Rhamnolipid im Zusammenhang mit der vorliegenden Erfindung werden insbesondere Verbindungen der allgemeinen Formel (IV) oder deren Salze verstanden,

(IV)

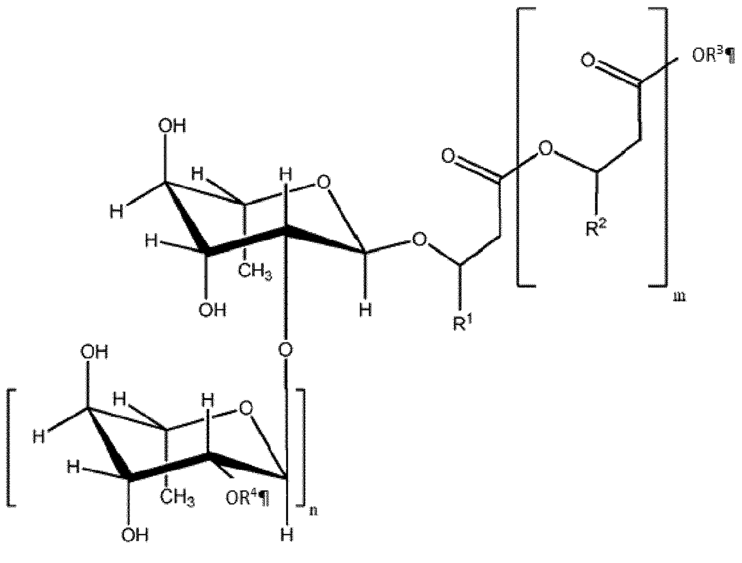

wobei

- m = 2, 1 oder 0,
- n = 1 oder 0,
- $R^1$ und $R^2$ = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter Kohlenwasserstoffrest, bevorzugt ausgewählt aus der Gruppe bestehend aus Pentenyl, Heptenyl, Nonenyl, Undecenyl und Tridecenyl und $(CH_2)_o$-$CH_3$ mit o = 1 bis 23, bevorzugt 4 bis 12, ganz besonders bevorzugt o = 6.
- $R^3$ - H, $CH_3$, Kation, insbesondere Alkalikation oder H, bevorzugt H
- $R^4$ - H oder die Gruppe $CH_3(CH_2)_aCH$=CH-CO, mit a als Zahlen zwischen 4 und 10, bevorzugt H,

Unter dem Begriff "di-Rhamnolipid" im Zusammenhang mit der vorliegenden Erfindung werden Verbindungen der allgemeinen Formel (IV) oder deren Salze verstanden, bei denen n =1.
Unter dem Begriff "mono-Rhamnolipid" im Zusammenhang mit der vorliegenden Erfindung werden Verbindungen der allgemeinen Formel (IV) oder deren Salze verstanden, bei denen n =0.
Rhamnolipide sind über Organismen wie Pseudomonas und Burkholderia, beides Wildtypen zugänglich. Desweiteren werden sie über rekombinante Zellen hergestellt. Kommerziell erhältlich sind Rhamnolipide bei der Firma Evonik unter dem Handelsnamen Rewoferm®, The Gene Biotech oder bei Jeneil Biosurfactant Co.LLC.
Besonders bevorzugt in dieser Erfindung sind die Rhamnolipide der Firma Evonik Rewoferm® und JBR 425 von Jeneil Biosurfactant, insbesondere JBR 425.

**Sophorolipide**

Unter dem Begriff Sophorolipid im Zusammenhang mit der vorliegenden Erfindung werden insbesondere Verbindungen der offenen Form oder Säureform, der Ring- oder Laktonform, oder deren Gemische verstanden, vorzugsweise Formel (V) folgend

(V)

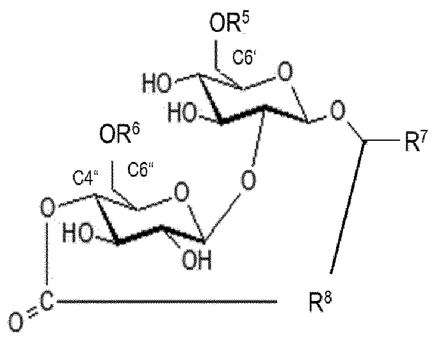

Ringform

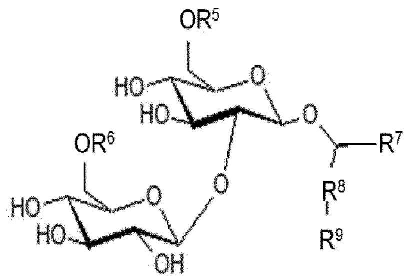

Offene Form

Wobei

- $R^5$ und $R^6$ unabhängig voneinander H oder Acetylgruppen sind
- $R^7$ H oder eine reine gesättigte oder ungesättigte, hydroxylierte oder nicht-hydroxylierte Kohlenwasserstoffkette mit 1-9 Kohlenstoffatomen ist, bevorzugt $CH_3$
- $R^8$ eine gesättigte oder ungesättigte, hydroxylierte oder nicht-hydroxylierte, lineare oder verzweigte Kohlenwasserstoffkette mit 1-22 Kohlenstoffatomen ist, bevorzugt besteht $R^8$ aus einer gesättigten Kohlenwasserstoffkette aus 11 bis 22 Kohlenstoffatomen oder aus einer einfach oder zweifach ungesättigten Kohlenwasserstoffkette aus 13 bis 22 Kohlenstoffatomen, besonders bevorzugt aus einer einfachen oder zweifach ungesättigten Kohlenwasserstoffkette aus 15 oder 16 Kohlenstoffatomen stammend aus Rapsöl als Substrat.
- $R^9$ ist COOH oder ein kationisches Salz desselben, oder $COO(CH_2)_mCH_3$ mit m zwischen 0 und 3,

In der Laktonkonfiguration kann die Laktonbildung der Carboxylgruppe mit Hydroxylgruppe an C4", wie hier exemplarisch in Formel (V) dargestellt, oder alternativ an C6" oder C6' erfolgen, an der C4" Position befindet sich dann eine Hydroxylgruppe.
Zusätzlich können die Sophorolipide Verunreinigungen enthalten, wie Fettalkohole, Fettsäureester, Fettsäuren, Triglyceride oder Öle, Zucker, besonders Glucose, Sophorose, oder organische Säuren.

Durch Verwendung unterschiedlicher Substrate kann das Biotensid strukturell modifiziert werden, z.B. kann das Verhältnis Laktonform zu freier Säure durch das Substrat gesteuert werden.

Erfindungsgemäss können unterschiedlichste Substrate verwendet werden, wie in C. Mulligan, Biosurfactants: Research Trends and Application, CRC Press 2014, S. 112. Beispiele für bevorzugten Substrate für Sophorolipide aus der Literatur sind: Maisöl (EP 0282942), Öl von C22 Fettsäuren (KR20100022289), Olivenöl (ES 2018637, CN 1431312), Rapsöl oder -ester (CN 102250790, US2008032383), Sonnenblumen- oder Rapsfettsäureester (DE4319540, FR 2692593) oder Abfallöle bzw. -fette (JP 2004254595, JP 2007252279, CN 101845468, CN 101948786).

Sophorolipide sind durch eine Vielzahl unterschiedlicher dem Fachmann bekannten Organismen wie Candida bombicola, Starmerella oder Wickerhamiella zugänglich.

Weiterhin sind sie kommerziell erhältlich, z.B. von der Firma Evonik z.B. unter dem Handelsnamen Rewoferm SL 446 oder von Soliance/Givaudan.

Besonders bevorzugt in dieser Erfindung sind die Sophorolipide mit den Handelsnamen Sophogreen, Sophoclean, Soliance S.

## Mannosylerythritollipide

Unter dem Begriff Mannosylerythritollipide im Zusammenhang mit der vorliegenden Erfindung werden insbesondere Verbindungen der allgemeinen Formel (VI) oder deren Salze verstanden,

(VI)

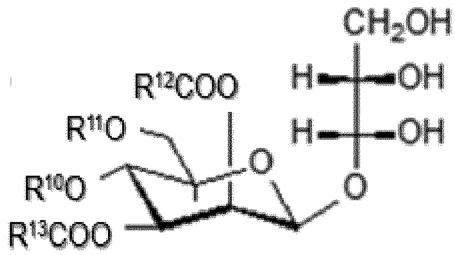

wobei

$R^{10}$ und $R^{11}$ unabhängig voneinander H oder $-COCH_3$

$R^{12}$ und $R^{13}$ unabhängig voneinander, lineare oder verzweigte $C_1$ bis $C_{23}$, vorzugsweise $C_1$ bis $C_{17}$, und besonders bevorzugt $C_7$ bis $C_{15}$Alkylgruppen; oder lineare oder verzweigte $C_2$ bis $C_{23}$, bevorzugt $C_2$ bis $C_{17}$, und besonders bevorzugt $C_7$ bis $C_{15}$ Alkenylgruppen; oder lineare oder verzweigte $C_5$ bis $C_{23}$, vorzugsweise $C_5$ bis $C_{17}$, und besonders bevorzugt $C_7$ bis $C_{15}$Alkadienylgruppen; oder lineare oder verzweigte $C_8$ bis $C_{19}$, bevorzugt $C_8$ bis $C_{17}$, und besonders bevorzugt $C_8$ bis $C_{15}$ Alkatrienylgruppen.

Mannosylerythritollipide können durch Pseudozyma Antarctica NBRC 10736 in einem Nährmedium mit Sojaöl erhalten werden, alternativ sind sie durch den Brandpilz Ustilago maydis zugänglich.

## Cellobiose Lipid

Unter dem Begriff Cellobioselipid im Zusammenhang mit der vorliegenden Erfindung werden insbesondere Verbindungen der allgemeinen Formel (VII) oder deren Salze verstanden

(VII)

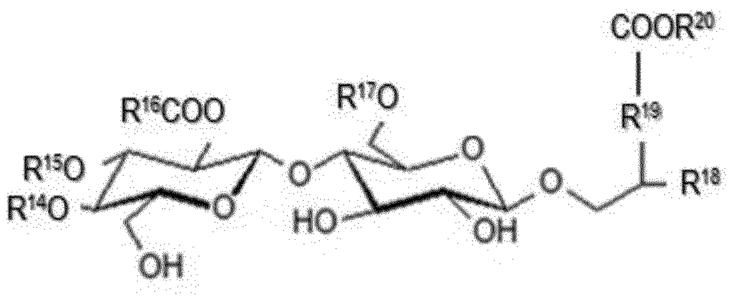

$R^{14}$, $R^{15}$ und $R^{17}$= unabhängig voneinander H oder $-COCH_3$,

$R^{16}$ = gesättigter oder ungesättigter, hydroxylierter oder nicht-hydroxylierter Kohlenwasserstoff mit 5 bis 23 Kohlenstoffatomen, oder $-CH_3$, bevorzugt - $CH_3$ oder - $CH_2$-CH(OH)-$(CH_2)_n$-$CH_3$ mit n= 2-4,

$R^{18}$= H oder-OH,

$R^{19}$= gesättigter oder ungesättigter, hydroxylierter oder nicht-hydroxylierter Kohlenwasserstoff mit 9 bis 21 Kohlenstoffatomen, bevorzugt 13 Kohlenstoffatome, besonders bevorzugt - $(CH_2)_n$-CH(OH)- mit n= 12,

$R^{20}$= H, oder ein Kation.

Cellobioselipide können durch literaturbeschrieben Verfahren durch Crypotcoccus humicola, Pseudozyma fusiformata oder durch den Brandpilz Ustilago maydis erhalten werden.

## Trehaloselipide

Unter dem Begriff Trehaloselipide im Zusammenhang mit der vorliegenden Erfindung werden insbesondere Verbindungen der allgemeinen Formel (VIII) oder deren Salze verstanden.

(VIII)

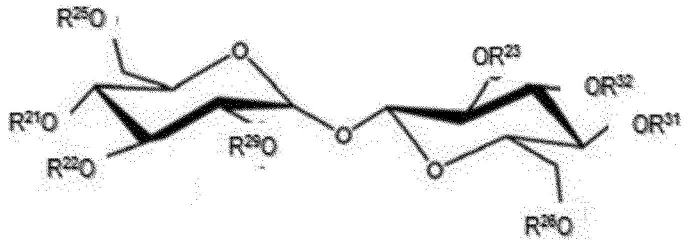

$R^{21}$, $R^{22}$, $R^{31}$ und $R^{32}$= jeweils unabhängig voneinander Wasserstoff oder $COR^{27}$ mit $R^{27}$ gesättigtem oder ungesättigtem, hydroxyliertem oder nicht-, hydroxyliertem Kohlenwasserstoff mit 5 bis 23 Kohlenstoffatomen;

$R^{25}$ und $R^{26}$ jeweils unabhängig voneinander Wasserstoff oder $COR^{28}$ mit $R^{28}$ gesättigtem oder ungesättigtem, verzweigtem oder nicht-verzweigtem, hydroxyliertem oder nicht-, hydroxyliertem Kohlenwasserstoff mit 5 bis 23 Kohlenstoffatomen oder $R^{28}$ = $CH[(CH_2)_cCH_3]CHOH(CH_2)_dCH_3$ mit c+d = 27;

$R^{29}$ und $R^{23}$ jeweils unabhängig voneinander Wasserstoff, $COR^{30}$ mit $R^{30}$ gesättigtem oder ungesättigtem, verzweigt oder nicht-verzweigtem, hydroxyliertem oder nicht-, hydroxyliertem Kohlenwasserstoff mit 5 bis 23 Kohlenstoffatomen oder $R^{30}$ = $(CH)_2COOR^{24}$ mit $R^{24}$= H oder Kation.

Erfindungsgemäss eignen sich vorzugsweise Tetralose Dimycolate, Tetralose Monomycolate, Succinyl Trehalose Lipide und Trehalose Tetraester oder Gemische derselben.

Trehaloselipide können durch literaturbeschriebene Verfahren durch Rhodococcus erythropolis, Arthobacter spec., Gordonia, Mycobacterium tuberculosis, Nocardia Cornynebacterium spec. oder Tsukamurella spec. erhalten werden.

Die Zubereitungen enthalten bevorzugt 0.1 bis 50 Gew.-% eines oder mehrerer Glycolipid-Biotenside, bevorzugter 0.1 bis 25 Gew.-%, besonders bevorzugt 0.1 bis 15 Gew.-% und ganz besonders bevorzugt 0.3 bis 10 Gew.-%, bezogen auf die Gesamtmenge der Zubereitung.

## Weitere Tenside

Ein weiterer Gegenstand der Erfindung richtet sich auf ein Mittel zusätzlich enthaltend mindestens eine Seife (C), und/ oder ein oder mehrere nichtionisches alkoxylierte Tenside (D) ausgewählt der Gruppe der alkoxylierten Fettsäureester, der alkoxylierten Fettsäureglyceridester, oder der alkoxylierten Pflanzenölester, der alkoxylierten Fettsäureamide sowie Mischungen derselben.

Überraschenderweise zeigen die Kombinationen (A), (B) und (C), oder (A), (B) und (D) oder (A), (B), (C), (D) eine unverändert hohe oder sogar bessere Reinigungskraft auf Kohlenhydrat- oder Farbflecken.

Dies ist insofern erstaunlich, da die Kombination (A)/(B) auch mit einem dritten Tensid (C) bzw. (D) synergistisch auf Kohlenhydratflecken oder Farbflecken wirkt.

**Seifen (C)**

In einer weiteren Ausführungsform, hat es sich als vorteilhaft erwiesen, wenn das Mittel zusätzlich mindestens eine Seife (C) enthält. Seifen sind Alkali- oder Ammoniumsalze gesättigter oder ungesättigter Fettsäuren von 6 bis 24 Kohlenstoffatomen.

Überraschenderweise können die Mittel bestehend aus dem oder den Tensiden (A) und dem oder den Glycolipid-Biotensiden (B) bei neutralem oder alkalischem pH zusätzlich mit einer Seife (C) kombiniert werden, so dass das Gesamtergebnis weiter verbessert wird -mit einer hohen Reinigungskraft auf Kohlenhydratflecken oder Farbflecken.Weiterhin kann die Zugabe Seife für zusätzliche Eigenschaften der Formulierung wie Konsistenz, Stabilität, Weichgriff und nicht zuletzt den Kosten wünschenswert sein. Wie in den Ausführungsbeispielen gezeigt, hat die Zugabe von Seife je nach Ausführungsform positive Auswirkungen auf die Reinigungsleistung.

Besonders bevorzugt in dieser Erfindung sind Salze von Fettsäuren der Formel (IX)

RCOOM (IX)

M ist ein Alkali- oder Ammoniumkation
R bzw. RCO wie in Formel (I) definiert und abgeleitet von C18-Pflanzenölen, d.h.
mit **R** einer gesättigten, ein- oder mehrfach ungesättigten Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt; und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (I); und wobei das Tensid (I) aus einem Gemisch unterschiedlicher Kettenlängen und Sättigungsgrade des Fettsäurerests RCO wie oben definiert besteht und abgeleitet ist von einem C-18-Pflanzenöl.

Beispielhafte Vertreter der erfindungsgemäss geeigneten Produkte sind
Natrium Olivenölseifen, Kalium Rapsölseifen, Ammonium Distelölseifen, Natrium Leinsamenölseifen, Natrium Sonnenblumenölseifen, Natrium Sojaölseifen, und andere.

Bevorzugt liegt die Seife (C) als Mischung gemäss der Fettsäureverteilung im nativen Öl vor oder wie sie bei der Umsetzung von natürlich vorkommenden Pflanzenölen oder -fetten anfallen.

In dieser Ausführungsform bevorzugt sind Mittel der Formel (XI), die dadurch gekennzeichnet sind, dass sie zu 0.001 bis 50 Gew.-%, bevorzugter 0.01 bis 20 Gew.-% und besonders bevorzugt 0.1 bis 20 Gew.-% und ganz besonders bevorzugt 1 bis 10 Gew.-% enthalten sind; Gewichtsprozent bezogen auf das Gesamtmittel.

**Nichtionische Tenside (D)**

In einer weiteren Ausführungsform, hat es sich als vorteilhaft erwiesen, wenn das Mittel zusätzlich ein oder mehrere nichtionisches alkoxylierte Tenside (D) enthält, ausgewählt der Gruppe der alkoxylierten Fettsäureester, der alkoxylierten Fettsäureglyceridester, der alkoxylierten Pflanzenölester dargestellt durch die Formel (X) und der alkoxylierten Fettsäureamide dargestellt durch die Formel (XI) sowie Mischungen derselben.

(X) $$RCO\text{-}O(C_mH_{2m}O)_o\text{-}\{CH_2\text{-}CH[O(C_mH_{2m}O)_pR^{33}]\text{-}CH_2O\}_w\text{-}(C_mH_{2m}O)_q\text{-}R^{34}$$

mit R und RCO wie bei Formel (I) beschrieben und abgeleitet von einem C-18-Pflanzenöl
wobei
m 2, 3 oder 4 ist, bevorzugt 2 oder 3, besonders bevorzugt 2;
o, p, q sind unabhängig voneinander Zahlen von 0 bis 75, bevorzugt 0-10, bevorzugter 0-8, wobei $o+p+q \neq 0$. Die Zahlen o, p, q repräsentieren den Alkoxylierungsgrad. Obwohl diese Zahlen auf molekularer Ebene nur ganze Zahlen einschliesslich der Null annehmen können, kann der Gesamtalkoxylierungsgrad x als Dezimalzahl angegeben werden, da dieser die stöchiometrischen Äquivalente von Butylenoxid, Propylenoxid oder Ethylenoxid darstellt,
$R^{33}$ ist H, oder COR,
$R^{34}$ ist H, COR, oder ein linearer oder verzweigter Alkylrest mit 1-8 C-Atomen.
w ist eine ganze Zahl zwischen 0 und 10;

(XI) $R\text{-}CO\text{-}NH\text{-}(C_mH_{2m}O)_n\text{-}H$

mit R und RCO wie bei Formel (I) beschrieben und abgeleitet von einem C-18-Pflanzenöl,
und wobei
m die ganze Zahlen 2 oder 3 ist, bevorzugt 2
n Zahl im Bereich von 2-10, bevorzugt im Bereich 2-8, ganz besonders bevorzug 2-4.

Überraschenderweise können die Mittel bestehend aus dem oder den Tensiden (A) und dem oder den Glycolipid-Biotensiden (B) bei beliebigem pH-Wert zusätzlich mit einem Tensid (D) kombiniert werden.
Es ist insbesondere erstaunlich, dass die Kombination (A)/(B) trotz Zugabe eines dritten Tensids (D) synergistisch auf Kohlenhydratflecken oder Farbflecken wirkt.

Beispielhafte Vertreter der erfindungsgemäss geeigneten Tenside (D) sind

PEG-6 Mandelöl, PEG-8 Mandelöl, PEG-8 Aprikosenkernöl, PEG-8 Buxus Chinensis Oil, PEG-6 Aprikosenkernöl, PEG-40 Aprikosenkernöl, PEG-8 Arganöl, PEG-8 Avocadoöl, PEG-11 Avocadoöl, PEG-8 Borretschsamenöl, PEG-8 Macademia Tenuifolia Öl, PEG-6 Maisöl, PEG-8 Maisöl, PEG-8 Traubenkernöl, PEG-8 Haselnussöl, PEG-8 Leinsamenöl, PEG-6 Olivenöl, PEG-7 Olivenöl, PEG-7 Olivenöl, PEG-8 Olea Europaea Öl, PEG-7 Olivenöl, PEG-7 Olivenöl, PEG-8 Olivenöl, PEG-10 Olivenöl, PEG-8 Oryza Sativa Öl, PEG-8 Prunus Dulcis, PEG-8 Persea Gratissma Öl, PEG-8 Passiflora edulis seed oil, PEG-6 Erdnussöl, PEG-45 Crambe Absyssinica Seed oil, PEG-75 Wiesenschaumkrautöl, PEG-8 Kürbiskernöl, PEG-3 Rapssamenöl, PEG-20 Rapssamenöl, PEG-8 Diestelöl, PEG-8 Schinziophyton Rautaneii Kernöl, PEG-8 Sesamsamenöl, PEG-8 Senum Indicum Öl, PEG-8 Sojabohnenöl, PEG-20 Sojabohnenöl, PEG-36 Sojabohnenöl, PEG-8 Sonnenblumenöl, PEG-32 Sonnenblumenöl, PEG-8 Süssmandelöl, PEG-8 Wassermelonenkernöl, PEG-8 Weizenkeimöl, PEG-8 Zea Mais Öl
PEG-6 Mandelglycerid, Mandelöl Glycereth-8 Ester, PEG-20 Mandelglycerid, PEG-35 Mandelglycerid, PEG-60 Mandelglycerid, Avocadoöl Glycereth-8 Ester, PEG-11 Avocadoglycerid, Arganöl Glycereth-8 Ester, Mandelöl Glycereth-8 Ester, PEG-14 Mandelglycerid, Maisöl Glycereth-8 Ester, PEG-20 Maisglycerid, PEG-60 Maisglycerid, PEG-20 Nachtkerzenglycerid, PEG-60 Nachtkerzenglycerid, Traubenkernöl Glycereth-8 Ester, Cannabis Sativa Kernöl Glycereth-8 Ester, Jojobaöl Glycereth-8 Ester, PEG-16 Macademiaglycerid, PEG-25 Moringaglycerid, PEG-2 Olivenglycerid, PEG-6 Olivenglycerid, PEG-7 Olivenglycerid, Olivenöl Glycereth-8 Ester, PEG-10 Olivenglycerid, PEG-40 Olivenglycerid, Pfirsichkernöl Glycereth-8 Ester, PEG-60 Passiflora edulis seed glycerid, PEG-60 Passiflora Incarnata seed glycerid, PEG-40 Diestelglycerid, Sojaöl Glycereth-8 Ester, PEG-35 Soja glycerid, PEG-75 Soja glycerid Ester, PEG-2 Sonnenblumenglycerid, PEG-7 Sonnenblumenglycerid, Sonnenblumenöl Glycereth-8 Ester, PEG-10 Sonnenblumenglycerid, PEG-13 Sonnenblumenglycerid, PEG-7 Rapsglycerid, PEG-4 Rapsglycerid, PEG-10 Canolaglycerid, PEG-5 Tsubakiateglycerid, PEG-10 Tsubakiateglycerid, PEG-20 Tsubakiateglycerid, PEG-60 Tsubakiateglycerid, Baumwollöl Glycereth-8 Ester, Reisöl Glycereth-8 Ester, Sesamöl Glycereth-8 Ester, Weizenkeimöl Glycereth-8 Ester.
Ethoxylierte Rapsmethylester (EO 7-15), Ethoxylierte Rapsethylester (EO 7-15), Ethoxylierte Sojamethylester (EO 7-15), Ethoxylierte Sojaethylester (EO 7-15).
Besonders bevorzugt sind alkoxylierte Tenside mit einem HLB > 10.5 und <12.0. Beispiele dieser Vertreter sind Rapsmethylester oxylat 7 EO, PEG-4 Rapssamenamid der Fa. Kao, Olivenöl Glycereth-PEG-8 Ester, Mandelöl PEG-7 Ester, PEG-10 Oliven Glyceride.
Ganz besonders bevorzugt sind die Vertreter PEG-4 Rapssamenamid der Fa.
Kao, Pflanzenöl Glycereth-y Ester, Pflanze-PEG-y-ester, Ethoxylierter Rapsmethylester (y EO), mit Pflanze = Aprikose(nkern), Avocado, Baumwolle, Distel, Hanf, Jojoba, Leinsamen, Macademia, Mandel, Mais, Olive, Pfirsichkern, Reis, Sesam, Soja, Sonnenblume, Traube(nkern), Weizen(keim), Raps, Rüböl mit y= 6-10, bevorzugt 7-8.
In dieser Ausführungsform bevorzugt sind Mittel, die dadurch gekennzeichnet sind, dass sie - bezogen auf ihr Gewicht - 0.1 bis 50 Gew.-%, bevorzugt 0.1 bis 10 Gew.-% und besonders bevorzugt 0.2 bis 5 Gew.-% eines oder mehrerer zusätzlicher alkoxylierter Tenside enthalten, jeweils bezogen auf das Gesamtmittel.
Das oder die Tenside (D) können in jedem beliebigen Verhältnis mit (A) und (B) und gegebenenfalls (C) kombiniert werden.
Bevorzugt liegen die Tenside (D) zu dem Tensid (A)/ Glycolipid-Biotensid (B)-Gemisch in einem Verhältnis zwischen (D) :(A/B) ≤ 50:1 und ≥1:50 vor; beispielsweise 20:1, 9:1, 8:1 u.s.w.
Besonders bevorzugt liegen die Tenside (D) in einem Verhältnis zwischen (D) :(A/B) ≤ 20:1 und ≥1:20 vor und ganz besonders bevorzugt liegen die Tenside (D) in einem Verhältnis zwischen (D) :(A/B) ≤ 10:1 und ≥1:10 vor, äusserst bevorzugt liegt (D) :(A/B) ≤ 3:1 und ≥1:10; jeweils bezogen auf Gewichtsprozent Aktivgehalt im gesamten Mittel.

**Weitere optionale Tenside (E)**

Unter Tensid werden in diesem Zusammenhang amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Micellen, lamellare Strukturen u.a. Im Zusammenhang mit dieser Erfindung werden als Tenside Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

Für weitere optionale Tenside, welche vom Fachmann frei mit dem erfindungsgemässen Mittel kombiniert werden können, wird auf die einschlägige Fachliteratur wie z.B. Richard J. Farn, Chemistry and Technology of Surfactants, Blackwell Publishing, verwiesen. Einige Beispiele sind im Folgenden genannt, wobei die Kohlenwasserstoffketten, abgeleitet aus Fettsäuren oder synthetischen Kohlenwasserstoffen gesättigt oder ungesättigt, substituiert oder nicht-substituiert, linear oder verzweigt mit 4-24 Kohlenstoffatomen in der Kohlenwasserstoffkette umfassen. Optionale Tenside umfassen bevorzugt weitere Tenside aus C18-Pflanzenölen, sind jedoch nicht auf diese beschränkt.

Als weitere optionale nichtionische Tenside welche vom Fachmann frei mit dem erfindungsgemässen Mittel kombiniert werden können, eignen sich beispielsweise Alkoholpolyglykolether d.h. ethoxylierte und/oder propoxylierte Alkohole mit 1-40 Ethylenoxid (EO) und/oder Propylenoxid (PO)- Einheiten, Aminoxide, weitere alkoxylierten Amide, Polyethylenglykolmercaptane, Glykolipide, wie zum Beispiel Alkylpolyglykoside mit 1-10 Glykosideinheiten, Polyhydroxyfettsäureamide, Polyhydroxyfettsäureester, Carbonsäureester, Sorbitanester, sowie alkoxylierte Sorbitanester, Alkanolamin-Carbonsäure-Kondensate, N-Alkylpyrrolidone, Amidoalkyl-2-pyrrolidone.

Geeignete optionale anionische Tenside, welche vom Fachmann frei mit dem erfindungsgemässen Mittel kombiniert werden können, sind Acyllactylat, weitere Fettalkoholcarboxylate, sowie weitere Alkylpolyglykolethercarboxylat bzw. deren Mischungen.

Daneben können zusätzlich optional die weniger bevorzugten Schwefeltenside, Phosphate oder Phosphonate verwendet werden. Beispiele sind Alkylbenzolsulfonate, Alkan-/Alkensulfonate, Alkylsulfate bzw. Fettalkoholsulfate, Alkylpolyglykolethersulfate mit 2 bis 6 Ethylenoxideinheiten (EO) im Etherteil, sowie Sulfosuccinate, Carbonsäureamidethersulfate, Sulfobernsteinsäuremono- und diAlkylester, $\alpha$-Olefinsulfonate, Alkylisethionat, Acylisethionat ,Alkylsulfoacetate, sulfonierte Fettsäuren, sulfonierte Fettsäureester, wie sulfonierte Fettsäureglycerinester und sulfonierte Fettsäuremethylester, N-Acylaminosulfonsäuren, Alkylphosphate und Alkyletherphosphate, Phosphor- und Polyphosphorsäureester.

Überraschenderweise zeigen die erfindungsgemässen Mittel eine vergleichbare Reinigungsleistung zu üblichen Mitteln mit Schwefeltensiden, auch ohne deren Verwendung. Aus Umweltschutzgründen und den zunehmenden Sulfatgehalten in Trinkwasser ist eine bevorzugte Ausführungsvariante ist frei von allen Schwefeltensiden.

Ebenso kann auf die gewässerbelastenden Phosphate und Phosphonate ohne Kompromisse an die Reinigungsleistung verzichtet werden. Eine weitere bevorzugte Ausführungsform ist phosphat- und phosphonatfrei.

Weiterhin kann das Mittel optional kationische Tenside enthalten, beispielsweise primäre, sekundäre, tertiäre oder quartäre Alkylammoniumsalze der Formel (RI)(RII)(RIII)(RIV)$N^+X^-$, in der RI bis RVI unabhängig voneinander gleich- oder verschiedenartige Alkylreste, verzweigt und unverzweigt, gesättigt oder ungesättigt, unsubstituiert, einfach oder mehrfach substituiert, oder H, wobei $X^-$ für ein Anion steht.

Weiterhin können optional amphotere Tenside, welche vom Fachmann frei mit dem erfindungsgemässen Mittel kombiniert werden können, enthalten sein, wie beispielsweise N-Alkylbetaine, Alkylamidobetaine, Imidazoliniumbetaine, Aminoxide, und weniger bevorzugt Sulfobetaine, Phosphobetaine und Sultaine.

Die erfindungsgemässen Mittel entwickeln einen stabilen Schaum und machen damit das häufig eingesetzte Cocoamidopropyl Betain entbehrlich. Diese Substanz weist ein erhebliches Irritationspotential auf. Eine bevorzugte Ausführungsvariante ist daher frei von Cocoamidopropyl Betain.

Eine weitere bevorzugte Ausführungsvariante ist frei von Cocamid DEA, Cocamid MEA, welche ebenso zur Schaumstabilisierung Anwendung finden und als hautreizend bekannt sind.

Besonders bevorzugt enthalten die Mittel Tenside (E), die abgeleitet sind von Fettsäuren RCOOH mit R wie oben definiert. Die Reste R liegen bevorzugt als Gemisch gemäss der Fettsäureverteilung im nativen Öl vor oder wie sie bei der Umsetzung von nativen Ölen entstehen.

**Zusammensetzung der Tenside im erfinderischen Mittel**

Bevorzugt enthält das Mittel einen Anteil an Tensiden, bestehend aus Tensid (A) der Formel (I), den Tensiden (C) und (D), und gegebenenfalls Tensiden (E), mit der Vorgabe, dass das oder die Tenside (E) von einem C-18-Pflanzenöl abgeleitet sind, und Biotensid-Glycolipide (B), welcher in Summe $\geq$ 35%, bevorzugt $\geq$ 60 %, besonders bevorzugt $\geq$ 95 % und äusserst bevorzugt $\geq$ 99 % beträgt, bezogen auf den Gesamtgehalt an Tensiden in Gew.-% in dem Mittel.

Dieser Anteil in % stellt den Anteil an Tensiden in dem Mittel dar, welcher von C-18-Pflanzen abgeleitet ist und wird bestimmt nach der Formel:

$$\text{Anteil} = [(A) + (B) + (C) + (D) + (E_{C18})] / [(A) + (B) + (C) + (D) + (E)]$$

Wobei

(A), (B), u.s.w. jeweils die Menge an dem oder den Tensiden (A), (B), u.s.w. angegeben, bezogen auf Gew.-% bezogen auf die Gesamtzusammensetzung,

wobei (C), (D) und (E) jeweils unabhängig voneinander Null sein können,

wobei (E) weitere optionale Tenside (RX mit X = hydrophiler Rest) wie oben definiert darstellt, welche entweder abgeleitet sind aus C-18-Pflanzen (Hier genannt $E_{C18}$) - mit R stammend aus den Fettsäuren RCOOH mit R, bzw. RCO definiert wie unter Formel (I) - oder abgeleitet sind von anderen Ölen oder Fetten wie beispielsweise Erdöl, Palmkernöl, Kokosöl, tierischen Fetten, Rizinusöl oder Silikontenside u.a. (hier genannt $E_{Rest}$). D.h. (E) = ($E_{C18}$ + $E_{Rest}$).

[(A) + (B) + (C) +(D) + (E)] stellt den Gesamtgehalt an Tensiden in Gew.-% in dem Mittel dar.

**Zusatzstoffe & Eigenschaften**

**Weitere Biotenside / Bioemulgatoren (F)**

Weiterhin können in der erfindungsgemässen Zubereitung weiterhin Emulgatoren enthalten sein. Für optionale Emulgatoren, welche vom Fachmann frei mit dem erfindungsgemässen Mittel kombiniert werden können, wird auf die einschlägige Fachliteratur verwiesen. Da die Grenzen von Tensiden und Emulgatoren fliessend sind, wird im Zusammenhang dieser Erfindung werden Emulgatoren und Tenside differenziert wie unter (E) beschrieben.

In der erfindungsgemässen Zubereitung können zusätzlich weitere optionale Bioemulgatoren (E) enthalten sein, insbesondere Lipopeptide, welche vorzugsweise aus einem Cycloheptapeptid, mit gesättigten, ungesättigten, substituierten oder unsubstituierten Fettsäurereste von Kettenlängen von 2-30 Kohlenstoffatomen bestehen, welche an die Aminosäurereste gebunden sind, beispielsweise Surfactin, Iturin, Fengycin.

Bioemulgatoren (F) werden von Mikroorganismen produziert. Bioemulgatoren gehören den Biotensiden an, werden aber in der Literatur als höhermolekulare Verbindungen als die klassischen Glycolipid- Biotenside verstanden (Chibuzo Uzoigwe et al., Front. Microbiol. 2015; 6; 245), welche aus komplexen Mischungen von Heteropolysacchariden, Lipopolysacchariden, Lipoproteinen und Proteinen bestehen. Weitere Beispiele für Bioemulgatoren (E) sind Emulsan, produziert durch Acinetobacter calcoaceticus, Alasan, produziert durch A. redioresistens, oder mannoprotein bioemulsifiers von Kluyveromyces marxianus, Saccharomyces cerevisiae, oder Acinetobacter sp.

Die bevorzugte erfindungsgemäße Zubereitungen können ein oder mehrere zusätzliche Bioemulgatoren (F) in einer Gesamtmenge von 0.05-50 Gew.-%, vorzugsweise 0.2-20 Gew.-%, insbesondere 0.5-10 Gew.-% enthalten, bezogen auf die Gesamtmenge der Zubereitung.

Lösungsmittel

Das erfindungsgemässe Mittel kann alle in Wasch-, Pflege- und Reinigungsmitteln übliche Lösungsmittel enthalten. Sie dienen der Stabilisierung der Formulierung, der Solubilisierung von schlecht löslichen Inhaltsstoffen und der Erhöhung der Reinigungsleistung.

In einer bevorzugten flüssigen oder gelförmigen Ausführungsform enthält das Mittel Wasser als Lösungsmittel, wobei mehr als 5 Gew.-%, bevorzugt mehr als 15 Gew.-% und besonders bevorzugt mehr als 25 Gew.% Wasser enthält, jeweils bezogen auf die Gesamtmenge des Mittels. Besonders bevorzugte Mittel enthalten - bezogen auf ihr Gewicht- 5 bis 98 Gew.-%, bevorzugt 10 bis 90 Gew.-%, besonders bevorzugt 25 bis 75 Gew.% Wasser. Alternativ kann es sich um wasserarme oder wasserfreie Mittel handeln, wobei der Gehalt an Wasser in einer bevorzugten Ausführungsform weniger als 10 Gew.-%, und mehr bevorzugt weniger als 8 Gew.-% enthält, jeweils bezogen auf das gesamte flüssige Mittel.

In einer weiteren flüssigen oder gelförmigen Ausführungsform ist das Mittel wasserfrei, wobei das Mittel ein organisches Lösungsmittel als Hauptlösungsmittel enthält. Dabei ist es bevorzugt, dass das Mittel 5 bis 98 Gew.-%, bevorzugt 10 bis 90 Gew.-%, besonders bevorzugt 25 bis 75 Gew.-% Lösungsmittel enthält.

Beispielhafte Lösungsmittel sind die folgenden gemäss INCI benannten Verbindungen: Alcohol denat. (Ethanol), Alkohole, Buteth-3, Butoxydiglycol, Butoxyethanol, Butoxyisopropanol, Butoxypropanol, n-Butyl Alcohol, t-Butyl Alcohol, Butyl-3-hydroxybutyrate, Butylene Glycol, Butyloctanol, C1-C6-Alkane, C7-C15-Alkane, Diethylene Glycol, Diethylenglycol monobutylether, Dimethoxydiglycol, Dimethyl Ether, Dimethyl 2-methylglutarat, Dipropylene Glycol, Dipropylenglycol Phenylether, Ethyllactat, 2-Ethyllactat, Ethyl levulinate glycerol ketal, Ethyl levulinate propylene glycol ketal, Ethyl levulinate ethylene glycol ketal, Ethoxydiglycol, Ethoxyethanol, Ethyl Hexanediol, Fettsäuremethylester z.B. auf Basis C18-Pflanzen, Gammalaverolacton, Glycol, Glycerin, Hexanediol, 1,2,6-Hexanetriol, Hexyl Alcohol, Hexylene Glycol, Isobutoxypropanol, Isopentyldiol, Isopropyl Alcohol (iso-Propanol), Lävulinsäure ester, 3-Methoxybutanol, Methoxydiglycol, Methoxyethanol, Methoxyisopropanol, Methoxymethylbutanol, Methoxy PEG-10, Methylal, Methyl Alcohol, Methyl-9-dodecenoate, Methyl Hexyl Ether, Methylpropanediol, 2-Methyl THF, Neopentyl Glycol, N,N-Dimethyl-9-decenamide, Polyole, PEG-4, PEG-6, PEG-7, PEG-8, PEG-9, PEG-6 Methyl Ether, Pentylene Glycol, PPG-7, PPG-2-Buteth-3, PPG-2 Butyl Ether, PPG-3 Butyl Ether, PPG-2 Methyl Ether, PPG-3 Methyl Ether, PPG-2 Propyl Ether, 1,2-Propanediol, 1,3-Propandiol, Propyl Alcohol (n-Propanol), Propylene Glycol, Propylene Glycol Butyl Ether, Propylene Glycol Propyl Ether, Terpene, z.B. Limonen, Thymol, u.a. insbesondere natürlichen Ursprungs wie Zitronenöl, Lavendelöl, Thymianöl, u.a., Tetrahydrofurfuryl Alcohol, Trimethylhexanol. Erfindungsgemäss können diese Lösungsmittel in einer dem Fachmann durchaus bekannten Art und Weise frei mit anderen Inhaltsstoffen kombiniert werden.

In einer bevorzugten Ausführungsform, werden Lösungsmittel aus der Gruppe Lösungsmittel, die aus pflanzlichen Rohstoffen gewonnen werden und biologisch abbaubar sind, verwendet. Besonders bevorzugt sind Lösungsmittel, die keine VOC (volatile organic compounds) enthalten.

Eine besonders bevorzugte Ausführungsform enthält zusätzlich Fettsäurealkylester der Formel $R\text{-}CO\text{-}O\text{-}R^{25}$ als Lösungsmittel
wobei der Fettsäurealkylester aus einem Gemisch unterschiedlicher Kettenlängen und Sättigungsgrade des Fettsäurerests RCO wie definiert bei Tensid (A) besteht und abgeleitet ist von einem C18 Pflanzenöl;
und wobei $R^{25}$ ein linearer oder verzweigter Kohlenwasserstoff von 1-5 Kohlenstoffatomen ist, bevorzugt bestehend aus einer Methyl- oder Ethylgruppe, besonders bevorzugt Methyl.

Bevorzugte Vertreter sind Rapsmethylester, Sonnenblumenmethylester, Distelmethylester oder Sojamethylester.

Enthärter und Komplexbildner,
INCI Chelating agents, Sequestrierungsmittel
Erfindungsgemäss geeignet sind alle in Wasch-, Pflege- und Reinigungsmitteln üblichen Komplexbildner. Sie erhöhen die Waschkraft sowie die Stabilität der Mittel.

Erfindungsgemäss geeignet sind beispielsweise Enthärter und Komplexbildner aus den Gruppen der Phosphate und Phosphonate, Schichtsilikate, Zeolithe, Carbonate und Polycarboxylate, Aminopolycarbonsäuren, wie Aminoessigsäuren und Polyaminoessigsäuren sowie deren Salze, Hydroxycarbonsäuren und deren Salze, Polyglycoside undgluconsäuren und deren Salze.

Geeignet sind beispielsweise die folgenden Komplexbildner: Aminotrimethylene Phosphonsäure, Beta-Alanine Acetessigsäure, Calcium Disodium EDTA, Chitosan, Zitronensäure und dessen Salze und Hydrate, Cyclodextrin, Cyclohexanediamin Tetraessigsäure, Diammonium Citrat, Diammonium EDTA, Diethylenetriaminepentaacetic Acid, Diethylenetriamine Pentamethylene Phosphorsäure, Dikalium EDTA, Dinatrium Azacycloheptane Diphosphonat, Dinatrium EDTA, Dinatrium Pyrophosphate, EDTA, Ethylenediamine- *N,N'*-disuccinic acid (EDDS), Etidronsäure, Galactarsäure, β-Glucan, Gluconsäure, Glucuronsäure, Glucoheptonsäure, HEDTA, Hydroxypropyl Cyclodextrin, Methyl Cyclodextrin, Pentapotassium Triphosphat, Pentasodium Aminotrimethylene Phosphonat, Phosphonobutane tricarbonsäure (PBTC), Pentasodium Ethylenediamine Tetramethylene Phosphonat, Pentasodium Pentetate, Pentasodium Triphosphate, Pentetic Acid, (DTPA), Phytic Acid, Potassium Citrate, Potassium EDTMP, Kalium Gluconate, Kalium Polyphosphate, Kalium Trisphosphonomethylamine Oxide, Ribonsäure, Natrium Chitosan Methylene Phosphonate, Natriumcitrat, Natrium Diethylenetriamine Pentamethylene Phosphonate, Natrium Dihydroxyethylglycinate, Natrium EDTMP, Natrium Glucoheptate, Natrium Gluconate, Natrium Glycereth-1 Polyphosphate, Natrium Hexametaphosphate, Natrium Metaphosphate, Natrium Metasilicate, Natrium Phytate, Natrium Polydimethylglycinophenolsulfonate, Natrium Trimetaphosphate, TEA-EDTA, TEA Polyphosphate, Tetrahydroxyethyl Ethylenediamin, Tetrahydroxypropyl Ethylenediamin, Tetrakalium Etidronat, Tetranatrium Iminodisuccinat (IDS), Tetrakalium Pyrophosphat, Tetranatrium EDTA, Tetranatrium Etidronat, Tetranatrium Pyrophosphat, Trikalium EDTA, Trikalium Dicarboxymethyl Alaninat, Trinatrium EDTA, Trinatrium HEDTA, Trinatrium Methylglycin dieacetic acid (MGDA- $Na_3$), Trinatrium NTA und Trinatrium Phosphate, Phytinsäure, Plfanzenextrakte wie z.B. Lupinus Albus Seed Extract, Carnosine, Bambusa Arundinacea Leaf Extract, Citrus Paradisi (Grapefruit) Peel Extract, Sambucus Nigra Extract; oder im Falle von Säuren, deren Salze.
Diese Chelatbildner können vom Fachmann frei mit anderen hier genannten Inhaltstoffen kombiniert werden.

In einer bevorzugten Ausführungsform enthalten die erfindungsgemässen Mittel Komplexbildner, die biologisch abbaubar sind. Bevorzugt enthalten die erfindungsgemässen Mittel daher keine Phosphate, keine Phosphonate, kein EDTA und keine Polycarboxylate.

Ganz besonders bevorzugt in dieser Erfindung sind folgende Komplexbildner auf Basis erneuerbarer Rohstoffe, wie beispielsweise Beta-Alanine Diacetic acid, Cyclodextrin, Diammonium citrat, Galactarsäure, Gluconsäure, Glucoronsäure, Methylcyclodextrin, Hydroxypropyl cyclodextrin, Polyasparaginsäure, Alkali Salze von Gluconate, Natrium Carbonat, Carboxy methyl inulin und Natrium Carboxymethyl inulin (NaCMI), Natrium Citrat, Natrium Dihydroxyethylglycinat, Natrium Gluconat, Natrium Glucoheptonat, Natrium Iminodisuccinat, Natrium Lactat, Natrium Lignosulfate, Tetranatrium GLDA (I-glutamic acid, N,N-di (acetic acid), tetrasodium salt), Zitronensäure und deren Salze,

Bevorzugte erfindungsgemäße Zubereitungen enthalten mindestens einen Komplexbildner in einer Gesamtmenge von 0.1-20 Gew.-%, vorzugsweise 0.2-15 Gew.-%, insbesondere 0.5-10 Gew.-%, bezogen auf die Gesamtmenge der Zubereitung.

**Abrasiva und Poliermittel**

Die erfindungsgemässen Zubereitungen zeigen eine gute Reinigungswirkung, so dass der Zusatz von Abrasiva entbehrlich ist. Nichtsdestoweniger können die Zubereitungen, insbesondere im Bereich der Mund- und Zahnpflege oder im Bereich der Grobreiniger gegebenenfalls für bestimmte Reinigungsanwendungen Abrasiva als Komponenten enthalten. Hierzu gehören Kunststoffreibemittel auf der Basis von Polyethylen oder Polyurethan, organische Polymere, mineralische Reibemittel wie Kieselsäuren z.B. Gelkieselsäuren, Hydrogelkieselsäuren und Fällungskieselsäuren, Aluminiumhydroxid, Aluminiumoxid, Magnesiumsulfat, Natriumaluminiumsilikate, Calciumcarbonat, Kaolin, Sand, Kreide, Calciumpyrophosphat, Dicalcium-phosphatdihydrat und andere sowie Reibemittel auf pflanzlicher Basis wie Cellulosederivate, Holzmehl oder Kern- und Schalenmehle, sowie deren Gemische.

Insbesondere bevorzugt sind für die Verwendung als Grobreinigung sind Reibemittel auf der Basis von natürlichen Kern- und/oder Schalenmehlen, insbesondere Walnussschalen, Mandelschalen, Hasselnussschalen, Olivenkern-, Aprikosenkern- und Kirschkernmehl oder Perlen aus Wachsen (z.B. Jojobawachse).

Für die Verwendung in der Zahnpflege bevorzugt geeignete Poliermittelkomponenten sind calciumarm wie Kieselsäuren, Aluminiumhydroxid, Aluminiumoxid, Natriumaluminiumsilikate, organische Polymere oder Gemische solcher Reibkörper.

Die Konzentration der Reibemittel kann bis zu 50 Gew.-% betragen, bevorzugt 0 - 30 Gew.-%, bezogen auf die Gesamtmenge der Zubereitung.

**Konservierungsmittel**

Das erfindungsgemässe Mittel kann alle in Wasch-, Pflege- und Reinigungsmitteln übliche Konservierungsmittel enthalten, welche vom Fachmann im Sinne dieser Anwendung frei mit anderen Inhaltsstoffen kombiniert werden können.

Dies sind beispielsweise Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff- und Stickstoff-Acetale sowie -Formale, Benzamidine, Isothiazole und deren Derivate wie Isothiazolinone, Phtalimidderivate, Pyridinderivate, oberflächenaktive Verbindungen, Guanidine, antimikrobielle amphoterer Verbindungen, Chinoline, 1,2-Dibrom-2,4-dicyanobutan, lodo-2-propynyl-butyl-carbamat, lod, lodophore und Peroxide.

Besonders bevorzugt ist die Konservierung des erfindungsgemässen Mittels auf Basis von antimikrobiellen Wirkstoffen ausgewählt aus antimikrobiellen Peptiden, Ethanol, Benzylalkohol, Dehydroessigsäure und deren Salzen, Sorbinsäure und Kaliumsorbat, pflanzlichen organischen Säuren und deren Salze, Ameisensäure, Glycerin, Zitronensäure, Milchsäure, Salicylsäure, sowie deren Salze.

Äusserst bevorzugt ist die Ausführungsform frei von chemischen Konservierungsmittel, wie in den Ausführungsbeispielen offenbart, d.h. insbesondere ohne Parabene, ohne formaldehydhaltige Konservierungsmittel bzw. Formaldehydabspalter, ohne Isothiazole und deren Derivate, ohne halogenhaltige Verbindungen, ohne Phtalimide, ohne Benzalkoniumchlorid, ohne Benzoesäure, ohne Phenoxyethanol.

**Antioxidantien**

Bevorzugt werden der kosmetischen Zubereitung Antioxidantien zugesetzt, einerseits um die ungesättigten Kohlenwasserstoffketten der erfinderischen Zubereitung zu schützen und andererseits, um bei der Anwendung schädliche oxidative Einflüsse auf Haut bzw. Haare zu mindern. Beispielsweise werden die Antioxidantien ausgewählt aus der Gruppe bestehend aus Aminosäuren (z.B. Glycin, Histidin, Tyrosin, Tryptophan) und deren Derivaten, Imidazole (z.B. Urocaninsäure) und deren Derivaten, Peptide wie D,L-Carnosin, D-Carnosin, L-Carnosin und deren Derivaten (z.B. Anserin), Carotinoide, Carotine und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, [gamma]-Linoleyl-, Cholesteryl- und Glycerylester) sowie deren Salze, Dilaurylthiodipropionat, Distearylthiodipropionat, Thiodipropionsäure und deren

Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. [alpha]-Hydroxyfettsäuren, Palmitinsäure, Phytinsäure, Lactoferrin), [alpha]-Hydroxysäuren (z.B. Citronensäure, Milchsäure, Apfelsäure), Numinsäure, Gallensäure, Gallenextrakte, Bilirubin, Biliverdin, EDTA, EGTA und deren Derivate, ungesättigte Fettsäuren und deren Derivate (z.B. [gamma]-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, [alpha]-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid), Superoxid-Dismutase und die erfindungsgemäss geeigneten Derivate (Salze, Ester, Ether, Zucker, Nukleotide, Nukleoside, Peptide und Lipide) dieser genannten Stoffe.

Erfindungsgemäss bevorzugt sind Antioxidantien, die auf Rohstoffen aus Pflanzen, bevorzugt C-18-Pflanzen beruhen, wie beispielsweise Antioxidantien aus den Gruppen der Aminosäuren, Peptide, Cartinoide, Chelatoren, Pflanzenextrakten und Hydroxysäuren, sowie Mischungen derselben.

Die Menge der Antioxidantien (eine oder mehrere Verbindungen) in den Zubereitungen beträgt vorzugsweise 0.001 bis 30 Gew.-%, besonders bevorzugt 0.05-20 Gew.-%, insbesondere 1-10 Gew.-%, bezogen auf die Gesamtmenge der Zubereitung. Diese Antioxidatien können vom Fachmann mit anderen hier genannten Inhaltstoffen kombiniert werden.

**Konditionierungsmittel**

Die erfindungsgemässe Zubereiung kann übliche, dem Fachmann bekannte Konditionierungsmittel enthalten, wie beispielsweise monographiert in dem "International Cosmetic Ingredient Dictionary and Handbook", 14. Auflage, K. Schrader, oder den "Fundamentals and Formulations of Cosmetics", 2. Auflage (Hüthig Verlag, Heidelberg, 1989), S. 782-815.

**Desodorierende Wirkstoffe**

Das Mittel kann Deodorant-Wirkstoffe enthalten. Hierunter werden Geruchsabsorber, desodorierend wirkende Ionenaustauscher, keimhemmende Mittel, präbiotisch wirksame Komponenten sowie Enzyminhibitoren oder Kombinationen der genannten Wirkstoffe verstanden.

Als Geruchsabsorber dienen Silicate, die auch gleichzeitig die Viskosität der erfindungsgemäßen Mittel vorteilhaft unterstützen können. Hierzu zählen Schichtsilicate, insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Sie werden in einer Menge von 0.1 - 10 Gew.-%, vorzugsweise 0.5 - 7 Gew.-% und insbesondere 1 - 5 Gew.-%, jeweils bezogen auf die Gesamtmenge der Zubereitung eingesetzt.

**Keimhemmende, fungizide und antimikrobielle Wirkstoffe**

Optional kann die erfindungsgemässe Zubereitung Wirkstoffe gegen Mikroorganismen wie Pilze oder Bakterien enthalten um Geruch, Schuppenbildung, Karies, Akne u.a. zu unterbinden.

Als keimhemmende oder antimikrobielle Wirkstoffe erfindungsgemäß einsetzbar sind Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, $\alpha$-Monoalkylglycerinether mit einem verzweigten oder linearen gesättigten oder ungesättigten, gegebenenfalls hydroxylierten $C_6$ - $C_{22}$-Alkylrest, besonders bevorzugt $\alpha$-(2-Ethylhexyl)glycerinether, Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Wirkstoffe sind ausgewählt aus sogenannten präbiotisch wirksamen Komponenten, worunter erfindungsgemäß solche Komponenten zu verstehen sind, die nur oder zumindest überwiegend die unerwünschten

Keime der Hautmikroflora hemmen, nicht aber die erwünschten, das heißt, diejenigen, die zu einer gesunden Hautmikroflora gehören. Dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbeondere Extrakte aus Picea sp., Paullinia sp., Panax sp., Lamium album oder Ribes nigrum sowie Mischungen dieser Substanzen.

Weitere bevorzugte Wirkstoffe sind ausgewählt aus den keimhemmend wirkenden Parfümölen bzw. ätherischen Ölen.

Weiterhin kann das Mittel Enzyminhibitoren enthalten, die die für die Schweißzersetzung verantwortlichen Enzyme, insbesondere die Arylsulfatase, β-Glucuronidase, Aminoacylase, die esterspaltenden Lipasen und die Lipoxigenase, hemmen, z. B. Trialkylcitronensäureester, insbesondere Triethylcitrat, oder Zinkglycinat.

Die Menge der Deodorant-Wirkstoffe in den erfindungsgemäßen Zusammensetzungen beträgt 0,1 - 10 Gew.-%, vorzugsweise 0,2 - 7 Gew.-%, insbesondere 0,3 - 5 Gew.-% und außerordentlich bevorzugt 0,4 - 1,0 Gew.-%, bezogen auf die Gesamtmenge der Zubereitung.

**Antitranspirant-Wirkstoffe**

Optional können auch Antitranspirant-Wirkstoffe enthalten sein wie wasserlösliche adstringierende anorganische und organische Salze des Aluminiums, Zirkoniums und Zinks bzw. beliebige Mischungen dieser Salze. Hierzu zählen Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat- Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat ($KAl(SO_4)_2 \cdot 12\ H_2O$, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat.

Die Menge der Antitranspirant-Wirkstoffe in den erfindungsgemäßen Zusammensetzungen kann bis zu 10 Gew.-% betragen, vorzugsweise 0.2 - 7 Gew.-%, insbesondere 0.3 - 5 Gew.-% und außerordentlich bevorzugt 0.4 - 1.0 Gew.-%, bezogen auf die Gesamtmenge der Zubereitung.

Eine bevorzugte Ausführungsvariante enthält keine Antitranspirant-Wirkstoffe.

**UV-Filter**

Es ist auch möglich und vorteilhaft im Sinne der vorliegenden Erfindung, einen Gehalt an UV-Schutzsubstanzen zu verwenden. Die erfindungsgemässe Zubereitung eignet sich insbesondere, da die Pigmente in der Kombination der Tenside wie offenbart ausserordentlich gut dispergiert werden. Neben dem Effekt, dass dadurch Pigmente gut von einem Substrat gelöst werden können, hat dies gleichzeitig den Effekt, dass Zubereitungen mit Farbe oder Pigmenten stabilisiert werden. Beispiele für pigmentäre UV-Filter sind Zinc oxid, Titan dioxid, Methylene Bis-Benzotriazolyl Tetramethylbutylphenol, u.a.

Vorteilhaft können daher erfindungsgemässe Zubereitungen Substanzen enthalten, die UV-Strahlung im UVA und UVB-Bereich absorbieren, wobei die Gesamtmenge der Filtersubstanzen an der gesamten Zubereitung vorzugsweise zwischen 0.1 Gew.-% bis 30 Gew.-% betragen kann. Bevorzugt sind handelsübliche wasser- oder öllösliche UV-Filter. Üblicherweise werden Kombinationen von UV-Filtern eingesetzt, die vom Fachmann mit anderen hier genannten Inhaltstoffen kombiniert werden können.

**Antischuppenmittel**

Das kosmetische Mittel kann Kompontenten enthalten, die gegen Kopfschuppen wirksam sind. Beispiele hierfür sind Zinkpyrithion, Selendisulfid, Piroctone Olamin, Climbazol oder auch Pflanzenextrakte wie Vitis Vinifera Seed Extract, Thymus Serpyllum Extrakt, Rosmarinus officialis Leaf Extrakt, Leuconostoc / Radish Root Ferment Filtrate, Leptospermone/ Isoleptospermone/ Flavesone, Leptospermum Scoparoi, Branch/ Leaf Oil, Fragaria ananassa (Strawberry) Seed oil, Adianthum Capillus Veneris Leaf Extrakt, Brennessel Extrakt, Wacholder Extrakt, Thymian Extrakt und andere.

In den erfindungsmässigen Zubereitungen mit Anti-Schuppen-Wirkstoff werden diese in Konzentrationen bis zu 7 Gew.-%, bevorzugt 0.1-4 Gew.-%, besonders bevorzugt 0.1-2 Gew.-% eingesetzt, jeweils bezogen auf die Gesamtmenge der Zubereitung.

***Weitere Wirkstoffe***

Weiterhin können andere Wirkstoffe in dem kosmetischen Mittel enthalten sein, wie beispielsweise Anti-Ageing-Wirkstoffe, Peptide, UV-Absorber, Vitamine & Mineralstoffe (z.B. Ascorbinsäure, Biotin, Tocopherol oder Rutin, Niacin), Mineralsalze wie z.B. Mangan-, Zink- oder Magnesiumsalze, weitere pflanzliche Extrakte.
Sie werden bevorzugt in Mengen von 0.01- 5 Gew.-% in die erfindungsgemäßen Zubereitungen eingesetzt, Gew.-% bezogen auf die Gesamtmenge der Zubereitung.

**Haarfarben**

Die erfindungsgemässen Zubereitungen können auch Farbstoffe zur direkten oder oxidativen Färbung von Haaren enthalten. Die Färbezubereitungen sollen das Haar schützen und möglichst wenig schädigen. Auf Basis der erfindungsgemässen Zubereitungen können sowohl Haarfärbemittel als auch Tönungsshampoos hergestellt werden.
Vorteil der erfindungsgemässen Zubereitung ist, dass nach dem Färben das Nachwaschen mit einem Shampoo entfallen kann, da die Farbstoffzubereitung bereits tensidhaltig ist.

**Bindemittel/ Konsistenzgeber**

Die erfindungsgemässe Zubereitung kann als Bindemittel bzw. Konsistenzregler dienen z. B. natürliche und/oder synthetische wasserlösliche Polymere wie Alginate, Carrageenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere Polyvinylalkohol, Polyvinylpyrrolidon, Polyethylenglycole.
Sie werden bevorzugt in Mengen von 0,01- 10 Gew.-% in die erfindungsgemäßen Zubereitungen eingesetzt, Gew.-% bezogen auf die Gesamtmenge der Zubereitung.

Fettsäuren

Die erfindungsgemässe Zubereitung kann zusätzlich lineare, gesättigte, ungesättigte, gegebenenfalls mehrfach ungesättigte Fettsäuren mit 6-24 Kohlenstoffatomen enthalten. Erfindungsgemäss bevorzugt sind Fettsäuren abgeleitet aus C18-Pflanzen.

Ölkörper

Die erfindungsgemässe Zubereitung kann übliche, dem Fachmann bekannte Ölkörper enthalten. Zu Ölkörpern zählen wasserunlösliche, organische Verbindungen, wobei die Wasserunlöslichkeit erfindungsgemäß als eine Löslichkeit von weniger als 10 Gew.-% bei 20° C verstanden wird.
Zu den erfindungsgemäß einsetzbaren Ölkörpern zählen beispielsweise Glyceride, Kohlenwasserstoffe, Silikonöle, Dialkylether, Dialkylcarbonate, Wachsester, etc. Auch beliebige Gemische von Ölkörpern sind erfindungsgemäß einsetzbar.
Glyceride sind Fettsäureester des Glycerins, die natürlicher (tierischer, mikrobieller, algenbasierter und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Triglyceride oder Glycerid-Gemische mit einem hohen Triglycerid-Anteil (> 90 Gew.-%) sind bevorzugt. Als Fettsäure sind erfindungsgemäß C6-C30-Fettsäuren geeignet. Die Fettsäuren können verzweigt oder unverzweigt, hydroxyliert oder nicht-hydroxyliert, gesättigt oder ungesättigt sein. Erfindungsgemäß bevorzugt ist die Verwendung Glyceride pflanzlicher Herkunft, besonders bevorzugt sind Glyceride der C-18-Pflanzen, z.B. Sojaöl, Baumwollsaatöl, Sonnenblumenöl, Leinöl, Mandelöl, Maisöl, Olivenöl, Rapsöl, Sesamöl, Distelöl, Weizenkeimöl, Pfirsichkernöl und dergleichen. Exemplarisch sind einige Zubereitungen mit Ölkörpern in den Ausführungsbeispielen offenbart.
Zu den erfindungsgemäß einsetzbaren Ölkörpern zählen auch natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkycyclohexane.
Erfindungsgemäß sind als Ölkörper auch Siliconöle geeignet. Zu diesen zählen z. B. Dialkyl- und Alkyl- arylsiloxane, wie beispielsweise Cyclomethicone, Dimethylpolysiloxan und Methylphenylpolysiloxan, sowie deren alkoxylierte und quaternisierte Analoga. Geeignete nicht-flüchtige Siliconöle, wie z. B. Polyalkylsiloxane, Polyalkylarylsiloxane und Polyethersiloxan-Copolymere sind in Cosmetics: Science and Technology, Hrsg.: M. Balsam und E. Sagarin, Bd.

1, 1972, S. 27-104 zu entnehmen.

Siliconöle sind aus Nachhaltigkeitsbetrachtungen in den erfindungsgemässen Zubereitungen nicht bevorzugt.

Geeignete und erfindungsgemäß bevorzugte Öle sind weiterhin verzweigte gesättigte oder ungesättigte Fettalkohole mit 6 - 30 Kohlenstoffatomen. Diese Alkohole werden häufig auch als Guerbet-Alkohole bezeichnet, da sie nach der Guerbet-Reaktion erhältlich sind. Beispiele bevorzugter Alkoholöle sind Hexyldecanol, Octyldodecanol, 2-Ethylhexylalkohol und die Handelsprodukte Guerbitol® 18, Isofol® 12, Isofol® 16, Isofol® 24, Isofol® 36, Isocarb® 12, Isocarb® 16 oder Isocarb® 24.

Weitere geeignete Ölkomponenten sind Mischungen aus Guerbetalkoholen und Guerbetalkoholestern, z.B. Hexyldecanol und Hexyldecyllaurat.

Als Ölkörper kommen auch Ester von linearen, gesättigten oder ungesättigten C6-C30-Fettsäuren mit linearen oder verzweigten, gesättigten oder ungesättigten C6-C30-Fettalkoholen bzw. Ester von verzweigten C6-C30-Carbonsäuren mit linearen oder verzweigten, gesättigten oder ungesättigten C6-$C_{30}$-Fettalkoholen, welche hydroxyliert sein können (sogenannte Wachsester) in Frage.

Unter den Wachsestern seien expemplarisch folgende Vertreter genannt: Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearyl- myristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearyl- behenat, Isostearyloleat, Oleylmyristat, Oleylpalmitat, Oleylstearat, Oleylisostearat, Oleyloleat, Oleylbe- henat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Vorzugsweise werden ein- oder mehrfach ungesättigte Wachsester, wie beispielsweise Oleyloleat und Oleylerucat eingesetzt. Insbesondere bevorzugt sind diese Wachsester abgeleitet aus C18-Pflanzen. Daneben eignen sich auch Ester von linearen C6-C22-Fettsäuren mit verzweigten Alkoholen, insbesondere 2-Ethylhexanol, Ester von C2-C38-Alkylhydroxycarbonsäuren mit linearen oder verzweigten C6-C22-Fettalkoholen, Ester von linearen und/oder verzweigten Fettsäuren mit mehrwertigen Alkoholen (wie z. B. Propylenglycol, Dimerdiol oder Trimertriol) und/oder Guerbetalkoholen, sowie Ester von C6-C22-Fettalkoholen und/oder Guerbet- alkoholen mit aromatischen Carbonsäuren, insbesondere Benzoesäure, Ester von C2-C12- Dicarbonsäuren mit linearen oder verzweigten Alkoholen mit 1 bis 22 Kohlenstoffatomen (z. B. Dioctyl Malate) oder Polyolen mit 2 bis 10 Kohlenstoffatomen und 2 bis 6 Hydroxylgruppen.

Weitere beispielhafte Vertreter sind: Hexyldecylstearat , Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Ethylenglycoldioleat und -dipalmitat, Linolylmyristat, Linolylpalmitat, Linoleylmyristat, Linoleylpalmitat, Isostearyloleat, Isostearylbehenat, Isostearyloleat, , Linoylstearat, Linoleylstearat, Oleyloleat, Oleyllinolat, Oleyllinolenat, Oleylrizinat, Oleylerucat, Linolyloleat Linolyllinolat, Linolylrizinat, Linolylerucat, Linolenyloleat, Linolenyllinolat, Linolenyllinenolat, Linolenylrizinat, Linolenylerucat, Erucyllinolat, Erucyllinolenat, Erucylrizinat, wobei die Linolenderivate alpha-, sowie gamma-Linolenderivate einschliessen.

Als Ölkörper geeignet sind lineare oder verzweigte, symmetrische oder unsymmetrische, gesättigte oder ungesättigte Di-n-alkyl(en)ether mit 12 bis 24 C-Atomen pro Alkyl(en)gruppe, wie z. B. Di-n-octylether, Di-(2-ethylhexyl)-ether, Laurylmethylether oder Octylbutylether, Didodecylether oder Dibehenylether, wobei bevorzugt Ether abgeleitet aus C18-Pflanzen verwendet werden.

Besonders bevorzugt sind als Ölkörper lineare oder verzweigte, gesättigte oder ungesättigte C6-C40-Fettalkoholcarbonate geeignet. Diese Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996). Typische Beispiele für Dialkyl(en)carbonate sind vollständige oder partielle Umesterungsprodukte von Dimethyl- und/oder Diethylcarbonat mit Capronalkohol, Caprylalkohol, 2-Ethylhexylalkohol, Caprinalkohol, Laurylalkohol, Isotride- cylalkohol, Myristylalkohol, Cetylalkohol, Palmoleylalkohol, Stearylalkohol, Isostearylalkohol, Oleylalko- hol, Elaidylalkohol, Petroselinylalkohol, Linolylalkohol, Linolenylalkohol, Elaeostearylalkohol, Arachidyl- alkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen anfallen. Bevorzugt geeignet Dialkylcarbonate abgeleitet von C-18-Pflanzenölen.

Weitere erfindungsgemäß geeignete Öle sind ausgewählt aus den Dicarbonsäureestern von linearen oder verzweigten $C_2$-$C_{10}$-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weiterhin geeignet sind Ölkörper aus Ester von linearen oder verzweigten, gesättigten oder ungesättigten Fettalkoholen mit 2-30 Kohlenstoffatomen mit linearen, oder verzweigten gesättigten oder ungesättigten Fettsäuren mit

2 - 30 Kohlenstoffatomen, die hydroxyliert sein können.
Weitere Öle, sind ausgewählt aus den Anlagerungsprodukten von Ethylenoxid und/oder Propylenoxid an ein- oder mehrwertige $C_{3-20}$-Alkanole wie Butanol, Butandiol, Myristylalkohol und Stearylalkohol, z. B. PPG-14-Butylether, PPG-9-Butylether, PPG-10-Butandiol, PPG-3-Myristylether und PPG-15-Stearylether.

Vorzugsweise werden Ölkörper eingesetzt, die auf C-18-Pflanzen basieren, wie beispielsweise Pflanzenöle und entsprechende Mono-/Di-/Triglyceridmischungen auf Basis von gesättigten und ungesättigten C6-C24-Fettsäuren, Ester von C6-C24-Fettalkoholen und/oder Fettalkohole mit Carbonsäuren oder Polyolen, wie zum Beispiel Zucker mit 2 bis 10 Kohlenstoffatomen und 2 bis 6 Hydroxylgruppen.
In einer bevorzugten Ausführungsform werden keine Silikone eingesetzt.

***Öle, Fette und Wachse***

Typische Beispiele für Fette und Öle sind Glyceride, d. h. feste oder flüssige pflanzliche oder tierische Produkte, die im Wesentlichen aus gemischten Glycerinestern höherer Fettsäuren bestehen. Als Wachse kommen u. a. natürliche Wachse, wie z. B. Bienenwachs, Bürzelfett, Candelillawachs, Carnaubawachs, Ceresin, Espartograswachs, Guarumawachs, Japanwachs, Korkwachs, Lanolin (Wollwachs), Mikrowachse, Montanwachs, Ozokerit (Erdwachs), Petrolatum, Paraffinwachse, Ouricourywachs, Reiskeimölwachs, Schellackwachs, Sonnenblumenwachs, Fruchtwachse wie Orangenwachse, Zitronenwachse, Grapefruitwachse, Zuckerrohrwachs, chemisch modifizierte Wachse (Hartwachse), wie z. B. Montanesterwachse, Sasolwachse, hydrierte Jojobawachse, hydriertes Rinzinusöl sowie synthetische Wachse, wie z. B. Polyalkylenwachse und Polyethylenglycolwachse in Frage.

Neben den Fetten kommen als Zusatzstoffe auch fettähnliche Substanzen, wie Lecithine und Phospholipide in Frage. Als Beispiele für natürliche Lecithine seien die Kephaline genannt. Daneben kommen auch Sphingosine bzw. Sphingolipide in Frage. Desweiteren sind auch Mischungen der genannten Öle und Wachse möglich. Erfinderisch besonders bevorzugt eingesetzt werden Pflanzenöle, bzw. Triglyceride, wie sie aus den Pflanzen der gemässigten Zonen isoliert werden können. Ganz besonders bevorzugt werden Öle der Pflanzen und Pflanzenfamilien wie sie in der Anmeldung im Abschnitt "C18-Pflanzen" beschrieben wurden.

Die Ölkörper können in den erfindungsgemässen Zubereitungen in einer Menge von 0.2 bis 80 Gew.-%, bevorzugt Mengen von 0.2 bis 70 Gew.-%, bezogen auf die Gesamtmenge der Zubereitung.

***Perlglanzwachse***

Als Perlglanzwachse kommen beispielsweise in Frage: Alkylenglycolester, speziell Ethylenglycoldistearat; Fettsäurealkanolamide, Partialglyceride, Ester von mehrwertigen, gegebenenfalls hydroxysubstituierte Carbonsäuren mit Fettalkoholen mit 6 bis 24 Kohlenstoffatomen, Fettstoffe, wie beispielsweise Fettalkohole, Fettketone, Fettaldehyde, Fettether und Fettcarbonate, die in Summe mindestens 24 Kohlenstoffatome aufweisen; Fettsäuren wie Stearinsäure, Hydroxystearinsäure oder Behensäure, Ringöffnungsprodukte von Olefinepoxiden mit 12 bis 24 Kohlenstoffatomen mit Fettalkoholen mit 12 bis 24 Kohlenstoffatomen und/oder Polyolen mit 2 bis 15 Kohlenstoffatomen und 2 bis 10 Hydroxylgruppen sowie deren Mischungen.

Erfindungsgemäss bevorzugt sind Perlglanzwachse basierend auf C18-Pflanzen.

Die Einsatzmenge der Perlglanzwachse kann - bezogen auf die Gesamtmenge der Zubereitung - bei 0.1 bis 5, vorzugsweise 0.5 bis 3 und insbesondere 1 bis 1.5 Gew.-% liegen.

**Weitere Inhaltsstoffe**

Neben den bisher genannten Komponenten können die erfindungsgemässen Zubereitungen weitere übliche Inhaltsstoffe für Kosmetika enthalten, die dem Fachmann durchaus bekannt sind.

Die erfindungsgemässen kosmetischen und dermatologischen Zubereitungen können dementsprechend ferner kosmetische Hilfsstoffe enthalten, wie sie üblicherweise in solchen Zubereitungen verwendet werden; beispielsweise, Desensibilisierende Zusätze (z.B. Hydroxylapatit, Arginin mit Calciumcarbonat, Zinn-, Kalium-, Strontiumchlorid, Kaliumnitrat u.a.), Konsistenzgeber, kationische Polymere, Filmbildner, Überfettungsmittel, Stabilisatoren, biogene Wirkstoffe, Trübungsmittel, ferner Eiweissderivate wie Gelatine, Collagenhydrolysate, Aminosäuren, Oligo- oder Polypeptide auf natürlicher und synthetischer Basis, DNA- oder RNA-Oligonucleotide, Desoxyzucker oder Desoxyzucker-Bausteine enthaltende Polysaccharide, Kreatin, Xanthin-Derivate, z.B. Coffein, Theophyllin, Theobromin, Aminophyllin, Eigelb, Honig, Lanolin und Lanolinderivate, Stoffe mit keratolytischer und keratoplastischer Wirkung, Enzyme und Trägersubstanzen, Pflanzenextrakte, Vitamine, Provitamine und deren Ester, Antiageing- Mittel, Füllstoffe, Pigmente, , Suspendiermittel, Puffergemische, oberflächenaktive Substanzen, weichmachende, anfeuchtende und/oder feuchthaltende Substanzen, entzündungshemmende Substanzen, Meristem Wirkstoffe, Flavonoide und flavonoid-reiche Pflanzenextrakte, Isoflavonoide und isoflavonoid-reiche Pflanzenextrakte, Ubichinon und ubichinonreiche Pflanzenextrakte, Silymarin, selbstbräunende Wirkstoffe, haarentfernende Wirkstoffe und haarwachstumanregende bzw. haarwuchsregulierende Wirkstoffe, sebumregulierende Wirkstoffe, hautaufhellende Wirkstoffe oder Weissmacher in Zahnpasten (z.B. Phosphate oder Papain), Lichtschutzmittel, Insektenrepellentien, Bakterizide, Salze, antimikrobiell, proteolytische oder wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte, sowie Gemische derselben.

Diese sind vorzugsweise in einer Menge von 0,001 - 20 Gew.-% in der Zubereitung enthalten.

**Schaum**

Das erfindungsgemässe Mittel bildet bereits ohne weitere Hilfsmittel einen stabilen Schaum. Weiterhin geeignet ist die zusätzliche Zugabe von Saponinen, beispielsweise Saponinen aus der indischen Waschnuss (Sapindus mukorossi), Koreanischen Ginsengs (Panax ginseng), Agavengewächsen, Inka-Gurke (Cyclanthera pedata), Süssholz *(Glycyrrhiza glabra),* Efeu (Hedera), Schlüsselblume (Primula veris), Vogelmiere (Stellaria media), Wald-Sanickel (Sanicula europaea), Dornige Hauhechel (Ononis spinosa), Hülsenfrüchten (Leguminosae), Spinat (Spinacia), Spargel (Asparagaceae), Hafer (Avena), (Ononis spinosa), Schattenblümchen (Maianthemum bifolium), Seifenkraut (Saponaria officinalis), Walnuss (Aesculus hippocastanum), Acker-Gauchheil (Anagallis arvensis), Gelber Hohlzahn (Galeopsis segetum), Karthäuser-Nelke (Dianthus carthusianorum), Ackerschachtelhalm (Equisetum arvense) und Seifenrinde (*Quillaja saponaria* Molina).

Die Menge an Saponinen beträgt üblicherweise bis zu 5 Gew.-%, vorzugsweise 0.001 bis 3 Gew.-%, insbesondere 0.01 bis 2 Gew.-%. (Gew.-% Aktivstoff bezogen auf das gesamte Mittel)

Die Saponine können in erfindungsgemässen Mittel frei mit anderen Inhaltsstoffen kombiniert werden.

pH- Stellmittel

Der pH-Wert des erfindungsgemässen Mittels kann mittels üblicher pH-Regulatoren eingestellt werden, wobei je nach Anwendung unterschiedliche pH-Bereiche von sauer (pH 0-4) zu neutral (pH 5-7) bis hin zu basisch (pH 8-14) eingestellt werden. Als pH-Stellmittel dienen Säuren und/oder Alkalien. Geeignete Säuren sind insbesondere organische Säuren wie die Ameisensäure, Essigsäure, Zitronensäure, Glycolsäure, Milchsäure, Bernsteinsäure, Adipinsäure, Apfelsäure, Weinsäure und Gluconsäure oder auch Amidosulfonsäure. Besonders bevorzugt sind Säuren, die aus pflanzlichen Rohstoffen gewonnen werden wie Essigsäure, Zitronensäure, Milchsäure, Apfelsäure und Weinsäure sowie die Mineralsäuren Salzsäure, Schwefelsäure und Salpetersäure bzw. deren Mischungen eingesetzt werden. Bevorzugte Basen stammen aus der Gruppe der Alkali-und Erdalkalimetallhydroxide und -carbonate. Daneben kann das Mittel Ammoniak und Alkanolamine enthalten.

Die Zubereitung ist über einen weiten pH-Bereich stabil, so werden unterschiedliche Ausführungsformen ermöglicht, wie zum Beispiel im kosmetischen Bereich Haarfärbebereich zwischen 5 und 11, Rasur/ Haarentfernung zwischen 3 und 10, Seifen, Deo und Gesichtswasser pH 5-9, insbesondere ein hautneutraler pH von 5.5. Vorzugsweise hat die Zubereitung einen pH-Wert zwischen 0-14, besonders bevorzugt zwischen 3 und 11.

Im Bereich der Detergenzien sind saure Reiniger wie Bad-, Sanitär-, oder WC-Reiniger, Neutralreiniger, wie Geschirrspülmittel, sowie alkalische Reiniger wie Fett- und Ölreiniger oder Waschmittel möglich.

**Lösungsvermittler**

Die erfindungsgemässen Mittel können neben den bereits genannten Stoffen weiterhin Lösungsvermittler, sog. Hydrotropika enthalten. Hierbei sind alle üblicherweise zu diesem Zweck in Wasch- und Reinigungsmitteln verwendeten Stoffe einsetzbar.

**Gerüststoffe**

Gerüststoffe, welche üblicherweise in Wasch- und Reinigungsmitteln eingesetzt werden, sind geeignet. Die Gerüststoffe können vom Fachmann im erfindungsgemässen Mittel frei mit anderen Inhaltsstoffen kombiniert werden. Besonders bevorzugt in dem erfindungsgemässen Mittel sind Gerüststoffe auf Basis erneuerbarer Rohstoffe, die aus Pflanzen der gemässigten Zone gewonnen werden können, wie zum Beispiel Polyaspartate, Polycarboxylate wie beispielsweise Citrate, sowie Gluconate, Succinate oder Malonate.

**Farb- und Duftstoffe**

Um den ästhetischen Eindruck des erfindungsgemässen Mittels zu verbessern, können dem erfindungsgemässen Mittel alle in Wasch- und Reinigungsmitteln üblichen Duft- und Farbstoffe zugesetzt werden.
Bevorzugte Farbstoffe und Duftstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- oder Reinigungsmittel. Die Farbstoffe weisen keine ausgeprägte Substantivität gegenüber Textilfasern oder harten Oberflächen aus und färben diese nicht an.

In einer weiteren bevorzugten Ausführungsform der Erfindung, werden wie in den Ausführungsbeispielen gezeigt, weder Farb-, noch Duftstoffe zugesetzt. Die Mittel weisen auch ohne Zugabe von Farb-oder Duftstoffen eine zufriedenstellende Ästhetik und einen angenehmen Duft aus, um so Ausführungsformen ohne Farb- und/oder Duftstoffe zu ermöglichen, wie beispielsweise für Konsumenten mit Allergien und/oder sensibler Haut.

Enzyme

Das Mittel zeigt eine so gute Reinigungsleistung, dass Enzyme entbehrlich sind. Das Mittel kann optional Enzyme enthalten, insbesondere in den Ausführungsformen der Textil-, Spezial- und Geschirrreinigung. Die Enzyme können in dem erfindungsgemässen Mittel vom Fachmann mit allen anderen hier genannten Inhaltsstoffen kombiniert werden. Vorzugsweise werden Proteasen, Lipasen, Amylasen, Hydrolasen und/oder Cellulasen eingesetzt. Sie können dem erfindungsgemässen Mittel in jeder nach dem Stand der Technik etablierten Form zugesetzt werden. Hierzu gehören bei flüssigen oder gelförmigen Mitteln insbesondere Lösungen der Enzyme, vorzugsweise hoch konzentriert, wasserarm und/oder mit Stabilisatoren versetzt. Des Weiteren können die Enzyme verkapselt angewendet werden.
Um ein in einem erfindungsgemässen Mittel enthaltenes Enzym vor Schädigungen wie beispielsweise Inaktivierung, Denaturierung oder Zerfall etwa durch physikalische Einflüsse, Oxidation oder proteolytische Spaltung zu schützen, können dem enzymhaltigen Mitteln Enzymstabilisatoren zugesetzt werden. Je nach Art des verwendeten Enzyms sind als Enzymstabilisatoren beispielsweise geeignet: Benzamidin-Hydrochlorid, Borax, Borsäuren, Boronsäuren oder deren Salze oder Ester, vor allem Derivate mit aromatischen Gruppen, etwa substituierte Phenylboronsäuren beziehungsweise deren Salze oder Ester; Peptidaldehyde, Aminoalkohole wie Mono-, Di-, Triethanol- und -Propanolamin und deren Mischungen, aliphatische Carbonsäuren bis zu C12, wie Bernsteinsäure, andere Dicarbonsäuren oder Salze der genannten Säuren; endgruppenverschlossene Fettsäureamidalkoxylate; niedere aliphatische Alkohole und vor allem Polyole, beispielsweise Glycerin, Ethylenglykol, Propylenglykol oder Sorbit; sowie Reduktionsmittel und Antioxidantien wie Natrium-Sulfit und reduzierende Zucker. Weitere geeignete Stabilisatoren sind aus dem Stand der Technik bekannt.

Erfindungsgemäss besonders geeignet sind biotechnologisch hergestellte Enzyme mithilfe von nicht genmodifizierten Organismen (non GMO), sowie Stabilisatoren auf Basis nachwachsender Rohstoffe und/oder mineralische Substanzen, beispielsweise die Borsäure und/oder Borax, reduzierende Zucker, Bernsteinsäure oder andere Dicarbonsäuren, Polyaminoverbindungen insbesondere auf Basis von natürlichen Aminosäuren.

Überraschenderweise wird in der enzymfreien Ausführungsform eine hervorragende Reinigungsleistung gegenüber Kohlenhydraten festgestellt, welche mit enzymhaltigen Mitteln im Markt durchaus vergleichbar ist, dies ist exemplarisch in den Ausführungsbeispielen offenbart.
Eine besonders bevorzugte Ausführungsform der Erfindung ist daher die ohne Cellulasen oder Amylasen, ganz besonders bevorzugt ist die enzymfreie Ausführungsform. Diese ist besonders vorteilhaft für Konsumenten mit Allergien und/oder sensibler Haut. Enzymfreie Ausführungsformen bei vergleichbarer Reinigungskraft sind in den Ausführungsbeispielen offenbart.

Viskosität

Die flüssige oder gelförmige Ausführungsform des erfindungsgemässen Mittels weist vorzugsweise eine Viskosität von 0.4 bis 10000 mPa.s. auf. Zu diesem Zweck kann das Mittel Viskositätsregulatoren enthalten. Die Menge an Viskositätsregulator beträgt üblicherweise bis zu 1.5 Gew.-%, vorzugsweise 0.001 bis 1.0 Gew.-%, insbesondere 0.01 bis 0.5 Gew.-%; Gew-% Aktivstoff bezogen auf das gesamte Mittel.

Geeignete Viskositätsregulatoren sind beispielsweise organische abgewandelte Naturstoffe (Carboxymethylcellulose und andere Celluloseether, Hydroxyethyl- und - propylcellulose und dergleichen, Kernmehlether), organische vollsynthetische Verdickungsmittel (Polyacryl- und Polymethacryl-Verbindungen, Vinylpolymere, Polycarbonsäuren, Polyether, Polyimine, Polyamide) und anorganische Verdickungsmittel (Polykieselsäuren, Schichtsilikate, Tonmineralien wie Montmorillonite, Zeolithe, Kieselsäuren), sowie organische natürliche Verdickungsmittel (Agar-Agar, Carrageen, Xanthan, Traganth, Gummi arabicum, Alginate, Pektine, Polyosen, Guar-Mehl, Johannisbrotbaumkernmehl, Stärke, Dextrine, Gelatine, Casein).

In einer bevorzugten Ausführungsform sind die Viskositätsregulatoren natürliche organische Verdickungsmittel aus pflanzlichen Rohstoffen - einschliesslich Algen - beispielsweise Polysaccharide wie Pektine oder Stärke.
In dieser Ausführungsform werden keinerlei organische vollsynthetischen Verdickungsmittel wie Polyacryl- und Polymethacryl-Verbindungen, Vinylpolymere, Polycarbonsäuren, Polyether, Polyimine, oder Polyamide eingesetzt. Bevorzugt sind zudem anorganische Verdickungsmittel.
Des Weiteren bevorzugt sind biotechnologisch hergestellte Verdickungsmittel mithilfe von nicht genmodifizierten Organismen (non GMO), wie beispielsweise Xanthan gum.
Bevorzugte erfindungsgemässen Mittel verdickt mit Xanthan gum sind in den Ausführungsbeispielen offenbart.
Für das erfindungsgemässe Mittel können die Viskositätsregulatoren vom Fachmann frei mit anderen hier genannten Inhaltsstoffen kombiniert werden.

Weitere Inhaltsstoffe

Neben den bisher genannten Komponenten kann das erfindungsgemässe Mittel weitere dem Fachmann bekannte Inhaltsstoffe enthalten, welche frei mit anderen hier genannten Inhaltsstoffen kombiniert werden können.

- Füll- und Hilfsstoffe wie Adsorptionsmittel, Bittermittel, Bleichmittel, Bügelhilfsmittel, weitere Basen, weitere Säuren, Einlaufverhinderer, Filmbildner, neutrale Füllsalze, Alkali- und Erdalkalisalze wie NaCl oder $MgSO_4$, weitere Gerüststoffe, Gleitmittel, Hydrotrope, weitere Lösungsmittel und Lösungsvermittler, Opacifier, Polymere, Puffer, Quellmittel, organische und anorganische Salze, Schauminhibitoren, Silikonöle, Co-Tenside, Viskositätsregulatoren, Wachse.
  Überraschenderweise zeigt das erfindungsgemässe Mittel eine sehr gute Reinigungsleistung gegenüber Farbflecken, wie in den Ausführungsbeispielen offenbart.
  Eine bevorzugte Ausführungsform ist daher ohne Bleichmittel.
- Prozesschemikalien wie Glyzerin, Vergällungsmittel, z.B. Methylethylketon, u.a., Stabilisatoren und Verunreinigungen bzw. Nebenkomponenten aus dem Herstellungsprozess.
  In einer bevorzugten Ausführungsform enthält das Mittel Glyzerin, welches als Nebenkomponente der Verseifungsreaktion der natürlichen Fettsäuren enthalten ist oder als Inhaltsstoff zusetzt wird, wobei der Anteil an Glyzerin bevorzugt zwischen 0.01 und 10 Gew.-%, besonders bevorzugt 0.01 bis 7 Gew.-% und ganz besonders bevorzugt 0.01 bis 3 Gew.-% beträgt, bezogen auf den Gesamtgehalt im Mittel.
- Funktionelle Mittel und Aktivstoffe wie Abrasiva, Antiredepositionsmittel, Antistatika, Bakterizide, Bleichaktivatoren, Desinfektionsmittel, Farbübertragungsinhibitoren, weitere Enzyme, Fluoreszenzmittel, Fungizide, Germizide, Hautschutz- und Hautpflegemittel, hydrophilierende Agenzien, Imprägniermittel, Insektizide, Knitterschutzmittel, Korrosionsinhibitoren, optische Aufheller, Oxidationsmittel und -katalysatoren, Parfümträger, Phobiermittel, probiotische Inhaltsstoffe, Schiebefestmittel , UV-Absorber, Vergrauungsinhibitoren, Wäschesteifen.
- Stabilisatoren wie Antioxidantien, Ascorbinsäure und Derivate, Tocopherol und Derivate, weitere antimikrobielle Wirkstoffe und weitere Konservierungsmittel,
- Duft- und Farbstoffe
- sowie Gemische derselben.

**Verfahren**

Ein weiterer Gegenstand der Erfindung bezieht sich auf ein Verfahren zur Reinigung und Pflege und zum Waschen gemäss Anspruch 6. Verfahren zur Reinigung zeichnen sich im allgemeinen dadurch aus, dass in mehreren Verfahrensschritten verschieden reinigungsaktive Substanzen auf das Reinigungsgut aufgebracht und nach der Einwirkzeit abgewaschen werden, oder dass das Reinigungsgut in sonstiger Weise mit einem Wasch-, Pflege-oder Reinigungsmittel oder einer Lösung dieses Mittels behandelt wird.
Bevorzugte Ausführungsvariante im Kosmetik-Bereich sind Rinse-off-Produkte zur Reinigung und Pflege.
Das Wasch- oder Reinigungsverfahren umfassend die Verfahrensschritte

a) Bereitstellen einer Wasch-, Pflege- oder Reinigungslösung umfassend ein Mittel gemäss den Ansprüchen 1-5,10-12.
b) in Kontakt bringen einer natürlichen oder hergestellten Oberfläche, eines Körpers oder Körperteils, wie Haut, Haare, Fell u.a., einer harten oder flexiblen Oberfläche, sowie Textilien, Teppiche oder Naturfasern mit der Waschlösung gemäss (a).

In dem beschriebenen Verfahren werden in der Ausführungsform des Waschmittels oder Waschzusatzes Temperaturen bis 90°C und weniger eingesetzt. Bevorzugt sind Temperaturen unter 60°C und ganz besonders bevorzugt sind Temperaturen, die zu Energiespargründen kein Aufheizen der Wassertemperatur benötigen (ca. 20°C). Diese Temperaturangaben beziehen sich auf die in den Waschschritten eingesetzten Temperaturen.
In einem weiteren Verfahren wird das Mittel auf festes Substrate, wie Tücher aus Textil, Verbundstoff, non woven, Vlies, Papier, Watte oder Filz u.a. aufgebracht. dar. Diese werden mit dem Mittel durch ein Press-, Tauch-,

Abstreif- oder Sprühverfahren imprägniert.

Alle Sachverhalte, Gegenstände und Ausführungsformen, die für die Mittel beschrieben sind, sind auch auf das Wasch- und Reinigungsverfahren sowie deren Verwendung anwendbar und umgekehrt.

## Verwendungen

Die Verwendungen sind in den Ansprüchen 7-9 offenbart. Wie oben beschrieben, betrifft die Erfindung auch die Verwendung des Mittels zum Verbessern der Wasch- oder Reinigungsleistung, insbesondere an Farbstoff -, Pigment- oder Kohlenhydratverschmutzungen.

Das erfindungsgemässe Mittel kann als oder zur Herstellung von Wasch- und Reinigungsmittel für Oberflächen aus natürlichen und hergestellten Materialien, harte und flexible, sowie Textilien, Teppiche oder Naturfasern verwendet werden. Zu den Wasch- und Reinigungsmitteln zählen im Rahmen der Erfindung ferner Waschhilfsmittel, die bei der manuellen oder maschinellen Reinigung zum eigentlichen Mittel zudosiert werden. Ferner zählen zu Waschmitteln im Rahmen der Erfindung auch Vor- und Nachbehandlungsmittel, also solche Mittel die vor der eigentlichen Reinigung angewendet werden, beispielsweise zum Anlösen von hartnäckigen Verschmutzungen.

Die Mittel können auf das Reinigungsgut aufgebracht werden, welches sich in Haushalt, Industrie, Gewerbe bzw. Institutionen, Hafenanlagen, sowie Industrie- und Freizeit-, sowie Sportanlagen finden. Bevorzugt wird das Mittel zur Reinigung von harten Oberflächen oder Textilien verwendet.

Harte Oberflächen im Sinne dieser Anmeldung sind dabei Fenster, Spiegel, sowie weitere Glasoberflächen, Oberflächen aus Keramik, Kunststoff, Metall oder Holz, eben oder uneben, lackiert sowie unlackiert, flexible Oberflächen sind beispielsweise Kunststoffplanen, Schaumstoffe, Haut, Erde oder andere.

Natürliche Oberflächen sind im Sinne dieser Anmeldung Oberflächen von Lebewesen, Mensch, Tier, Pflanzen oder Erdreich wie beispielsweise Haut, Haare, Erde, Pflanzen und deren Früchte oder Blätter, Leder.

Textilen und Fasern sind im Sinne der Anmeldung Stoffe, Kleidung, Polster, Teppiche, Garne, u.a.

Gegenstand dieser Anmeldung ist die weiterhin die Verwendung der erfindungsgemässen Zubereitungen als oder zur Herstellung von Reinigungs- und Pflegemitteln für Körper, Mund und Zähne, Haut und Haare sowie zur Tierpflege.

Insbesondere betrifft die Erfindung die Verwendung der Zubereitung zur Entfernung von Farb - oder Kohlenhydratverschmutzungen.

In einer bevorzugten Ausführungsform wird das Mittel in bei saurem pH zwischen 0 und 7, bevorzugt zwischen 1 und 6 eingesetzt und ganz besonders bevorzugt bei einem pH zwischen 2 und 4. Überraschenderweise zeigt die erfindungsgemässe Kombination eine ausgesprochen gute Eignung für saure Reiniger und zeigt insbesondere eine sehr gute Kalklösekraft bei saurem pH. Das erfindungsgemässe Mittel eignet sich dadurch für die Verwendung kalklösenden Wasch- oder Reinigungsmitteln besonders als Sanitärreiniger, WC-Reiniger, Entkalker, Klarspüler, Geschirrspülmittel, Entferner für Zement - und Kalkschleier, Urinstein, Bierstein, Moos, Algen, Rost, Wasserränder, Pilzflecken, Beläge von Messing und Kupfer, Ölflecken etc. und andere.

Bei kosmetischer Verwendung der erfindungsgemässen Mittel, eignet es sich für die Verwendung in Haarentfernern, medizinischen Produkten wie zum Beispiel gegen Schuppenflechte oder Neurodermitis, insbesondere zu deren Prävention, weiterhin für Haarfärbeprodukte und für die Verwendung im Intimbereich.

In einer anderen bevorzugten Ausführungsform wird das Mittel bei alkalischem pH zwischen 7 und 14 eingesetzt, bevorzugt zwischen 8 und 12 eingesetzt. Typische Beispiele für die Verwendungen bei alkalischem pH sind Waschmittel, Oberflächenreiniger, Küchenreiniger, Grill- und Ofenreiniger, Felgenreiniger und andere.

Typische Beispiele für kosmetische Verwendungen bei alkalischem pH sind Rasierseife, Haarfärbemittel, Hautreinigung auf Seifenbasis.

In einer weiteren bevorzugen Ausführungsform wird das Mittel bei neutralem pH zwischen 5 und 8 eingesetzt, z.B. wenn ein hautneutraler pH wünschenswert ist, wie beispielsweise in einem Geschirrspülmittel.

Kosmetische Anwendungen sind beispielsweise Handseife, Gesichtpflege, Gesichtswasser, Augen-Makeup-Entferner, Makeup Entferner, Shampoo, Duschzubereitungen, Kontaktlinsenreinigung und andere.

Die erfindungsgemässen Zubereitungen werden als oder für die Herstellung von Reinigungs- und Pflegemitteln verwendet, bevorzugt als kosmetisches, dermatologische oder medizinisches Reinigungs- und/oder Pflegemittel, welches direkt oder indirekt mit dem menschlichen Körper in Berührung kommt. Dies sind beispielsweise: Duschzubereitungen, Badezusätze, Handseifen, auch zur Grobreinigung, Handpasten, Intimreinigung, Reinigung des Augenbereichs, Kontaktlinsenreinigung, Makeup-Entferner, Augenmakeup-Entferner, Körperreinigung, Mund- und Zahnpflege, Haarshampoos, konditionierende Shampoos, Antischuppen-Shampoo, Babyshampoo, Reinigungs- und Pflegeprodukte bei Schuppenflechte, Neurodermitis, Akne; insbesondere für die Prävention, Körper-, bzw. Gesichtstonic, Gesichtslotion, Feuchttücher, auch mit pflegenden oder anderen Wirkstoffen, z.B. desodorierend, anti-ageing u.a., Haarentfernung und Rasierprodukte, Haar-Tonicen, Haarfärbeshampoos, Haarfärbemittel und Haarstyling-Zubereitungen, Mundpflegeprodukt (Mundhygieneprodukt), insbesondere in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Mundspray.

Eingeschlossen ist erfindungsgemäss die Tierpfege und -reinigung.

Die erfindungsgemässen Mittel eignen sich in der Verwendung für Reinigungs- und Waschzubereitungen wie beispielsweise Handseifen, Handgeschirrspülmittel, Maschinengeschirrspülmittel, Geschirrspülmaschinenreiniger, Waschmaschinenreiniger, Toilettenreiniger- bzw. WC-Reiniger, Universal- bzw. Allzweckreiniger, Küchenreiniger, Bad- bzw. Sanitärreiniger, Fussbodenreiniger, Backofen- und Grillreiniger, Glas- und Fensterreiniger, Metallputzmittel, Polster- und Teppichreiniger, Vollwaschmittel, Colorwaschmittel, Feinwaschmittel, Textilhilfsmittel, Vorbehandlungsmittel, Spezialwaschmittel und -reinigungsmittel, sowie weiteren Mitteln zur industriellen & gewerblichen, bzw. institutionellen Reinigung, Mittel für die Textil- und Faserbehandlung, Mittel der Lederbehandlung, sowie weitere Zubereitungsformen.

Im Sinne dieser Anmeldung kann das Mittel als Flüssigkeit, Lösung oder Dispersion, Emulsion, Lotion, Gel, Tunkflüssigkeit, Spray oder Schaum verwendet werden. So können sie z. B. eine Lösung, eine Emulsion (O/W), oder eine multiple Emulsion, beispielsweise vom Typ Wasser-in-Öl-in-Wasser (W/O/W), ein Gel, eine Hydrodispersion, eine lamellare Phase, eine flüssige isotrope Lösungsphase oder eine micellare Phase, darstellen. Es kann an Pulver, Granulat oder Tabs adsorbiert werden.

Die Mittel eignen sich hierbei sowohl zur verdünnten Anwendung, als auch zur direkten Applikation auf das zu reinigende Substrat. Es eignet sich zur direkten Applikation, als auch zur Anwendung über ein Hilfsmittel, wie z.B. ein Tuch. Eine besondere Produktform stellen feste Substrate, wie Tücher dar. Diese werden mit dem erfindungsgemässen Mittel getränkt, besprüht, bestrichen oder über ein anderes Verfahren aufgebraucht. Feste Substrate haben den Vorteil, dass in ihnen die Zubereitung bereits in der richtigen Dosierung vorgegeben ist. Dies kommt insbesondere dem Konsumentenwunsch der Convenience entgegen, sie sind einfach handhabbar, direkt zu verwenden ohne zusätzliche Arbeitsschritte und können auch unterwegs, z.B. auf Reisen gut angewendet werden, auch wenn kein Wasser zur Verfügung steht.

Bevorzugt ist die flüssige oder gelförmige Ausführungsform mit Wasser oder einem organischen Lösungsmittel, ganz besonders bevorzugt ist die wässrige Ausführungsform.

Weitere Aspekte der vorliegenden Erfindung ergeben sich aus den nachfolgenden Beispielen sowie den beigefügten Patentansprüchen.

**Ausführungsbeispiele:**

Für die Beispiele wurden folgende Tenside verwendet. Alle Konzentrationsangaben beziehen sich auf den Aktivgehalt der Inhaltsstoffe.

| | | |
|---|---|---|
| Tensid A | Natrium Olivenoil PEG-7 Carboxylat | Hallstar |
| Tensid R | Natrium laureth-5carboxylate | Akypo Foam RL 40, Kao |
| Biotensid 1 | Sophorolipid | Sophoclean, Soliance |
| Biotensid 2 | Sophorolipid | Soliance S Soliance |
| Biotensid 3 | Sophorolipid | Sophogreen Soliance |
| Biotensid 4 | Rhamnolipid | JBR 425 Jeneil |
| Biotensid 5 | Mannosylerythritollipide | via Ustilago maydis |
| Biotensid 6 | Cellobioselipid | via Ustilago maydis |
| Biotensid 7 | Trehaloselipid | via Rhodococcus |

Flecklösekraft

**Versuchsdurchführung auf Textilien:**

Vergleichende Waschtests wurden gemäss AISE Protokoll November 2013 durchgeführt.

Waschtemperatur: 40°C, Dosierung: 70 ml, Waschmaschinenbeladung: 3kg, Wasserhärte: 4,36 mmol $CaCO_3$/l (Hartwasser- Bedingungen)

Repräsentatives Fleckenset gemäss A.I.S.E. auf Baumwolle: Tee, Kaffee, Rotwein, Fruchtsaft, Tomatenpüree, Karotten Babybrei, Französischer Senf, Schokolade, Gras, Gras/Schlamm, Blut, Unbenutztes Motorenöl, Frittierfett, Make up. Standardisiertes angeschmutztes Testgewebe kann beispielsweise erworben werden bei EMPA (Eidgenössische Material- und Prüfanstalt Schweiz). Auswertung durch statistische Evaluierung der Waschergebnisse.

Für die Auswertung des Flecklösevermögens wurden alle Flecken statistisch ausgewertet und visuell nach einer Skala von 0 (sehr gut, Fleck nicht mehr sichtbar) bis 5 (ungenügend, unbehandelter Referenzfleck) ausgewertet.

Als Referenz wurde ein enzymhaltiges Marktprodukt mit EU Ecolabel verwendet.

Zur Beurteilung der Ergebnisse wurden die Flecken nach kohlenhydratreichen Flecken und Farbflecken (Pigmente und Farbstoffe) kategorisiert. Es wurde dann die statistische Waschleistung pro Fleckenart, die Gesamtwaschleistung als arithmetisches Mittel aller Flecken, die gemittelte Waschleistung auf überwiegend kohlenhydrathaltigen Flecken wie Cellulose oder Stärke sowie die gemittelte Waschleistung der überwiegend bleichbaren Flecken (Farbstoffflecken) der unterschiedlichen Versuchsrezeptur verglichen.

Die erfindungsgemässen Mittel zeigen überraschend gute Reinigung bei Kohlenhydratflecken wie Stärke und Cellulose, sowie auf Farbflecken (Tabelle 1).

Tabelle 1: Synergie A/ B1, Gesamttensidkonzentration 9%, pH = 9.7

| | 1 | 2 | 3 | 4 | Referenz A | Referenz B | Referenz C |
|---|---|---|---|---|---|---|---|
| Leinsamenfettsäuren, Na-Salze | - | | | 3.0% | Enzymhaltiges Marktprodukt: Tandil Voll Waschmittel mit EU Ecolabel: DE/006/022 | 9.0% | |
| Tensid A | - | 9.0% | 4.5% | 3.0% | | - | - |
| Tensid R | - | - | - | - | | - | 4.5% |
| Biotensid 1 | 9.0% | - | 4.5% | 3.0% | | - | 4.5% |
| Natriumcitrat | 1.0% | 1.0% | 1.0% | 1.0% | | 1.0% | 1.0% |
| Tetrasodium Glutamate Diacetate | 1.0% | 1.0% | 1.0% | 1.0% | | 1.0% | 1.0% |
| Natriumgluconat | 2.4% | 2.4% | 2.4% | 2.4% | | 2.4% | 2.4% |
| NaOH (10%-ig) | pH-Einst. | pH-Einst. | pH-Einst. | pH-Einst. | | pH- Einst. | pH- Einst. |
| Wasser | ad 100 | ad 100 | ad 100 | ad 100 | | ad 100 | ad 100 |
| **Waschergebnisse** | | | | | | | |
| Tee | 4 | 4 | 4 | 4 | 4 | 4 | 4 |
| Kaffee | 4 | 4 | 3 | 3 | 4 | 4 | 5 |
| Rotwein | 4 | 3 | 2 | 1 | 4 | 4 | 4 |
| Fruchtsaft | 1 | 1 | 0 | 0 | 1 | 1 | 2 |
| Tomate | 2 | 1 | 1 | 1 | 1 | 2 | 3 |
| Karotte | 2 | 2 | 1 | 0 | 2 | 2 | 3 |
| Senf | 3 | 3 | 2 | 2 | 3 | 3 | 4 |
| Schokolade | 2 | 2 | 2 | 2 | 2 | 3 | 3 |
| Gras | 3 | 3 | 2 | 1 | 3 | 4 | 4 |
| Erde | 1 | 3 | 1 | 1 | 1 | 4 | 2 |
| Blut | 4 | 3 | 3 | 3 | 3 | 3 | 4 |
| Motorenöl | 2 | 2 | 2 | 2 | 1 | 3 | 2 |
| Butter | 0 | 0 | 0 | 0 | 0 | 0 | 0 |
| Make-up | 4 | 3 | 4 | 4 | 4 | 4 | 4 |
| Kugelschreiber | 4 | 4 | 2 | 2 | 4 | 4 | 2 |
| Bratensosse | 2 | 1 | 1 | 0 | 1 | 2 | 2 |
| Eigelb | 1 | 1 | 0 | 0 | 1 | 1 | 2 |

(fortgesetzt)

| **Gemittelte Waschleistung** | | | | | | | |
|---|---|---|---|---|---|---|---|
| alle Flecken | 2.53 | 2.35 | 1.76 | 1.53 | 2.29 | 2.82 | 2.94 |
| Kohlenhydratflecken | 2.2 | 1.8 | 1.2 | 0.7 | 1.8 | 2.3 | 3.0 |
| Pigmentflecken | 3.4 | 3.6 | 2.4 | 2.2 | 3.4 | 4.0 | 3.4 |
| Kohlenhydrate: Stärke, Cellulose (Fruchtsaft, Tomatenpüree, Karotte, Senf, Gras, Bratensosse)<br>Anfärbungen: bleichbare Flecken (Tee, Kaffee, Rotwein, Erde, Kugelschreiber) | | | | | | | |

Ergebnis: Die Formulierungen mit der synergistischen Kombinationen der Tenside A (Tabelle 1) mit den Glycolipid-Biotensiden (erfindungsgemässe Beispiele 3, 4) übertreffen im Bereich der kohlenhydrathaltigen Flecken deutlich die Waschleistung der Einzeltensiden (Bsp 1 und 2) und übertreffen - trotz Verzicht auf kohlenhydratabbauende Enzyme wie Amylasen oder Cellulasen - sogar das enzymhaltige Referenz-Marktprodukt (Referenz A).

Um Effekte durch die Waschbasis auszuschliessen und die spezifischen Effekte der erfindungsgemässen Tensidkombination darzulegen, wird zudem Referenz B (Waschlösung nur mit Seife) in Tabelle 1 abgebildet.

Referenz C (Tabelle 1) und Referenz D (Tabelle 2) zeigt die Kombination des Biotensids mit einem vergleichbaren Polyoxyalkylen Carboxylat auf Laurinsäurebasis aus Palmöl (Sodium laureth-5-carboxylate). In den Referenzbeispielen wird keine Synergie mit dem Biotensid festgestellt.

Ähnliche Ergebnisse werden mit dem Biotensid 4 erzielt, wie in Tabelle 2 dargestellt.

Tabelle 2: Synergie A/ B4, Gesamttensidkonzentration 9%, pH = 9.7

| | 5 | 6 | 7 | Ref D |
|---|---|---|---|---|
| Leinsamenfettsäuren, Na-Salze | - | | 3.0% | |
| Tensid A | - | 4.5% | 3.0% | |
| Tensid R | | | | 4.5% |
| Biotensid 4 | 9.0% | 4.5% | 3.0% | 4.5% |
| Natriumcitrat | 1.0% | 1.0% | 1.0% | 1.0% |
| Tetrasodium Glutamate Diacetate | 1.0% | 1.0% | 1.0% | 1.0% |
| Natriumgluconat | 2.4% | 2.4% | 2.4% | 2.4% |
| NaOH (10%-ig) | pH- Einst. | pH- Einst. | pH- Einst. | pH- Einst. |
| Wasser | ad 100 | ad 100 | ad 100 | ad 100 |
| **Gemittelte Waschleistung** | | | | |
| alle Flecken | 2.47 | 1.71 | 1.53 | 2.71 |
| Kohlenhydratflecken | 2.2 | 1.0 | 0.7 | 2.5 |
| Pigmentflecken | 3.2 | 2.4 | 2.2 | 3.6 |
| Kohlenhydrate: Stärke, Cellulose (Fruchtsaft, Tomatenpüree, Karotte, Senf, Gras, Bratensosse)<br>Anfärbungen: bleichbare Flecken (Tee, Kaffee, Rotwein, Erde, Kugelschreiber) | | | | |

Generell zeigen die erfindungsgemässen Mittel zudem eine unerwartete synergistische Waschkraftverbesserung gegenüber den Vergleichsrezepturen im Bereich der bleichbaren Flecken (Farbflecken). Diese Flecken sind im Allgemeinen nicht wasserlöslich. Eine besonders erhöhte Waschleistung wird bei Kugelschreiber, Kaffee und Rotwein erzielt. Trotz Verzicht auf ökologisch oft bedenkliche Bleichmittel, zeigen die erfindungsgemässen Mittel eine ausserordentlich gute Reinigungsleistung. Weitere Beispiele nach Zugabe eines 3. Tensids sind in Tabelle 3 abgebildet.

**3. Tensid (D)**

Tabelle 3: Beispiele mit einem zusätzlichen nichtionischen Tensid

| | 8 | 9 | 10 | 11 |
|---|---|---|---|---|
| Tensid A | 4.5% | 3.0% | 2.0% | 3.0% |
| Biotensid 1 | | | | |
| Biotensid 3 | 4.5% | | | |
| Biotensid 4 | | 3.0% | 4.0% | 3.0% |
| PEG-4 Rapssamenamid | | | | 3.0% |
| Rapssamen methylester oxylat, 7 EO | | | 2.0% | |
| Olivenöl Glycereth-PEG-8-ester | | 3.0% | | |
| Mandelöl PEG-7 esters | 1.0% | | | |
| Natriumcitrat | 1.0% | 1.0% | 1.0% | 1.0% |
| Tetrasodium Glutamate Diacetate | 1.0% | 1.0% | 1.0% | 1.0% |
| Natriumgluconat | 2.4% | 2.4% | 2.4% | 2.4% |
| NaOH (10%-ig) | pH- Einst. | pH- Einst. | pH- Einst. | pH- Einst. |
| Wasser | ad 100 | ad 100 | ad 100 | ad 100 |
| **Gemittelte Waschleistung** | | | | |
| alle Flecken | 1.53 | 1.59 | 1.47 | 1.53 |
| Kohlenhydratflecken | 1.3 | 1.0 | 1.0 | 1.2 |
| Pigmentflecken | 1.8 | 2.2 | 2.4 | 2.2 |
| Kohlenhydrate: Stärke, Cellulose (Fruchtsaft, Tomatenpüree, Karotte, Senf, Gras, Bratensosse)<br>Anfärbungen: bleichbare Flecken (Tee, Kaffee, Rotwein, Erde, Kugelschreiber) | | | | |

**Reinigungskraft Farbstoffe, Pigmente, Make-up**

Versuchsdurchführung: Das Make-up wird bei mehreren Versuchspersonen als Fleck von ca. 1 cm Durchmesser auf die Haut (Arm, oder Bein) aufgetragen und 5 min getrocknet. Als Testsubstanzen wurden typisches Augen- und Gesichtsmakeup verwendet (Lidschatten L'Oreal luminous; Mascara L'Oreal Volume Million Lashes extra black, wasserlöslich; Make-up der Löscher, Silikonbasis; Maybelline, Kajal Expression Maybelline). Anschliessend wird ein Kosmetiktuch mit 1 ml Testlösung getränkt und der Fleck ohne Druck einmalig abgewischt und beurteilt. Anschliessend wird versucht den Fleck vollständig zu entfernen. Die Versuche werden nach folgender Bewertungsskala von 0 bis 4 bewertet (Tabelle 3) und statistisch ausgewertet. Die gemittelten und gerundeten Ergebnisse sind der Tabelle 4 zu entnehmen.

Tabelle 3: Bewertungsskala Makeup-Entferner

| Bewertungsskala | Ergebnis bei einmaliges Wischen | Notwendige Schritte zurvollständigen Entfernung |
|---|---|---|
| 4 | Farbe leicht angelöst | Nicht möglich |
| 3 | Farbe angelöst, Streifen | Kräftiges Reiben mit Druck notwendig |
| 2 | Farbe deutlich angelöst | Leichtes Reiben mit Druck notwendig |
| 1 | Farbe fast vollständig entfernt | Sanftes 2-3 maliges Wischen ohne Druck |
| 0 | Farbe vollständig entfernt | Sanftes 1-maliges Reiben ohne Druck |

Tabelle 4: Synergistische Reinigungswirkung zur Makeup-Entfernung Gesamttensidkonzentration 8% in Wasser

| | Tensid A | Biotensid B | A/B = 1:1 |
|---|---|---|---|
| Lidschatten | 1 | 2 | 0 |
| Make-up | 4 | 3 | 2 |
| Mascara (wasserlösl.) | 3 | 4 | 3 |

(fortgesetzt)

| | Tensid A | Biotensid B | A/B = 1:1 |
|---|---|---|---|
| Lidstrich Kajal | 2 | 4 | 2 |
| | 2.50 | 3.25 | 1.75 |

Ergebnis: Überraschenderweise wird eine synergistische Wirkung zwischen Tensid A und Biotensid B (hier beispielhaft Biotensid B1) festgestellt. Für alle repräsentativen Makeup-Kategorien wurde eine gleich gute oder bessere Reinigungswirkung für die erfindungsgemässe Kombination beider Tenside festgestellt. Besonders markant sind diese Ergebnisse für Lidschatten und Makeup auf Basis von Farbstoffen und Pigmenten. Betrachtet man das gemittelte Gesamtergebnis, so liegt die erfindungsgemässe Kombination bei 1.1 Bewertungspunkten über der Bewertung der gemittelten Einzeltenside, was einer Verbesserung des Gesamtreinigungseffektes von 22 % entspricht.

Die Durchführung mit anderen Biotensiden lieferte analoge Ergebnisse, bspw. liefert das Biotensid-Glycolipid 4 ein Gesamtergebnis von 3.3 als 8%-ige Lösung, während die 1:1-Mischung mit Tensid (A) eine Gesamtwaschkraft von 2.0 aufweist. Bei weiteren Versuchen wurde weiterhin bestätigt, dass die Synergie für andere Farb- bzw. Pigmentreinigung wie bspw. Kugelschreiber, Erde, etc. auch auf unterschiedlichen Oberflächen gegeben ist. Exemplarisch sind einige Beispielrezepturen bei den Ausführungsbeispielen offenbart.

**Schaum in Abhängigkeit des Tensidverhältnisses**

Biotenside zeichnen sich durch ein recht geringes Schaumverhalten aus. In manchen Verwendungen sind jedoch schäumende Mittel erwünscht. Es ist daher wünschenswert durch geeignete Tensidkombinationen von Biotensiden schäumende Mittel zu erhalten. Tensid A ist für ein gutes Schaumverhalten, jedoch primär in Kombination mit dem ebenfalls gut schäumenden Sodium laureth sulfat, wo die Schaumbildung in allen Kombinationsverhältnissen unverändert hoch ist. Das Verhalten von Tensid A in Kombination mit den wenig schäumenden Biotensiden in Abhängigkeit der Tensidverhältnisse ist nicht vorhersagbar.

Die Schaumbildung wird bestimmt durch unmittelbares Messen der Schaumhöhe nach Schütteln der Testmischung. Die Schaumstabilität wird bestimmt durch Messen der Schaumhöhe nach 15 min. Die Mittelwerte aus 5 Bestimmungen sind in den Diagrammen 1-2 aufgezeigt.

Diagramm 1: Schaumbildung (am Beispiel von Biotensid B1)

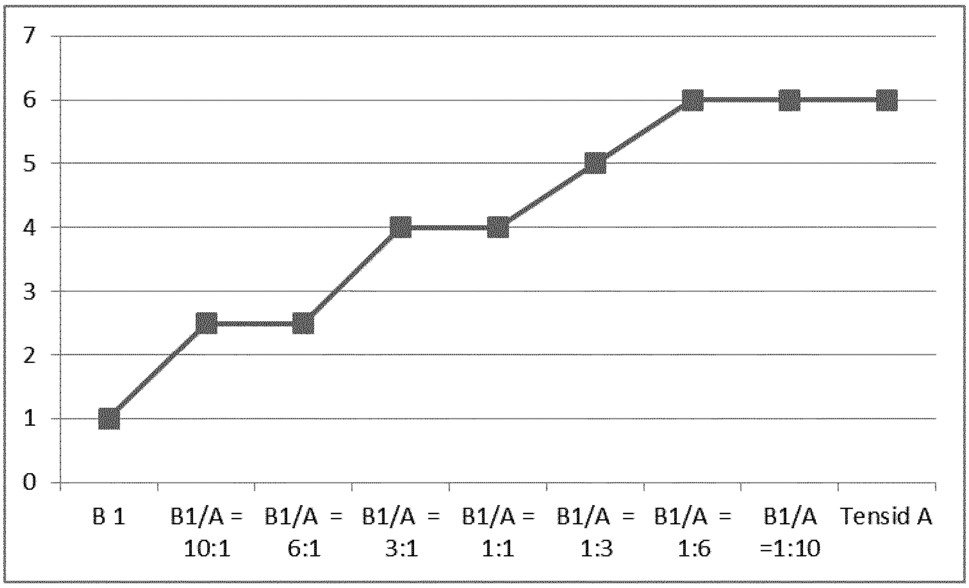


Ergebnis: Tensid A zeigt eine ca. 6x höhere Schaumentwicklung als Biotensid 1. Die erfindungsgemässen Mischungen verhalten sich betreffend Schaumbildung näherungsweise additiv.

Diagramm 2: Schaumstabilität nach 15 min (am Beispiel B1)

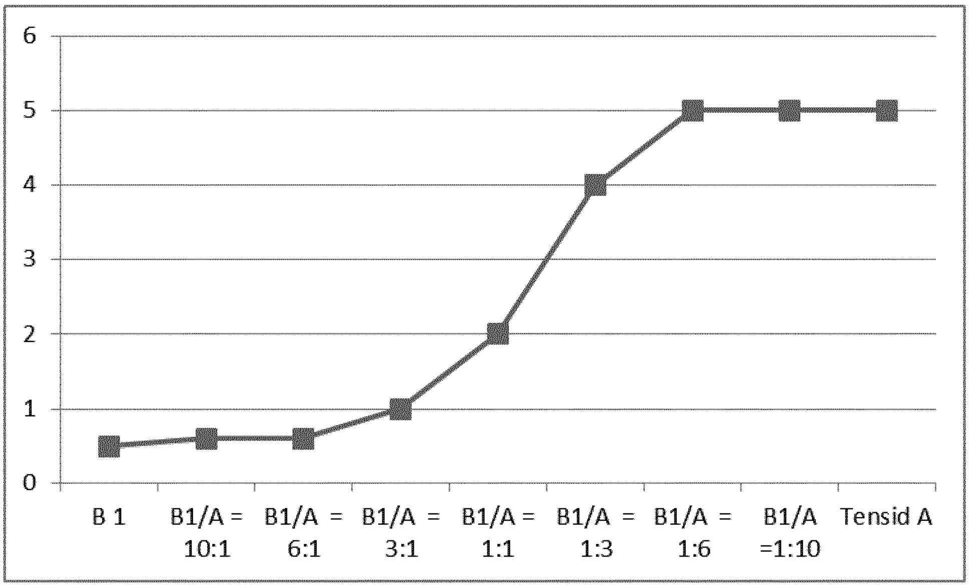


Ergebnis: Tensid A zeigt überraschenderweise eine schaumstabilisierende Wirkung auf B1 beginnend bei einem Verhältnis von A: B $\geq$ 1:6.

Ab einem Verhältnis von A: B $\geq$ 6:1 entspricht die Schaumhöhe dem von Tensid A.

Ein weiterer Vorteil der Kombination von Biotensiden mit Tensid A ist, dass einige der Biotenside, z.B. B1 und B2, in Wasser absetzen können. Durch die Kombination mit Tensid A erhält man stabile, transparente Lösungen in Wasser.

**Kohlenhydratwaschkraft in Abhängigkeit des Tensidverhältnisses**

Versuchsdurchführung: Zur Bestimmung des optimalen Tensidverhältnisses auf die Waschkraft von Kohlenhydraten wurde über eine 2.5%-ige Tensidlösung in Wasser gemessen. Hierzu wurde eine definierte Menge an Saccharose/Glucosegemisch verdichtet und eingefärbt, bei 20°C in die Testlösung eingebracht und die Zeit bestimmt, nach der die Tablette ohne Rühren oder andere mechanische Einwirkung vollständig aufgelöst ist (= Waschkraft).

Auswertung: Zur vergleichenden Bestimmung der Waschkraft der Kohlenhydrate werden die Versuchsreihen jeweils auf die Waschkraft desjenigen Tensids standardisiert, welche die bessere Waschkraft auf Kohlenhydrate zeigt, hier Tensid A. Die Testwerte der Tensidmischungen werden relativ zur Waschkraft des Standards angegeben, wobei das aufgeführte Ergebnis jeweils der Mittelwert der Einzelergebnisse pro Versuchsreihe ist (Diagramm 3).

Diagramm 3: Waschkraft auf Kohlenhydrate in Abhängigkeit des Tensidverhältnisses Gesamtkonzentration an Tensiden jeweils 2.5%, hier am Beispiel Biotensid 1

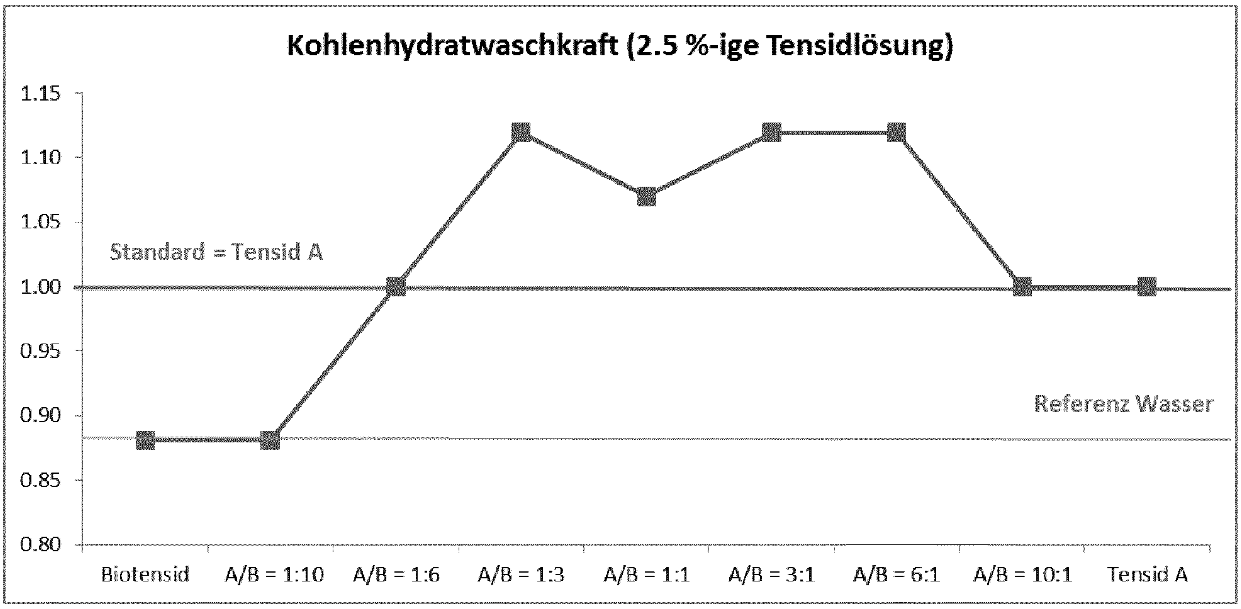


Ergebnis: In einem Verhältnis A : B ≥ 10:1 und A : B ≤ 1:10 wird hier kein Effekt auf die Waschkraft von Kohlenhydraten festgestellt. Die Waschkraft entspricht jeweils dem entsprechenden Tensid, welches im Überschuss vorhanden ist. Die synergistische Wirkung wird deutlich in dem Bereich zwischen A:B ≥ 1:6 und A:B ≤ 10:1. Dort zeigen die erfindungsgemässen Mischungen eine Waschkraft, die - bei gleicher Konzentration - deutlich über der Waschkraft der Einzeltenside liegt. Als Referenz wurde reines Wasser gewählt. Die Waschkraft von reinem Wasser liegt bei 88% bezogen auf Tensid A (Standard) und entspricht dem Wert des reinen Biotensids B1.

Nach demselben Verfahren wurde die Kohlenhydratwaschkraft einer 1%-igen Tensidlösung Tensid A und Biotensid B1 im Vergleich zu einer 1%-igen Tensidlösung Tensid R und Biotensid B1, jeweils im Verhältnis 1:1 gemessen. Tensid A und R sind beides Polyoxyethylen Carboxylate, mit dem Hauptunterschied, dass Tensid A von einer C-18-Pflanze abgeleitet ist, während Referenztensid R auf Basis von Kokosfettsäuren beruht (Tabelle 5), wiederum bezogen auf Tensid A als Standard = 100%.

Tabelle 5: Kohlenhydratwaschkraft Tensid A versus Referenztensid (1%-ige Gesamttensidkonzentration in Wasser)

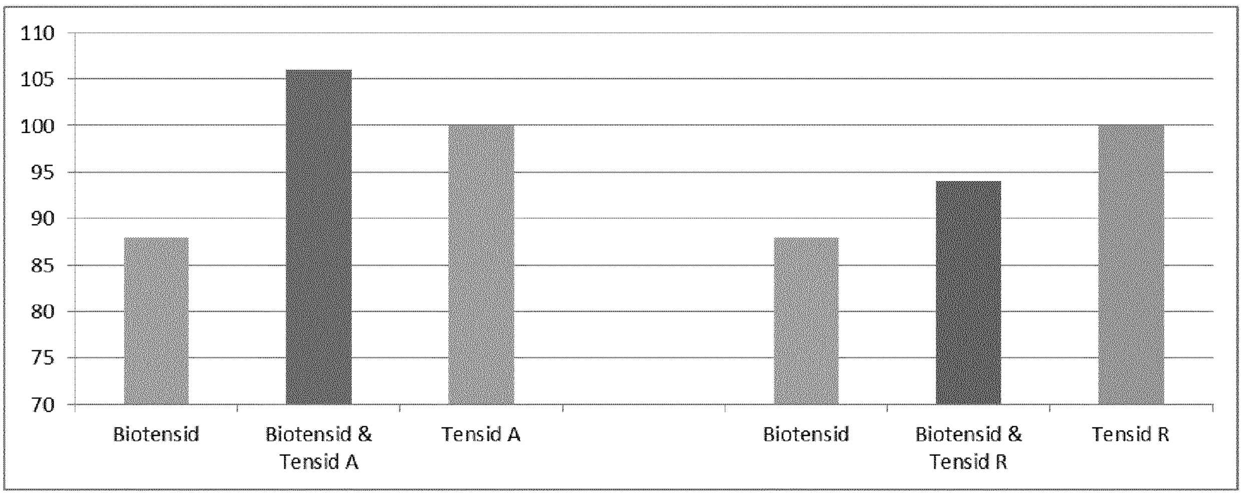


Ergebnis: Tensid A und Tensid R zeigen als alleinige Tenside vergleichbare Waschkraft auf Kohlenhydrate. Kombiniert

man jedoch die Tenside, erhält man eine erhöhte Waschkraft bei Tensid A in Kombination mit Biotensid B1, während die Kombination mit Tensid R eine reduzierte Waschkraft aufweist. Nur die Kombination mit Tensid A verhält sich synergistisch.

Die Versuche wurden auch mit anderen Kohlenhydratarten, wie z.B. Sorbit mit vergleichbaren Ergebnissen durchgeführt.

Insbesondere überraschend ist in diesen Versuchen, dass die synergistische Wirkung bereits bei niedrigen Konzentrationen von nur 1% Tensidgemisch in Lösung festgestellt werden kann.

**Saurer pH-Wert**

Die synergistische Wirkung zur Entfernung von Kohlenhydratschmutz oder Farbstoffschmutz/Kugelschreiber kann weiterhin in der Reinigung von harten oder flexiblen, natürlichen oder synthetischen Oberflächen verwendet werden, auch bei saurem pH.

Voraussetzung für diese Anwendung ist, neben der Stabilität der Mittel im Sauren, dass die Tensidmischung die Wirkung einer Säure auf Kalk nicht negativ beeinflusst.

Versuchsdurchführung:

Die Methode zur Bestimmung des Kalklösevermögens ist angelehnt an die Empfehlungen zur Qualitätsbewertung für Badezimmerreiniger bzw. WC- Reiniger der IKW (Industrieverband Körperpflege- und Waschmittel e. V.) und basiert auf der quantitativen Bestimmung des Gewichtsverlusts an $CaCO_3$ (Marmor) nach Tauchen der Marmorplatten für 10 min in die Testflüssigkeit.

Auswertung: Zur vergleichenden Bestimmung des Kalklösevermögens werden die Versuchsreihen jeweils auf das Kalklösevermögen des verwendeten Biotensids (= Referenz 100%) standardisiert. Der Testwert der Tensidmischungen wird relativ zum Kalklösevermögen der Referenz angegeben, wobei das aufgeführte Ergebnis jeweils der Mittelwert der Einzelergebnisse pro Versuchsreihe ist.

Das Kalklösevermögen der erfindungsgemässen Mittel (hier exemplarisch in dem Verhältnis Tensid A/ Glycolipid Biotensid B 1 = 1:1; 1%-ige Tensidlösung) werden in 5,3%-iger Zitronensäure gegenüber der Referenz R, basierend auf Kokosfettsäuren, ermittelt.

**Tabelle 11:** Kalklösekraft 1%-ige Tensidlösungen mit Zitronensäure bei pH = 2.1-2, hier bezogen auf das Kalklösevermögen der 1%-igen Biotensidlösung

| | B1 | A | A:B1 = 1:1 | R | R:B1 = 1:1 |
|---|---|---|---|---|---|
| Tensid A | | 1% | 0.50% | | |
| Tensid R | | | | 1% | 0.50% |
| Biotensid B1 | 1% | | 0.50% | | 0.50% |
| Zitronensäure | 5.30% | 5.30% | 5.30% | 5.30% | 5.30% |
| Wasser | ad 100 | ad 100 | ad 100 | ad 100 | ad 100 |
| Kalklösevermögen | 100% | 86% | 97% | 95% | 60% |

**Tabelle 12:** Kalklösekraft Tensid A + Biotensid im

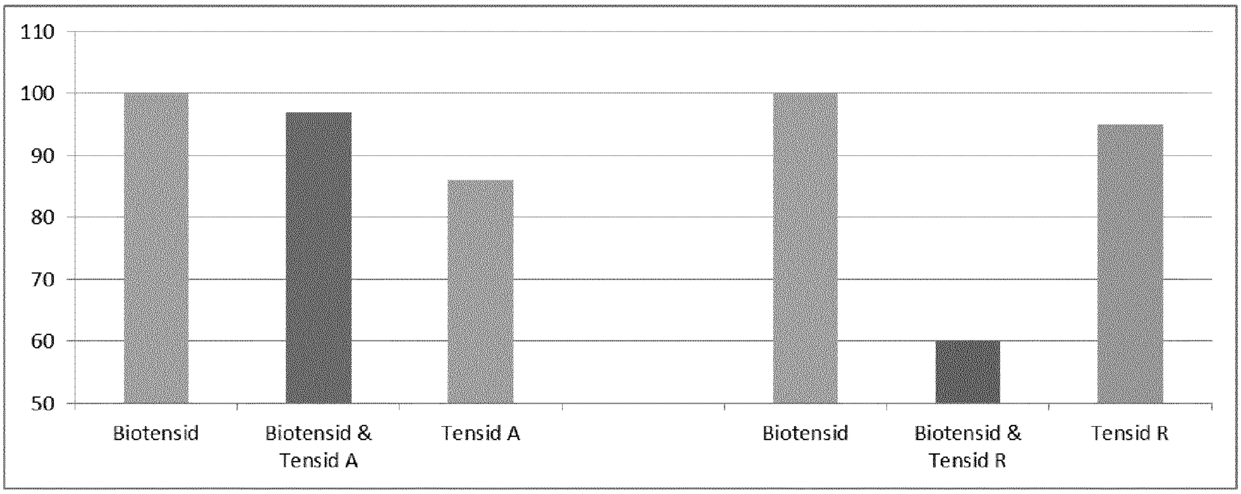


Ergebnis: Häufig wird die Kalklösekraft einer Säurelösung durch Zugabe von Tensiden vermindert, insbesondere bei Zugabe von mehreren Tensiden, welche zusätzlich durch Interaktionen zwischen den Tensiden und Kalk beeinflussen.

Die erfindungsgemässen Mittel dagegen (hier exemplarisch in dem Verhältnis Tensid A/ Glycolipid Biotensid B 1 = 1:1; 1%-ige Tensidlösung) erhalten erstaunlicherweise annährend die Kalklösekraft der Säurelösung. Durch Versuche mit anderen Biotensiden konnte dies generell bestätigt werden..

Anders verhält sich dies bei den üblicherweise eingesetzten C-12-Tensiden: Werden die Untersuchungen mit analogen C12-Tensiden der analogen Tensidklasse R durchgeführt, wird die Kalklösekraft erheblich gemindert und liegt nur noch bei ca. 60% der Kalklösekraft der Einzelkomponenten (Tabelle 12).

Die 1%-ige Tensidkonzentration mit Zitronensäure bei pH = 2.1- 2.2 ist repräsentativ für saure Reiniger wie z.B. Oberflächenreiniger, WC-Reiniger oder Badreiniger.

**Weitere Beispiele**

Im Folgenden sind erfindungsgemässe Beispielformulierungen offenbart. In den Tabellen 13-23 finden sich folgende Beispiele für kosmetische Zubereitungen und Reinigungsmittel. Die Angaben beziehen sich jeweils auf Gew.-% des Aktivstoffes im gesamten Mittel.

Tabelle 13 und 14: Reinigungszubereitungen für Haut- und Haar:

| | |
|---|---|
| 1-4 | Shampoo mit Wirkstoffen |
| 5 | Nährendes Schampoo |
| 6 | Conditionierendes Shampoo |
| 7 | Reinigungszubereitung mit Seife |
| 8 | Mildes Shampoo |
| 9-11 | Handreinigung |
| 12, 13 | Badezusätze |

Tabelle 15: Gesichtspflege-Produkte

| | |
|---|---|
| 14-16 | Makeup Entferner |
| 17-19 | Reinigende Gesichtsmilch |

Tabelle 16: Pflegende Waschlotionen, insbesondere als Flüssigkeit für Feuchttücher

| | |
|---|---|
| 20- 32 | Reinigende Lotionen |

Tabelle 17: Haarpflege (ohne Konservierungsmittel)

| | |
|---|---|
| 33 | Haarkur |
| 34, 35 | Konditioner |
| 39 | Haarfarbe |
| 40 | Tönungsshampoo |

Tabelle 18: Rasierprodukte

Tabelle 18: Produkte zur Mundhygiene

Tabelle 20: Alkalische Reiniger und Waschmittel bspw. für die Verwendung als Grill- oder Ofenreiniger, Felgenreiniger, Fettreiniger, Oberflächenreiniger, u.a

Tabelle 21: Reiniger mit Lösungsmitteln bspw. Oberflächenreiniger, Anti-Graffiti- Reiniger, Glas- oder Fensterreiniger, Reinigungslösungen für Feuchttücher u.a.

Tabelle 22: Reiniger bei neutralem pH bspw. für die Verwendung als Neutralreiniger, Oberflächenreiniger, Geschirrspülmittel

Tabelle 23: Saure Reiniger, bspw. für die Verwendung als WC-Reiniger, Sanitärreiniger, Badreiniger, Kalklöser, u.a.

**Verwendete Inhaltsstoffe**

Leinsamenfettsäuren, Cremer
PEG-40-sunflower-glyceride, Levenol SR 152, Kao
Natriumgluconat, Akzo Nobel
PEG-4 Rapssamenamid
Rapssamen methylester oxylat, 7 EO
Raps methyl ester, UCY
Propylenglycol, Dow
Lemon oil, Symrise
Propanol, Dow
Butoxypropanol, Dow
Walnussschalen, Walnut Shell Powder 40/100, Elementis Specialties
Saponin, Baja YE, Desert King
Limonene, Symrise
Saponin (Saponaria officinalis), Organic Soapwort Extract - BCE4523, Biocosmethic
Tetranatrium Glutamate Diacetat, Akzo Nobel
Tetranatrium Iminosuccinat, Lanxess
Xanthan gum, Keltron, CP Kelco
Cocamid DEA Rewomid DC 212 S, Evonik Industries
Panthenol, BASF
Tocopheryl Acetat, BASF
Sodium Lactat, Evonik Industries
Hydrolyzed Wheat Protein, Gluadin WLM, BASF
Zinc Pyrithion, Lonza
Glycerin, Cremer
Hydroxypropylmethylcellulose, Evonik
Brassicoamidopropyldimethylamine, Kao
Mandelöl- PEG Glyceridester, Mulsifan, Zschimmer & Schwarz
1,2-Propandiol, Dow
EDTA, Dow
Teebaumöl, Lucas Meyer

Brennnessel Extract, Phytotex Nettle, Crodarom
Rosemary Officinalis, Rosmary Eco, Provital
Sonnenblumenöl Glycereth-8 ester
Rapsmethylester, UCY
Sojamethylester, Stepan
Limonen, Symrise
Walnussschalenmehl, Walnut Shell Powder 40/100, Elementis Specialties
Carboxmethylcellulose, BASF
Tetrasodium Iminodisuccinat, Lanxess
Ethylendiamine-N,N-disuccinic acid,
Aprikosenkernöl, Refined Apricot Kernel Oil - BCE1021, Biocosmethic
Nachtkerzenöl, Refined Evening Primrose Oil - BCE1014, Biocosmethic
Cathamus Tinctorius Öl, Seatons Safflower Oil, Croda
Mandelöl, Refined Sweet Almond Oil - BCE1082, Biocosmethic
Aloe Barbadensis Gel, Aloe Vera Gel - Eco Provital Group
Lecithin, Bergasom sun 75, Berg & Schmidt
Bentonite, Gelwhite H, Eckart Effect Pigments
PEG-20-Sojabohnenöl
Haarfarben, BASF
Natrium GLDA, Akzo Nobel
Herbasec Henna, Lipoid Cosmetics
Beta Vulgaris Wurzelextrakt, Day Moist CLR, CLR Chemisches Laboratorium Dr. Kurt Richter GmbH
Oenothera Biennis Öl, Organic Evening Primrose Oil - BCE1516, Biocosmethic
1,3- Propanol, Dow
Chitosan, Marine Biopolymer, Lucas Meyer
Triethylcitrat, Jungbunzlauer
Salicylsäure, A& E Connock
Niacinamid, Lonza
Matrixyl Synthe 6, Sederma
Glycerin (and) Aqua (and) Malpighia Glabra (Acerola) Fruit Extract, Fruitliquid, Crodarom
Dimethyl glutarate, Solvay
Kalium Sorbate, Tri-K Industries
Hydrated Silica, Evonik
Sorbitol 70% Food Grade, Global Starch
Zinklactat, Jungbunzlauer
Xylit, Foodchem
Natrium Gluconate, Jungbunzlauer
Titandioxid, AEROXIDE® TIO2 P 25, Evonik Corporation Silica
Natrium saccharinate/Saccharine, I.H.C. Chempharm
Beta Glucan, Clariant
Citrus Paradisi (Grapefruit) Peel Extract, Solid Extract Grapefruit FR-O, Frutarome Industries Ltd.
Natrium Benzoate, I.H.C. Chempharm
Camellia Oleifera Seed Oil, Organic Camellia Oil - BCE1542, Biocosmethic
Natrium Glucoheptonate, Akzo Nobel

Tabelle 13: Beispiele für Reinigungszubereitungen für Haut- und Haar

| | 1 | 2 | 3 | 4 | 5 | 6 | 7 | 8 |
|---|---|---|---|---|---|---|---|---|
| Biotensid 3 | 3.5 | | 3.5 | | | | 3 | |
| Biotensid 4 | | 2.5 | | | | | | 5 |
| Biotensid 5 | | | | 2.5 | | | | |
| Biotensid 6 | | | | | | 1.5 | | |
| Biotensid 7 | | | | | 2.5 | | | |
| Tensid A | 3 | 2.5 | 1.5 | 2.5 | 2.5 | 5 | 8 | 5 |

(fortgesetzt)

| | 1 | 2 | 3 | 4 | 5 | 6 | 7 | 8 |
|---|---|---|---|---|---|---|---|---|
| Kaliumsalze der Sonnenblumenfettsäuren | | | | | | | 2 | |
| PEG-4 Rapssamenamid | 2 | 2 | | | | | | |
| Brassicamidopropyl dimethylamine | | | | | | 1.5 | | |
| Mandelöl Glycereth-8 ester | | | 0.5 | | 2 | | 2 | |
| Sonnenblumen PEG-8 Ester | | | | | | | | 2 |
| Saponin | | | | 2 | | | | |
| Brassica alcohol | | | | | | | 0.4 | |
| Glycerin | | | 0.5 | 0.5 | | | 3 | 3 |
| 1,2-Propandiol | | 0.5 | | | 0.5 | 0.5 | | |
| Tetranatrium GLDA | 0.2 | 0.2 | | | | | | |
| Zinc Omadine | 2 | 2 | | | | | | |
| Piroctone Olamine | | | | | | | | |
| Teebaumöl | | | | 0.5 | | | | |
| Brennessel Extrakt | | | 2 | | | | | |
| Rosmarinus Officinalis (Rosemary) Blätterextrakt | | | | | 5 | 5 | | |
| Panthenol | | | | | | | 1 | 1 |
| Optional: Verdicker, Parfum, Konservierung, silikone, pH-Stellmittel, Actives, weitere Actives | | | | | | | | |
| Wasser | ad 100 | ad 100 | ad 100 | ad 100 | ad 100 | ad 100 | ad 100 | ad 100 |

Tabelle 14: Beispiele für Reinigungszubereitungen für Haut- und Haar

| | 9 | 10 | 11 | 12 | 13 |
|---|---|---|---|---|---|
| Biotensid 2 | | | 0.2 | | |
| Biotensid 3 | | 6 | 0.6 | | |
| Biotensid 4 | 6 | | | | |
| Biotensid 5 | | | | | 3 |
| Biotensid 6 | | | | 3 | |
| Tensid A | 1.5 | 1.5 | 4 | 5 | 1 |
| Natrium Olivenölfettsäuren | | | 0.5 | | |
| PEG-4 Rapssamenamid | 1.5 | 1.5 | | 8 | |
| Sonnenblumenöl PEG-20 | 4 | 4 | | | 5 |
| Sonnenblumenöl Glycereth-8 ester | 1.5 | | | | 10 |
| Mandelöl PEG-7 ester | | 1.5 | | | |
| Ethoxylierter Rapsmethylester (7EO) | | | 5 | | |
| Apri kosenkernöl | | | | | 10 |
| Saponin | | | | 2 | |

(fortgesetzt)

| | 9 | 10 | 11 | 12 | 13 |
|---|---|---|---|---|---|
| Ethanol | | 7 | | | |
| Rapsmethylester | 0.3 | | | | |
| Sojamethylester | | 0.3 | | | |
| Limonene | | 0.5 | | | |
| Walnussschalenmehl | 15 | | | | |
| Quarzsand | | | 35 | | |
| Aloe Vera extract | | | | 0.3 | |
| Hydrolisiertes Weizenprotein | | | | 4 | |
| Lecithin | | | | | 0.5 |
| Carboxymethylcellulose | 0.6 | 0.6 | | | |
| Natriumcarbonat | | | 0.5 | | |
| Tetrasodium iminodisuccinate (IDS) | | 0.2 | | | |
| Ethylendiamine-N,N'-disuccinic acid (EDDS) | | | 0.2 | | |
| Panthenol | | | | | 0.5 |
| Optional: Verdicker, Parfum, Konservierung, silikone, pH-Stellmittel, Actives | | | | | |
| Wasser | ad 100 | ad 100 | ad 100 | ad 100 | ad 100 |

Tabelle 15: Beispiele für Gesichtspflege-Produkte

| | 14 | 15 | 16 | 17 | 18 | 19 |
|---|---|---|---|---|---|---|
| Biotensid 1 | | 3 | | | | |
| Biotensid 2 | 0.5 | | | | | |
| Biotensid 3 | | | | 5 | | |
| Biotensid 4 | | | | | | 15 |
| Biotensid 5 | | | 2 | | | |
| Biotensid 6 | | | | | | 0.5 |
| Biotensid 7 | | | | | 0.3 | |
| Tensid A | 3 | 0.8 | 0.6 | 1 | 0.6 | 5 |
| Aprikosenkernöl | | | | 5 | | |
| Nachtkernzenöl | | | | | 5 | |
| Cathamus Tinctorius Öl | | | | | | 40 |
| Mandelöl | | | | 5 | 5 | |
| PEG-4 Rapssamenamid | | | | | | 3 |
| Sonnenblumenöl Glycereth-8 ester | 1.75 | 5 | | | | 20 |
| Alkohol denat. | | | | 20 | 20 | |
| Glycerin | 2 | | | 10 | 10 | |
| 1,2-Propandiol | 6 | | 10 | 5 | 5 | 5 |
| Limonene | | | | 0.001 | 0.001 | |

(fortgesetzt)

| | 14 | 15 | 16 | 17 | 18 | 19 |
|---|---|---|---|---|---|---|
| Aloe Barbadensis Gel | 0.05 | | 0.05 | 0.05 | 0.05 | |
| Lecithin | | | | 0.5 | 0.5 | 0.5 |
| Hydrolisiertes Weizenprotein | | | | 0.2 | 0.2 | |
| Bentonite | | | | 0.1 | 0.1 | |
| Panthenol | | 0.5 | | 0.1 | 0.1 | |
| Optional: Verdicker, Parfum, Konservierung, silikone, pH-Stellmittel, Actives | | | | | | |
| Wasser | ad 100 | ad 100 | ad 100 | ad 100 | ad 100 | ad 100 |

Tabelle 16: Beispiele für pflegende Waschlotionen, insbesondere als Flüssigkeit für Feuchttücher

| | 20 | 21 | 22 | 23 | 24 | 25 | 26 | 27 | 28 | 31 | 32 |
|---|---|---|---|---|---|---|---|---|---|---|---|
| Biotensid 1 | | | | | | | 5 | | | | |
| Biotensid 2 | 3 | | 2 | | 2 | | | | | | |
| Biotensid 3 | | | | | 0.1 | | | | | | |
| Biotensid 4 | | 2 | | 5 | | 2 | | 5 | | | |
| Biotensid 5 | | | | | | | | | 5 | | |
| Biotensid 6 | | | | | | | | | | | 3 |
| Biotensid 7 | | | | | | | | | | 3 | |
| Tensid A | 0.8 | 0.6 | 0.6 | 1 | 0.6 | 0.6 | 5 | 5 | 5 | 1 | 1 |
| Olivenöl glycereth-8 esters | | | | | 2 | 3 | | | 10 | | |
| Rapsmethylester | | | | | | | | 10 | | | |
| Oenothera Biennis Öl | | | | | | | | | | 10 | 10 |
| Alkohol | 25 | 40 | | | | | 40 | 40 | | | |
| Glycerin | | | | | 1 | | 10 | | 40 | 5 | 5 |
| 1,2-Propandiol | | 3 | ad 100 | ad 100 | ad 100 | 5 | | | | 2 | 2 |
| Chitosan | 0.2 | | | | | | | | | | |
| Glycolsäure | 0.08 | 0.04 | | | | | | | | | |
| Triethylcitrat | | 2 | | | | | | | | | |
| Niacinamid | | | 0.1 | 0.1 | | | | | | | |
| Salicylsäure | | | 0.1 | 0.1 | | | | | | | |
| Aloe Barbadensis Gel | | | | | | | | | | 0.2 | 0.2 |
| Hydroxy Propyl Methyl Cellulose | 0.8 | | | | | | | | | | |
| Matrixyl Synthe 6 | | | | | 2 | | | | | | |
| Glycerin (and) Aqua (and) Malpighia Glabra (Acerola) Fruit Extract | | | | | | 3 | | | | | |
| Dimethyl glutarate | | | | | | | 40 | 40 | 40 | | |
| Potassium sorbate | | | | | 0.1 | 0.2 | | | | 0.2 | 0.2 |

(fortgesetzt)

| | 20 | 21 | 22 | 23 | 24 | 25 | 26 | 27 | 28 | 31 | 32 |
|---|---|---|---|---|---|---|---|---|---|---|---|
| Optional: Verdicker, Parfum, Konservierung, silikone, pH-Stellmittel, Actives | | | | | | | | | | | |
| Wasser | ad 100 | ad 100 | - | - | | ad 100 | | | | ad 100 | ad 100 |

Tabelle 17: Beispiele für die Haarpflege

| | 33 | 34 | 35 | 39 | 40 |
|---|---|---|---|---|---|
| Biotensid 1 | | | | | 4 |
| Biotensid 3 | 0.5 | | | | |
| Biotensid 4 | | 2 | | | |
| Biotensid 5 | | | 2 | | |
| Biotensid 6 | | | | 3 | |
| Tensid A | 2 | 7 | 5 | 0.6 | 2 |
| PEG-4 Rapssamenamid | | | | | 3 |
| Hydrolisiertes Mandelprotein | 0.6 | | | | |
| Hydrolisiertes Weizenprotein | 0.2 | | | | 0.2 |
| Weizenkeimöl PEG-8 ester | 0.5 | 0.3 | 0.3 | | |
| Brassicamidopropyl dimethylamine | | 0.9 | 0.9 | | |
| Ethanol | 10 | | | 7 | |
| Tocopherol | 0.2 | | | | |
| Propylenglycol | | 2 | 2 | 2 | |
| Herbasec Henna | | | | 5 | |
| Beta Vulgaris Wurzelextract | | | | 0.2 | |
| Juglans Regia Schalenextrakt | | | | | 0.2 |
| Mignonette Tree extract | | | | 0.4 | 0.2 |
| Optional: Verdicker, Parfum, Silikone, pH-Stellmittel, Actives | | | | | |
| Wasser | ad 100 | ad 100 | ad 100 | ad 100 | ad 100 |

Tabelle 18: Beispiele für Rasierprodukte

| | 41 | 42 | 45 | 46 | 47 | 48 |
|---|---|---|---|---|---|---|
| Biotensid 2 | | | | | 0.3 | |
| Biotensid 3 | | | | | | 5 |
| Biotensid 4 | 3.5 | | | | | |
| Biotensid 5 | | 6 | | | | |
| Biotensid 6 | | | 2 | | | |
| Biotensid 7 | | | | 2 | | |
| Tensid A | 1.5 | 3 | 5 | 5 | 0.6 | 5 |

(fortgesetzt)

| | 41 | 42 | 45 | 46 | 47 | 48 |
|---|---|---|---|---|---|---|
| Olivenöl Fettsäuren | | | | 5 | | 5 |
| Natrium/Kalium Olivenölseife | 18 | | | | 25 | |
| Monoethanolamin | | | | 1.3 | | |
| Nachtkerzenöl | 5 | 10 | 0.5 | | | |
| Arnicaöl | | 10 | | | | 3 |
| Camellia Oleifera Seed Oil | | 10 | | | | |
| PEG-4 Rapssamenamid | | | 1 | 2 | | |
| Sorbitan olivate | | | | | | 5 |
| PEG-20 sonnenblumenöl | | | 4 | | | |
| Traubenkernöl Glycereth-8 ester | | | | 0.2 | | |
| Glycerin | 5 | | | 5 | 10 | 2 |
| Natrium Glucoheptonate | | | | 0.2 | | |
| Tetranatrium GLDA | | | | | | 0.3 |
| Natrium Glucuronat | | | | | 0.2 | |
| Panthenol | 0.5 | | 0.2 | | | |
| Allantoin | 0.5 | | | | 10 | |
| Aloe Vera | | | 0.2 | | | 0.3 |
| Hydroxypropyl Methylcellulose | | | | 0.7 | | |
| Optional: Verdicker, Parfum, Konservierung, silikone, pH-Stellmittel, Actives, | | | | | | |
| Wasser | ad 100 | ad 100 | ad 100 | ad 100 | ad 100 | ad 100 |

Tabelle 19: Beispiele für Produkte zur Mundhygiene

| | 49 | 50 | 51 | 52 | 53 | 54 | 55 | 56 | 57 |
|---|---|---|---|---|---|---|---|---|---|
| Biotensid 2 | 3 | | | | | 0.8 | | | |
| Biotensid 3 | | | | | | | 0.5 | | |
| Biotensid 4 | | 3 | | | | | | | |
| Biotensid 5 | | | 3 | | | | | 0.5 | |
| Biotensid 6 | | | | 1.5 | | | | | 0.5 |
| Biotensid 7 | | | | | 3 | | | | |
| Tensid A | 0.6 | 0.7 | 0.7 | 0.25 | 0.9 | 0.6 | 0.6 | 0.25 | 0.25 |
| Glycerin | 60 | 10 | | | 10 | | 13 | | |
| Hydrated Silica | 20 | 25 | 25 | 25 | 25 | | | | |
| Natrium fluorid | | 0.2 | 0.1 | 0.2 | | | | | 0.2 |
| Sorbitol | | 35 | 35 | 20 | 20 | | | | 5 |
| Meersalz | | | 7 | | | | | | |

(fortgesetzt)

| | 49 | 50 | 51 | 52 | 53 | 54 | 55 | 56 | 57 |
|---|---|---|---|---|---|---|---|---|---|
| Pflanzenextrakte (z.B. Kamille, Pfefferminze, Myrrhe, Thymian, Salbei, etc.) | 1.2 | | 0.8 | | | 3.5 | | | |
| Alkohol | 0.3 | | | | | | | 15 | |
| 1,2-Propandiol | | | | | | | | | 5 |
| Xanthan gum | | 1 | 0.8 | | | | | | |
| hydroxycellulose | | 1 | | 1.3 | 1 | | | | |
| Natrium gluconate | | 0.8 | | | | | | | |
| Titandioxid | | 0.8 | | | | | | | |
| Natrium saccharinate | | 0.7 | | | 0.5 | | | | 0.2 |
| Chitosan | | | | | 0.1 | | 0.1 | | |
| ß- Glucan | | | | | 0.3 | | | | |
| Citrus Paradisi (Grapefruit) Peel Extract | | | 0.1 | | | | | 0.2 | |
| Saccharin | | 0.1 | | 0.1 | | | | | |
| Natrium benzoate | | 0.2 | | | | 0.2 | | | 0.2 |
| optional: Geschmacksstoffe, Farbstoffe, pH-Stellmittel | | | | | | | | | |
| Wasser | ad 100 | ad 100 | ad 100 | ad 100 | ad 100 | ad 100 | ad 100 | ad 100 | ad 100 |

Tabelle 20: Beispiele für alkalische Reiniger

| | 58 | 59 | 60 | 61 | 62 | 63 | 64 | 65 | 66 | 67 | 68 | 69 |
|---|---|---|---|---|---|---|---|---|---|---|---|---|
| Biotensid 1 | 2.0% | | | | | 1.0% | | 1.0% | | | | |
| Biotensid 4 | | 2.0% | | | | | 0.5% | | 1.0% | | | |
| Biotensid 5 | | | 2.0% | | | | | | | 0.5% | | |
| Biotensid 6 | | | | 2.0% | | | | | | | 1.0% | |
| Biotensid 7 | | | | | 2.0% | | | | | | | 1.0% |
| Tensid A | 0.6% | 0.6% | 1.5% | 3.0% | 1.5% | 0.7% | 1.0% | 0.7% | 0.7% | 1.5% | 1.0% | 1.0% |
| Olivenölseife (Kaliumsalz) | | | | | | | | | | 0.5% | 5.0% | 2.0% |
| PEG-4 Rapssamenamid | | | | | | | 0.5% | | | | | |
| Olive oil glycereth-8 ester | | | | | | | | 0.2% | | | | |
| Rapssamen methylester oxylat, 7 EO | 0.2% | | | | | | | | 0.2% | | | |
| Natriumgluconat | 1.2% | 1.2% | 1.2% | 1.2% | 1.2% | | | | | 1.2% | 0.5% | |
| Natriumcarbonat | | | | | | 0.2% | 0.2% | 0.2% | 0.2% | | | |
| Tetrasodium Glutamate Diacetate | 1.0% | 1.0% | 1.0% | 1.0% | 1.0% | | | 1.3% | 0.5% | 0.5% | 0.5% | 0.5% |
| Ethanol | 5.0% | 5.0% | 5.0% | 5.0% | 5.0% | | | | | | 3.0% | |
| Propylenglycol | | | | | | | | | 3.0% | | | |
| Lemon oil | | | | | | 0.5% | 0.5% | | | | | |
| Milchsäure | | | | | | | | | | pH = 8.5 | pH = 8.5 | pH = 8.5 |
| Wasser | ad 100 | ad 100 | ad 100 | ad 100 | ad 100 | ad 100 | ad 100 | ad 100 | ad 100 | ad 100 | ad 100 | ad 100 |

Tabelle 21: Beispiele für Reiniger mit Lösungsmittel

| | 70 | 71 | 72 | 73 | 74 | 75 | 76 | 77 | 78 | 79 | 82 | 83 | 84 |
|---|---|---|---|---|---|---|---|---|---|---|---|---|---|
| Biotensid 1 | 1.5% | 0.8% | 0.8% | 1.2% | 1.5% | 1.0% | 0.2% | | | | | | |
| Biotensid 5 | | | | | | | | 0.8% | | | 1.1% | | |
| Biotensid 6 | | | | | | | | | 0.8% | | | 2.0% | |
| Biotensid 7 | | | | | | | | | | 0.8% | | | 2.0% |
| Tensid A | 1.0% | 1.0% | 1.2% | 0.8% | 1.0% | 1.0% | 0.3% | 0.8% | 4.0% | 0.6% | 2.0% | 2.0% | 2.0% |
| Rapssamen methylester oxylat, 7 EO | | | | | | 1.5% | | | | | | | |
| Raps methyl ester | | | | 2.0% | 2.0% | | | | | 2.0% | 2.0% | 2.0% | |
| Ethanol | 15.0% | 97.8% | | | 15.0% | 96.0% | 5.0% | | 15.0% | | 94.5% | | |
| Propylenglycol | | | 97.6% | 95.6% | | | | 8.0% | | 15.0% | | 93.8% | 95.6% |
| Butoxypropanol | | | | | | | 0.5% | | | | | | |
| Limonene | 0.2% | 0.4% | 0.4% | 0.4% | 0.2% | 0.5% | | 0.4% | 0.2% | 0.3% | 0.4% | 0.2% | 0.4% |
| Wasser | ad 100 | | | | ad 100 | | ad 100 | ad 100 | ad 100 | ad 100 | | | |

Tabelle 22 (Fortsetzung): Beispiele für Reiniger mit Lösungsmittel

| | 85 | 86 | 87 | 88 | 89 | 90 | 91 |
|---|---|---|---|---|---|---|---|
| Biotensid 4 | 1.5% | 0.8% | 2.0% | 1.2% | 4.0% | 5.0% | 0.2% |
| Tensid A | 0.6% | 1.0% | 0.3% | 0.8% | 3.5% | 1.0% | 0.3% |
| IDS | 0.5% | 0.1% | | | | | |
| PEG-4 Rapssamenamid | 1.0% | | 0.3% | | | | |
| Raps methyl ester | | | | 2.0% | 2.0% | | |
| Ethanol | 15.0% | 97.8% | | | 15.0% | 89.0% | 5.0% |
| Propylenglycol | | | 94.4% | | | | |
| Glycerin | | | 3.0% | | | | |
| Propanol | | | | 95.6% | | | |
| Butoxypropanol | | | | | | | 0.3% |
| Limonene | | 0.3% | | 0.4% | 0.2% | 5.0% | |
| Kaliumsorbat | | | 0.2% | | | | 0.2% |
| Wasser | ad 100 | | | | ad 100 | | ad 100 |

Tabelle 21: Beispiele für Reiniger bei pH 5.5 -7.0

| | 92 | 93 | 94 | 95 | 96 | 97 | 98 | 99 | 100 | 101 | 102 | 103 |
|---|---|---|---|---|---|---|---|---|---|---|---|---|
| Biotensid 1 | 0.5% | 5.0% | 2.0% | 4.0% | | 3.0% | | | | | | |
| Biotensid 2 | | | | | | | | 0.5% | | | | |
| Biotensid 3 | | | | | | | 2.0% | | | | | |
| Biotensid 4 | | | | | | 3.0% | | | 4.0% | | | |
| Biotensid 5 | | | | | 2.0% | | | | | 1.5% | | |
| Biotensid 6 | | | | | | | | | | | 1.5% | |
| Biotensid 7 | | | | | | | | | | | | 2.0% |
| Tensid A | 1.0% | 2.0% | 2.0% | 4.0% | 2.0% | 2.0% | 4.0% | 1.0% | 2.0% | 0.8% | 0.8% | 2.0% |
| Olive oil Glycereth-PEG-8-ester | 4.6% | | | | | | 2.0% | | | | 2.0% | |
| PEG-4 Rapssamenamid | | | | 4.0% | | | | | | | | 0.8% |
| Rapssamenmethylester Oxylat 7 EO | | | 3.0% | | | | | | | 3.0% | | |
| Saponin (Saponaria officinalis) | | | | | 3.0% | | | 4.6% | 3.0% | | | |
| Natriumcitrat | 1.0% | | 1.0% | | | 1.0% | 1.0% | | | | 1.0% | |
| Tetranatrium Glutamate Diacetat | 0.5% | | | | | | | | | | 0.2% | |
| Tetranatrium Iminosuccinat | | 0.5% | | | | | | | | | | 0.5% |
| Natriumlactat | | | | | 1.0% | | | 1.0% | | | | |
| Alkohol | | 2.0% | | | | | | | 3.0% | | 3.0% | |
| Propylenglycol | | | | | | | | | 0.3% | 0.3% | | 0.3% |
| Limonen | | 0.2% | | | | | | | | | 0.1% | |
| Milchsäure | pH-Einst. | pH-Einst. | pH-Einst. | pH-Einst. | pH-Einst. | pH-Einst. | pH-Einst. | pH-Einst. | pH-Einst. | pH-Einst. | pH-Einst. | pH-Einst. |
| Wasser | ad 100 | ad 100 | ad 100 | ad 100 | ad 100 | ad 100 | ad 100 | ad 100 | ad 100 | ad 100 | ad 100 | ad 100 |

Tabelle 22: Beispiele für saure Reiniger

| | 104 | 105 | 106 | 107 | 108 | 109 | 110 | 111 | 112 | 113 | 114 | 115 |
|---|---|---|---|---|---|---|---|---|---|---|---|---|
| Biotensid 1 | 3.0% | 5.0% | | | 3.0% | | 2.0% | | | | | 0.5% |
| Biotensid 2 | | | | | | | | | | 0.5% | | |
| Biotensid 4 | | | 5.0% | | | | | 2.0% | | | 2.0% | 0.5% |
| Biotensid 5 | | | | | | 1.5% | | | | | | |
| Biotensid 6 | | | | 1.5% | | | | | | | | |
| Biotensid 7 | | | | | | | | | 0.5% | | | |
| Tensid A | 1.0% | 2.5% | 2.5% | 1.5% | 1.5% | 1.5% | 0.6% | 0.6% | 1.5% | 0.6% | 1.0% | 0.6% |
| Leinsamenfettsäuren | | | | | | 0.1% | | | | | | |
| PEG-4 Rapssamenamid | | | | | 0.5% | | | | | | | |
| Rapssamen methylester oxylat, 7 EO | | | | | | | | | | 0.2% | | |
| Olive oil Glycereth-PEG-8-ester | 0.2% | | | | | | | | | | | |
| Zitronensäure | 1.9% | | | | | | 15.0% | 1.9% | 1.9% | 1.9% | 15.0% | 1.9% |
| Milchsäure | 0.9% | 3.8% | 3.8% | 3.8% | 3.8% | 3.8% | | 0.9% | 0.9% | 0.9% | | 0.9% |
| Ameisensäure | | | | 0.9% | | | | | | | | |
| Xanthan gum | | 0.4% | | | 0.2% | | | 0.4% | | | | |
| Ethanol | | | | | | | 8.0% | | | | 8.0% | |
| Wasser | ad 100% | ad 100% | ad 100% | ad 100% | ad 100% | ad 100% | ad 100% | ad 100% | ad 100% | ad 100% | ad 100% | ad 100% |

## Patentansprüche

1. Mittel enthaltend mindestens ein alkoxyliertes Tensid (A) aus der Gruppe der Polyoxyalkylen Carboxylate der Formel (I) und mindestens ein Glycolipid-Biotensid (B) umfassend Rhamnolipide ,Sophorolipide, Mannosylerythritollipide, Cellobioselipide und Trehaloselipide, wobei das oder die Tenside (A) in einem Verhältnis von ≥ 1: 6 zu den Biotensiden (B) vorliegen; bezogen auf Gewichtsprozent Aktivstoff im gesamten Mittel;

(I) $R'-O(C_bH_{2b}O)_o-\{CH_2-CH[O(C_bH_{2b}O)_pR'']-CH_2O\}_r-(C_bH_{2b}O)_q-R'''$

mit

**b** einer ganzen Zahl zwischen 2 und 4,
**o, p, q** unabhängig voneinander Zahlen von 0 bis 75, wobei o+p+q mindestens 2 ist,
**r** ist 0 oder 1,
**R', R'' und R'''** sind jeweils unabhängig voneinander H, $CH_2COOM$, oder COR, wobei einer oder zwei, bevorzugt einer der Reste R', R'' und R''' $CH_2COOM$ darstellen: Wenn R' = $CH_2COOM$ dann gilt o≠0, wenn R'' = $CH_2COOM$ dann gilt p≠0, wenn R'''= $CH_2COOM$ dann gilt q≠0, mit **M** = H, Alkali- oder Ammoniumkation,
und wobei einer oder zwei der Reste R', R'' und R''' RCO darstellen;
mit **R** einer gesättigten, ein- oder mehrfach ungesättigten Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt;
und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (I);
und wobei das Tensid (I) aus einem Gemisch unterschiedlicher Kettenlängen und Sättigungsgrade des Fettsäurerests RCO wie oben definiert besteht und abgeleitet ist von einem C-18-Pflanzenöl aus der Gruppe umfassend: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Moringa, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen.

2. Mittel gemäss Anspruch 1 **dadurch gekennzeichnet, dass** das mindestens eine Polyoxyalkylen Carboxylate der Formel (I) als ein Gemisch unterschiedlicher Kettenlängen und Sättigungsgrade der Fettsäurereste RCO vorliegt, wobei der Anteil an gesättigten und ungesättigten Fettsäurereste RCO mit 20 oder mehr Kohlenstoffatomen zusätzlich > 0.01 Gew.-% und besonders bevorzugt > 0.05 Gew.-% an Fettsäuren und ganz besonders bevorzugt > 0.1 Gew.% und äusserst bevorzugt >= 0.2 Gew.%.

3. Mittel gemäss einem der vorgehenden Ansprüche zusätzlich enthaltend mindestens eine Seife (C) der Formel (IX)

RCOOM (IX)

M ist ein Alkali- oder Ammoniumkation,
wobei das oder die Seifen (IX) als ein Gemisch unterschiedlicher Kettenlängen und Sättigungsgrade der Fettsäurereste RCO vorliegen und RCO abgeleitet ist von einem C-18-Pflanzenöl mit R und RCO wie in vorgehenden Ansprüchen.

4. Mittel gemäss einem der vorgehenden Ansprüche zusätzlich enthaltend mindestens ein nichtionisches Tensid (D) ausgewählt aus der Gruppe der alkoxylierten Fettsäureester, der alkoxylierten Fettsäureglyceridester, der alkoxylierten Pflanzenölester der Formel (X) oder der alkoxylierten Fettsäureamide der Formel (XI):

(X) $RCO-O(C_mH_{2m}O)_o-\{CH_2-CH[O(C_mH_{2m}O)_pR'']-CH_2O\}_w-(C_mH_{2m}O)_q-R'''$

m ist 2, 3 oder 4,
o, p, q sind unabhängig voneinander Zahlen von 0 bis 75, wobei o+p+q ≠ 0, R" ist H, oder COR,
R"' ist H, COR, oder ein linearer oder verzweigter Alkylrest mit 1-8 C-Atomen,
w ist o oder 1;

(XI) $R\text{- CO-NH-}(C_mH_{2m}O)_n\text{ -H}$

wobei

**m** die ganze Zahlen 2 oder 3 ist,
**n** Zahl im Bereich von 2-10, bevorzugt im Bereich 2-8, ganz besonders bevorzugt 2-4,

wobei das oder die Tenside (X) und (XI) unabhängig voneinander als ein Gemisch unterschiedlicher Kettenlängen und Sättigungsgrade der Fettsäurereste RCO vorliegen und RCO abgeleitet ist von einem C18-Pflanzenöl mit R und RCO wie definiert in vorgehenden Ansprüchen.

**5.** Mittel gemäss einem der vorgehenden Ansprüche, **dadurch gekennzeichnet, dass** der Anteil an den ausgewählten Tensiden, bestehend aus Polyoxyalkylen Carboxylaten (A) der Formel (I), den optionalen Tensiden (C) und (D), und gegebenenfalls Tensiden (E), mit der Vorgabe, dass (E) von C-18-Pflanzenölen abgeleitet sind, und Biotensid-Glycolipide (B), in Summe ≥ 35 Gew.-% beträgt, bevorzugt ≥ 60 Gew.-%, besonders bevorzugt ≥ 95 Gew.-% und äusserst bevorzugt ≥ 99 Gew.-%, bezogen auf den Gesamtgehalt an Tensiden in dem Mittel.

**6.** Wasch-, Pflege- oder Reinigungsverfahren umfassend die Verfahrensschritte

a) Bereitstellen einer Wasch-, Pflege- oder Reinigungslösung umfassend ein Mittel gemäss einem der vorstehenden Ansprüche
b) In Kontakt bringen eines Textils, einer Oberfläche oder eines Körperteils, Haut oder Haar, mit dem Wasch-, Pflege- und Reinigungsmittel gemäss a)

**7.** Verwendung des Mittels gemäss einem der vorhergehenden Ansprüche zur Verbesserung der Reinigungsleistung eines Waschmittels, eines Reinigungs und Pflegemittels oder eines getränkten Tuchs zur Reinigung, insbesondere bei Kohlenhydrat- oder Farbanschmutzungen.

**8.** Verwendung des Mittels gemäss einem der vorhergehenden Ansprüche als Wasch- oder Reinigungsmittel, insbesondere als Handseifen, Handgeschirrspülmittel, Maschinengeschirrspülmittel, Geschirrspülmaschinenreiniger, Waschmaschinenreiniger, Toilettenreiniger-, WC-Reiniger, Universal- und Allzweckreiniger, Küchenreiniger, Bad- und Sanitärreiniger, Fussbodenreiniger, Backofen- und Grillreiniger, Glas- und Fensterreiniger, Metallputzmittel, Polster- und Teppichreiniger, Vollwaschmittel, Colorwaschmittel, Feinwaschmittel, Textilhilfsmittel, Vorbehandlungsmittel, Spezialwaschmittel und -reinigungsmittel, Mittel zur industriellen, gewerblichen und institutionellen Reinigung, Mittel für die Textil- und Faserbehandlung, Mittel für die Lederbehandlung

**9.** Verwendung des Mittels gemäss einem der vorhergehenden Ansprüche als kosmetisches, dermatologische oder medizinisches Reinigungs- und/oder Pflegemittel, wie Duschzubereitungen, Badezusätze, Handseifen, auch zur Grobreinigung, Handpasten, Intimreinigung, Reiniger des Augenbereichs, Kontaktlinsenreiniger, Makeup-Entferner, Augenmakeup-Entferner, Mittel zur Körperreinigung, Mund- und Zahnpflege, Haarshampoos, konditionierende Shampoos, Antischuppen-Shampoo, Babyshampoo, Reinigungs- und Pflegeprodukte insbesondere Körper- und Gesichtstonic, Gesichtslotion, Feuchttücher, insbesondere mit pflegenden, desodorierenden, antimikrobiellen oder anti-ageing Wirkstoffen, Haarentfernung und Rasierprodukte, Haarfärbeshampoos, Haarfärbemittel und Haarstyling-Zubereitungen, Mundhygieneprodukt, insbesondere in Form von Zahnpasta, Zahncreme, Zahngel, Zahnputzflüssigkeit, Zahnputzschaum, Mundwasser, Mundwasserkonzentrat, Mundspray.

**10.** Mittel gemäss Anspruch 1, **dadurch gekennzeichnet, dass** das Glycolipid-Biotensid (B) ein Sophorolipid oder Rhamnolipid ist.

**11.** Mittel gemäss Anspruch 1, **dadurch gekennzeichnet dass** Tensid (A) Natrium Olivenöl PEG-7 Carboxylat ist.

**12.** Mittel gemäss Anspruch 4, **dadurch gekennzeichnet, dass** Tensid (D) ausgewählt ist aus den Vertretern Pflanzenöl Glycereth-y Ester, Pflanze-PEG-y-ester, Ethoxylierter Rapsmethylester (y EO), mit Pflanze = Aprikose(nkern), Avocado, Baumwolle, Distel, Hanf, Jojoba, Leinsamen, Macademia, Mandel, Mais, Olive, Pfirsichkern, Reis, Sesam, Soja, Sonnenblume, Traube(nkern), Weizen(keim), Raps, Rüböl mit y= 6-10, bevorzugt 7-8 und PEG-4 Rapssamenamid.

## Claims

**1.** Composition containing at least one alkoxylated surfactant (A) from the group of polyoxyalkylene carboxylates of the formula (I) and at least one glycolipid biosurfactant (B) comprising rhamnolipids, sophorolipids, mannosylerythritol lipids, cellobiose lipids and trehalose lipids, wherein the surfactant or surfactants (A) are present in the ratio of ≥ 1: 6 to the biosurfactants (B); based on percent by weight of active agent in the whole composition;

(I) $$R'\text{-}O(C_bH_{2b}O)_o\text{-}\{CH_2\text{-}CH[O(C_bH_{2b}O)_pR'']\text{-}CH_2O\}_r\text{-}(C_bH_{2b}O)_q\text{-}R'''$$

with

**b** as an integer between 2 and 4,
**o, p, q** are independently numbers from 0 to 75, wherein o + p + q is at least 2,
**r** is 0 or 1,
**R', R" and R"'** are each independently H, CH $_2$COOM, or COR, wherein one or two, preferably one of the radicals R', R" and R"' represents $CH_2COOM$: if R' = $CH_2COOM$ then o≠0 applies, if R" = $CH_2COOM$ then p ≠ 0 applies, if R"' = CH $_2$COOM then q≠0 applies, with **M** = H, alkali or ammonium cation,
and wherein one or two of radicals R', R" and R"' represent RCO;
with **R** being a saturated, mono- or polyunsaturated hydrocarbon chain having 5-23 carbon atoms and RCO derived from a fatty acid mixture, wherein the proportion of 18 and more carbon atoms of the fatty acid radicals RCO is above 60 wt.-%, preferably above 72 wt.-% and particularly preferably above 77 wt.-%,
and wherein the proportion of unsaturated fatty acid radicals is above 55 wt.-%, preferably above 65 wt.-%, and particularly preferably above 72 wt.-%, in each case based on the total amount of fatty acid radicals RCO of the surfactant (I) used ;
and wherein the surfactant (I) consists of a mixture of different chain lengths and degrees of saturation of the fatty acid radicals RCO as defined above and is derived from a C18 vegetable oil from the group comprising: amaranth, anise, apple, apricot, argan, arnica, avocado, cotton, borage, nettle, broccoli, canola, chia, hemp, hazelnut, beech, boxwood, thistle, spelt, peanut, tigernut, lilac, garden cress, barley, pomegranate, oat, hemp, hazelnut, blueberry, elderberry, jasmine, currant, St. John's wort, jojoba, camellia, chamomile, caraway, carrot, cherry, coriander, mullein, crambe, caper spurge, squash, Iberian dragon head, lavender, camelina, linseed, privet, lupine, lucerne, macadamia, corn, almond, marula, mirabelle, melon, poppy, mongongo, moringa, evening primrose, olive, oil radish, rocket, passionflower, pecan, peach, plum, pistachio, cranberry, jatropha, rapeseed, rice, marigold, turnip rape, safflower, sage, sea buckthorn, black cumin, sesame, sesame leaf, mustard, sunflower, soybean, tobacco, walnut, grape, wheat, meadowfoam and wild rose; and combinations thereof.

**2.** Composition according to claim 1, **characterized in that** the at least one polyoxyalkylene carboxylate of the formula (I) is present as a mixture of different chain lengths and degrees of saturation of the fatty acid radicals RCO, wherein additionally the proportion of saturated and unsaturated fatty acid radicals RCO having 20 or more carbon atoms is > 0.01 wt.-% and preferably > 0.05 wt.-% of the fatty acid radicals and very preferably > 0.1 wt.-% and extremely preferably >= 0.2 wt.-%.

**3.** Composition according to one of the preceding claims additionally containing at least one soap (C) of the formula (IX)

RCOOM (IX)

M is an alkali or ammonium cation,
wherein the soap or soaps (IX) are present as a mixture of different chain lengths and degrees of saturation of the fatty acid radicals RCO, and RCO is derived from a C18 vegetable oil with R and RCO as defined in the preceding claims.

**4.** Composition according to one of the preceding claims, additionally containing at least one non-ionic surfactant (D)

selected from the group of alkoxylated fatty acid esters, alkoxylated fatty acid glycerol esters, alkoxylated vegetable oil esters of formula (X) or alkoxylated fatty acid amides of formula (XI):

$$\text{(X)} \qquad RCO\text{-}O(C_mH_{2m}O)_o\text{-}\{CH_2\text{-}CH[O(C_mH_{2m}O)_pR'']\text{-}CH_2O\}_w\text{-}(C_mH_{2m}O)_q\text{-}R'''$$

m is 2, 3 or 4,
o, p, q are, independent of one another, numbers from 0 to 75, wherein o+p+q ≠ 0,
R" is H or COR,
R"' is H, COR, or a linear or branched alkyl radical having 1-8 carbon atoms,
w is 0 or 1;

$$\text{(XI)} \qquad R\text{- }CO\text{-}NH\text{-}(C_mH_{2m}O)_n\text{ -}H$$

wherein

**m** is the integer 2 or 3,
**n** is a number in the range of 2-10, preferably in the range of 2-8, most preferably 2-4,

wherein the surfactant or the surfactants (X) and (XI) are, independently of one another, present as a mixture of different chain lengths and degrees of saturation of the fatty acid radicals RCO, and RCO is derived from a C18 vegetable oil with R and RCO as defined in the preceding claims.

**5.** Composition, according to one of the preceding claims, **characterized in that** the proportion of the selected surfactants consisting of polyoxyalkylene carboxylates (A) of the formula (I), the optional surfactants (C) and (D), and if appropriate, surfactants (E), with the requirement that (E) is derived from C18 vegetable oils, and biosurfactant glycolipids (B), is in total ≥ 35 wt.-%, preferably ≥ 60 wt.-%, more preferably ≥ 95 wt.-% and most preferably ≥ 99 wt.-%, based on the total content of surfactants in the composition.

**6.** A washing, care, or cleaning method comprising the process steps:

a) providing a washing, care or cleaning solution comprising a composition as defined in one of the preceding claims
b) contacting a fabric, a surface or a body part, skin or hair, with the washing, care and cleaning agent as defined under a).

**7.** Use of the composition according to one of the preceding claims for improving the cleaning performance of a detergent, a cleaning and care product, or an impregnated cloth for cleaning, particularly from carbohydrate or colored stains.

**8.** Use of the composition according to one of the preceding claims as detergents or cleaning agents, in particular as hand soaps, hand dishwashing detergents, machine dishwashing detergents, dishwasher cleaners, washing machine cleaners, toilet cleaners, WC cleaners, universal and all-purpose cleaners, kitchen cleaners, bathroom and sanitary cleaners, floor cleaners, oven and grill cleaners, glass and window cleaners, metal cleaners, upholstery and carpet cleaners, heavy-duty detergents, color detergents, light-duty detergents, textile auxiliary agents, pretreatment agents, specialty detergents and cleaners, products for industrial, commercial and institutional cleaning, products for textile and fiber treatment, products for leather treatment.

**9.** Use of the composition according to one of the preceding claims as a cosmetic, dermatological or medical cleaning and/or care product, such as shower preparations, bath additives, hand soaps, also for heavy cleaning, hand washing paste, intimate cleansing, cleansers for the eye area, contact lens cleaners, makeup removers, eye make-up removers, body cleansing preparations, oral and dental care, hair shampoos, conditioning shampoos, anti-dandruff shampoo, baby shampoo, cleansing and care products, in particular body and facial toners, face lotions, wipes, in particular with care, deodorizing, antimicrobial or anti-aging active ingredients, hair removal and shaving products, hair dyeing shampoos, hair dyeing and hair styling preparations, oral hygiene products, in particular in the form of toothpaste, dentifrice, tooth gel, tooth cleaning liquid, tooth cleaning foam, mouthwash, mouthwash concentrate, mouth spray.

**10.** Composition according to claim 1 **characterized in that** the glycolipid biosurfactant (B) is a sophorolipid or rhamnolipid.

**11.** Composition according to claim 1 **characterized in that** the surfactant (A) is sodium olive oil PEG-7 carboxylate.

**12.** Composition according to claim 4 **characterized in that** the surfactant (D) is selected from the representatives of vegetable oil glycereth-y esters, plant PEG-y esters, ethoxylated rapeseed methyl esters (y EO), with plant = apricot (kernel), avocado, cotton, thistle, hemp, jojoba, linseed, macademia, almond, corn, olive, peach kernel, rice, sesame, soy, sunflower, grape (seed), wheat (germ), rapeseed, colza with y = 6-10, preferably 7-8, and PEG-4 rapeseed amide.

## Revendications

**1.** Composition comprenant au moins un tensioactif alcoxylé (A) du groupe des polyoxyalkylène carboxylates de formule (I) et au moins un biotensioactif glycolipidique (B) comprenant des rhamnolipides, des sophorolipides, des lipides de mannosylérythritol, des lipides de cellobiose et des lipides de tréhalose, où le ou les tensioactifs (A) sont présents dans un rapport $\geq$ 1:6 aux biotensioactifs (B); basé sur % en poids d'agent actif dans la composition entière;

(I) $$R'-O(C_bH_{2b}O)_o-\{CH_2-CH[O(C_bH_{2b}O)_pR'']-CH_2O\}_r-(C_bH_{2b}O)_q-R'''$$

avec

b un entier compris entre 2 et 4,
o, p, q sont indépendamment des nombres de 0 à 75, où o + p + q est au moins 2,
r est 0 ou 1,
R', R" et R"' sont chacun indépendamment H, $CH_2COOM$ ou COR, où un ou deux, de préférence un des radicaux R', R" et R"' représente $CH_2COOM$: si R' = $CH_2COOM$ alors o $\neq$ 0, si R" = $CH_2COOM$ alors p $\neq$ 0, si R"'= $CH_2COOM$ alors q $\neq$ 0, avec M = H, un cation de métal alcalin ou d'ammonium,
et dans lequel un ou deux radicaux parmi R', R" et R"' représentent RCO;
avec R étant une chaîne hydrocarbonée saturée, mono- ou polyinsaturée, ayant 5-23 atomes de carbone et RCO dérivé d'un mélange d'acides gras dans lequel la proportion de résidus d'acide gras RCO de 18 atomes de carbone et plus est supérieure à 60% en poids, de préférence supérieure à 72% en poids et de manière particulièrement préférée supérieure à 77% en poids;
et dans lequel la proportion de résidus d'acides gras insaturés est supérieure à 55% en poids, de préférence supérieure à 65% en poids et de manière particulièrement préférée supérieure à 72% en poids, chaque fois basé sur la totalité des résidus d'acide gras RCO dans le tensioactif (I) utilisé;
et dans laquelle le tensioactif (I) consiste en un mélange de différents longueurs de chaîne et degrés de saturation des residus d'acide gras RCO tel que défini ci-dessus et est dérivé d'une huile végétale C-18 du groupe comprenant:
amarante, anis, pomme, abricot, argan, arnica, avocat, coton, bourrache, ortie, brocoli, canola, chia, chanvre, noisette, hêtre, buis, chardon, épeautre, arachide, souchet comestible, lilas, cresson alénois, orge, grenade, avoine, chanvre, noisette, bleuet, sureau, jasmin, groseille, millepertuis, jojoba, camélia, camomille, carvi, carotte, cerise, coriandre, molène, crambe, euphorbe épurge, courge, la tête de dragon ibérique, lavande, caméline, graines de lin, troène, lupin, luzerne, macadamia, maïs, amande, marula, mirabelle, melon, pavot, mongongo, moringa, onagre, olive, radis oléagineux, roquette, passiflore, noix de pécan, pêche, prune, pistache, airelle, jatropha, colza, riz, souci, navette, carthame, sauge, argousier, cumin noir, sésame, feuille de sésame, moutarde, tournesol, soja, tabac, noyer, raisin, blé, limnanthe, et rose musquée, ainsi que leurs combinaisons;

**2.** Composition selon la revendication 1, **caractérisée en ce que** le au moins un polyoxyalkylène carboxylate de formule (I) est présent sous la forme d'un mélange de différents longueurs de chaîne et de degrés de saturation des résidus d'acide gras RCO, dans lequel en outre la proportion de résidus d'acide gras saturés et insaturés RCO avec 20 atomes de carbone ou plus est > 0,01 % en poids, et plus préférentiellement > 0,05% en poids des résidus d'acides gras, et de manière tout particulièrement préférée > 0,1% en poids, et très préférentiellement > = 0,2% en poids.

**3.** Composition selon l'une des revendications précédentes contenant en outre au moins un savon (C) de formule (IX)

RCOOM (IX)

M est un cation de métal alcalin ou d'ammonium,
dans laquelle le savon ou les savons (IX) sont présents sous forme d'un mélange de différents longueurs de chaîne et degrés de saturation des résidus d'acide gras RCO, et RCO est dérivé d'une huile végétale C-18, avec R et RCO comme dans les revendications précédentes.

**4.** Composition selon l'une des revendications précédentes contenant en outre au moins un tensioactif non ionique (D) choisi dans le groupe des esters d'acides gras alcoxylés, les esters de glycérides d'acide gras alcoxylés, les esters d'huile végétale alcoxylés de formule (X) ou des amides d'acides gras alcoxylés de formule (XI):

(X) $RCO\text{-}O(C_mH_{2m}O)_o\text{-}\{CH_2\text{-}CH[O(C_mH_{2m}O)_pR'']\text{-}CH_2O\}_w\text{-}(C_mH_{2m}O)_q\text{-}R'''$

m est 2, 3 ou 4,
o, p, q sont, indépendamment l'un de l'autre, des nombres de 0 à 75, où $o+p+q \neq 0$, R" est H ou COR,
R"' est H, COR ou un radical alkyle linéaire ou ramifié ayant 1 à 8 atomes de carbone,
w est 0 ou 1 ;

(XI) $R\text{-}CO\text{-}NH\text{-}(C_mH_{2m}O)_n\text{-}H$

dans lequel

m est le nombre entier 2 ou 3,
n est compris entre 2 et 10, de préférence entre 2 et 8, tout particulièrement entre 2 et 4,

dans laquelle le ou les tensioactifs (X) et (XI) sont indépendamment présents sous forme d'un mélange de différents longueurs de chaîne et degrés de saturation des résidus d'acide gras RCO, et RCO est dérivé d'une huile végétale C18 avec R et RCO tels que définis dans les revendications précédentes.

**5.** Composition selon l'une des revendications précédentes, **caractérisée en ce que** la proportion des tensioactifs choisis, constituée des polyoxyalkylène carboxylates (A) de formule (I), éventuellement des tensioactifs (C) et (D), et éventuellement des tensioactifs (E), à condition que (E) est dérivé d'huiles végétales C-18, et des tensioactifs glycolipidiques (B) est au total ≥ 35% en poids, de préférence ≥ 60% en poids, de manière particulièrement préférée ≥ 95% en poids et le plus préférablement ≥ 99% en poids, sur la base de la teneur totale en tensioactifs dans la composition.

**6.** Procédé de lavage, de soin ou de nettoyage comprenant les étapes du processus

a) fournir une solution de lavage, de soin ou de nettoyage comprenant une composition selon l'une quelconque des revendications précédentes
b) la mise en contact d'un textile, d'une surface ou d'une partie du corps, de la peau ou des cheveux, avec l'agent de lavage, de soin et de nettoyage selon a).

**7.** Utilisation de la composition selon l'une des revendications précédentes pour améliorer les performances de nettoyage d'un agent de lavage, d'un agent de nettoyage et de soin ou d'un chiffon imbibé pour le nettoyage, en particulier pour les taches de glucides ou de couleur.

**8.** Utilisation de la composition selon l'une des revendications précédentes comme détergents ou nettoyants, notamment comme savons pour les mains, détergents pour vaisselle à main, détergents pour lave-vaisselle, nettoyants pour machine à laver la vaisselle, nettoyants pour lave-linge, nettoyants pour toilettes, nettoyants pour WC, nettoyants universels et nettoyants multi-usage, nettoyants pour la cuisine, nettoyants pour salle de bains et nettoyants sanitaires, nettoyants pour plancher, nettoyants pour four et nettoyants pour barbecue, nettoyants pour verre et pour vitres, nettoyants pour métaux, nettoyants pour meubles rembourrés et pour tapis, lessives universelles, détergents pour couleur, détergents pour linges délicats, produits auxiliaires pour textiles, préparations de prétraitement, détergents et nettoyants spéciaux, produits de nettoyage industriels, commerciaux et institutionnels, produits de traitement de fibre et de textile, produits de traitement de cuir.

**9.** Utilisation de la composition selon l'une des revendications précédentes comme produit de nettoyage et / ou de soin cosmétique, dermatologique ou médical, tel que préparations pour la douche, produits pour le bain, savons pour les mains, également pour le nettoyage grossier, les pâtes pour les mains, le nettoyage intime, le nettoyant pour le contour des yeux, le nettoyant pour lentilles de contact, le démaquillage, le démaquillage des yeux, préparations nettoyantes pour le corps, soins bucco-dentaires, shampooings, shampooing de conditionnement, shampooings antipelliculaires, shampooings pour bébés, produits de nettoyage et de soins personnels, en particulier toniques pour le corps et le visage, lotions pour le visage, lingettes, notamment avec des substances actives soignantes, désodorisantes, antimicrobiennes ou anti-âge, produits pour l'épilation et le rasage, shampooings colorants, teintures capillaires et des préparations du hair styling, produits pour l'hygiène buccale, notamment le dentifrice sous forme de pâtes, de crèmes, de gels, de liquides, de mousses nettoyantes, bain de bouche, concentré pour bain de bouche, spray pour la bouche.

**10.** Composition selon la revendication 1, **caractérisée en ce que** le biotensioactif glycolipidique (B) est un sophorolipide ou un rhamnolipide.

**11.** Composition selon la revendication 1, **caractérisée en ce que** le tensioactif (A) est sodium PEG-7 olive oil carboxylate.

**12.** Composition selon la revendication 4, **caractérisée en ce que** le tensioactif (D) est choisi parmi les représentants des glycereth-y esters de l'huile végétale, des PEG-y-esters de l'huile végétale, des esters méthyliques d'huile de colza éthoxylés (y EO), avec l'huile végétale = abricot (noyant d'abricot), avocat, coton, chardon, chanvre, jojoba, graines de lin, macadamia, amande, maïs, olive, noyau de pêche, riz, sésame, soja, tournesol, raisin (noyau), blé (germe), colza, huile de navette avec y = 6-10, de préférence 7-8 et PEG-4 amide de colza.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 209783 A
- DE 19600743, Henkel
- WO 2011051161 A1, Henkel
- EP 0499434 A1, Unilever
- EP 1445302 A1
- US 2014113818 A, Ecover
- JP 2009275145 B, Seraya
- WO 2012617815 A, Ecover
- WO 2016050439 A, Evonik
- WO 2014166796 A
- WO 2014118095 A
- WO 2016066464 A, Henkel
- EP 0499434 A
- US 5520839 A
- US 20040265264 A, Beiersdorf
- WO 2013098066 A, Evonik
- DE 10147049, Beiersdorf
- US 7985722 B
- WO 03006146 A
- JP 60183032 A
- DE 19648439
- JP 1304034 A
- CN 1337439
- JP 2006 A
- JP 274233 A
- KR 2004033376
- JP 2006083238 A
- JP 2006070231 A
- WO 03002700 A
- FR 2740779
- DE 2939519
- US 7556654 B
- FR 2855752
- EP 1445302 A
- JP 2008062179 A
- JP 2007181789 A
- WO 2004020647 A
- JP 20042544595 B
- EP 0282942 A
- KR 20100022289
- ES 2018637
- CN 1431312
- CN 102250790
- US 2008032383 A
- DE 4319540
- FR 2692593
- JP 2004254595 B
- JP 2007252279 B
- CN 101845468
- CN 101948786


### In der Beschreibung aufgeführte Nicht-Patentliteratur


- Safety Assessment of PEGylated Oils as Used in Cosmetics. *International Journal of Toxicology,* November 2014, vol. 33
- Safety Assessment of PEGylated Alkyl Glycerides as Used in Cosmetics. *Cosmetic Ingredient Review (CIR),* 2014
- International Cosmetic Ingredient Dictionary and Handbook. 2010
- **C. MULLIGAN.** Biosurfactants: Research Trends and Application. CRC Press, 2014, 112
- **CHIBUZO UZOIGWE et al.** *Front. Microbiol.,* 2015, vol. 6, 245
- Fundamentals and Formulations of Cosmetics. **K. SCHRADER.** International Cosmetic Ingredient Dictionary and Handbook. Hüthig Verlag, 1989, 782-815
- Polyalkylsiloxane, Polyalkylarylsiloxane und Polyethersiloxan-Copolymere. *Cosmetics: Science and Technology,* 1972, vol. 1, 27-104
- *Chem.Rev.,* 1996, vol. 96, 951